# FASCICULE DE BREVET EUROPEEN

(11) **EP 2 901 160 B1**
(45) Date de publication et mention de la délivrance du brevet: **19.10.2016**
(21) Numéro de dépôt: 13782738.2
(22) Date de dépôt: 24.09.2013
(51) Int. Cl.: G01N 33/566, C07F 9/30, G01N 33/573, G01N 33/68, C07F 9/653, C07F 9/6558

(54) **MÉTHODE POUR DÉTECTER SPÉCIFIQUEMENT DANS UN ECHANTILLON UNE MÉTALLOPROTÉASE MATRICIELLE (MMP) D'INTÉRÊT UNIQUEMENT DANS SA FORME ACTIVE**
VERFAHREN ZUM SPEZIFISCHEN NACHWEIS EINER NUR IN AKTIVER FORM RELEVANTEN MATRIX-METALLOPROTEINASE (MMP) IN EINER PROBE
METHOD FOR SPECIFICALLY DETECTING A MATRIX METALLOPROTEINASE (MMP) WHICH IS ONLY OF INTEREST IN THE ACTIVE FORM THEREOF, IN A SAMPLE

(30) Priorité: 25.09.2012 FR 1258996
(43) Date de publication de la demande: 05.08.2015
(73) Titulaire: Commissariat à l'Energie Atomique et aux Energies Alternatives, 75015 Paris (FR)
(72) Inventeur: BREGANT, Sarah, F-75014 Paris (FR); DIVE, Vincent, F-91120 Palaiseau (FR); NEVERS, Marie-Claire, F-91140 Villebon Sur Yvette (FR)
(74) Mandataire: Gallois, Valérie
(86) Numéro de dépôt international: PCT/FR2013/052224
(87) Numéro de publication internationale: WO 2014/049255

(56) Documents cités:
- SARAH BREGANT ET AL: "Detection of Matrix Metalloproteinase Active Forms in Complex Proteomes: Evaluation of Affinity versus Photoaffinity Capture", JOURNAL OF PROTEOME RESEARCH, vol. 8, no. 5, 1 mai 2009 (2009-05-01), pages 2484-2494, XP055062316, ISSN: 1535-3893, DOI: 10.1021/pr801069c
- LAPAN PETER ET AL: "Optimization of total protein and activity assays for the detection of MMP-12 in induced human sputum", BMC PULMONARY MEDICINE, BIOMED CENTRAL, LONDON, GB, vol. 10, no. 1, 2 août 2010 (2010-08-02), page 40, XP021076155, ISSN: 1471-2466, DOI: 10.1186/1471-2466-10-40 cité dans la demande
- ANNE-SOPHIE DABERT-GAY ET AL: "Molecular determinants of matrix metalloproteinase-12 covalent modification by a photoaffinity prob", JOURNAL OF BIOLOGICAL CHEMISTRY, AMERICAN SOCIETY FOR BIOCHEMISTRY AND MOLECULAR BIOLOGY, US, vol. 283, no. 45, 7 novembre 2008 (2008-11-07), pages 31058-31067, XP002615480, ISSN: 0021-9258, DOI: 10.1074/JBC.M805795200 [extrait le 2008-09-05]
- SONA KRIZKOVA ET AL: "Assays for determination of matrix metalloproteinases and their activity", TRAC, TRENDS IN ANALYTICAL CHEMISTRY, vol. 30, no. 11, décembre 2011 (2011-12), pages 1819-1832, XP028125210, ISSN: 0165-9936, DOI: 10.1016/J.TRAC.2011.06.016 [extrait le 2011-10-08]
- CATHERINE NURY ET AL: "A Pan Photoaffinity Probe for Detecting Active Forms of Matrix Metalloproteinases", CHEMBIOCHEM, vol. 14, no. 1, 2 janvier 2013 (2013-01-02), pages 107-114, XP055062681, ISSN: 1439-4227, DOI: 10.1002/cbic.201200583
- C. NURY ET AL: "Detection of Endogenous Matrix Metalloprotease-12 Active Form with a Novel Broad Spectrum Activity-based Probe", JOURNAL OF BIOLOGICAL CHEMISTRY, vol. 288, no. 8, 22 février 2013 (2013-02-22), pages 5636-5644, XP055062683, ISSN: 0021-9258, DOI: 10.1074/jbc.M112.419499

## Description

La présente invention est relative à une méthode de détection spécifique d'une métalloprotéinase de la matrice (MMP).

### CONTEXTE DE L'INVENTION

Les MMPs sont capables de dégrader collectivement l'ensemble des composants de la matrice extracellulaire (ECM). La dégradation de l'ECM, en plus de permettre la migration cellulaire, conduit à la libération de molécules de signalisation initialement liées à l'ECM comme les chémokines, les cytokines ou encore des facteurs de croissance. Les MMPs contribuent également à l'activation de molécules de signalisation.

L'activité des MMPs de remodelage des tissus est fortement régulée *in vivo,* notamment par des inhibiteurs naturels que sont les TIMP (inhibiteurs tissulaires des métalloprotéinases) ou encore par un état de formes latentes que sont les proformes des MMPs. En effet, il peut coexister une forme active des MMPs dont le site actif est libre et donc capable d'interagir avec ses substrats naturels, une proforme inactive des MMPs dont le site actif est occupé par le pro-peptide, et une forme inhibée des MMPs dont le site actif est occupé par l'inhibiteur naturel.

La dérégulation de cette activité, associée à la présence de formes actives compétentes pour la protéolyse, accompagne l'évolution et la progression de pathologies. Les pathologies concernées sont le cancer (plusieurs MMPs biomarqueurs, notamment MMP-2, MMP-9), les infections virales (MMP-9, MMP-2), l'arthrose et l'arthrite rhumatoide (MMP-13), la maladie de Dupuytren (MMP-2, MMP-14), l'athérosclérose (MMP-12), ou encore des pathologies respiratoires à composantes inflammatoires, comme la broncho-pneumopathie chronique obstructive (BPCO) (MMP12), l'emphysème, et l'asthme (MMP8). Les MMPs ont également un rôle dans les maladies neurodégénératives : sclérose en plaque (MMP-3, MMP-8, MMP-9), myasthénie gravis (MMP-2, MMP-3, MMP-9), accident vasculaire cérébral (MMP-9, MMP-3), la sclérose amyotrophique latérale (MMP-1 et MMP-2), la maladie d'Alzheimer (MMP-3, MMP-9, et MMP-10).

Les formes actives des MMP constituent donc des marqueurs de ces pathologies et leur détection constitue un véritable challenge diagnostique auquel l'invention décrite dans ce brevet se propose de répondre.

En effet, bien que de nombreuses méthodes de détection des MMPs soient décrites, il n'existe aucune méthode satisfaisante permettant de détecter une MMP particulière et spécifiquement la forme active de celle-ci, c'est-à-dire en excluant les formes inactives (proformes et formes inhibées).

Les études de transcriptomes ne nous informent, par mesure des ARN messager, que sur la quantité d'ARNm codant pour la protéine existant au final dans les fluides sous différentes formes. Ainsi, la mesure de ARNm transcrits n'informe pas sur la proportion de formes actives.

Les méthodes basées sur des substrats synthétiques de MMPs posent d'autres problèmes. En effet, la plupart des kits vendus utilisant des substrats pour la détection de MMPs implique une étape d'activation de l'enzyme elle-même par l'ajout d'un sel mercurique qui abolit toute mesure spécifique de la présence d'une forme initialement active. Mais surtout, l'utilisation de substrat n'informe pas en outre sur l'identité de la MMP impliquée dans le clivage du substrat puisqu'il n'existe pas de forte spécificité des substrats synthétiques entre les différentes MMPs. Ainsi, ces méthodes ne sont pas spécifiques d'une MMP particulière.

Toutes les techniques basées sur l'électrophorèse utilisent une étape de dénaturation partielle ou totale qui empêche la distinction entre une forme enzymatique active et une forme enzymatique dont l'activité est initialement régulée par la présence d'un inhibiteur naturel bloquant le site actif. En effet, l'étape nécessaire de dénaturation de ces techniques abolit l'existence de MMPs inactivées qui sont alors analysées comme forme active. De plus, l'utilisation d'anticorps dans le Western-Blot reste peu sensible pour la détection de protéines présentes à des concentrations très faibles dans les échantillons. Ainsi, ces techniques détectent à la fois les formes activées et inactivées et ne sont pas très sensibles.

La technique dite de zymographie, très spécifique pour la détection de MMP2 et MMP9 (appelées également gélatinases), souffre également de l'inconvénient d'une étape de dénaturation partielle de l'échantillon, nécessaire à sa mise en place. De plus, l'utilisation d'électrophorèse en routine limite la quantité totale d'échantillon analysée de par des contraintes méthodologiques de migration limitant ainsi la quantité d'analyte d'intérêt dans l'analyse.

Les tests de type ELISA, dont certains sont commercialisés pour la détection de MMP12, utilisent un jeu d'anticorps complémentaires sans permettre de distinguer formes actives, proformes, et formes inactivées, empêchant ainsi un accès à l'information fonctionnelle de la protéine.

L'ensemble de ces éléments fait que des recherches ont été faites pour permettre de détecter spécifiquement les formes actives de MMPs impliquées dans les pathologies par leur activité protéolytique. Pour les méthodes développées antérieurement dans ce but, notamment les sondes basées sur l'activité (« *activiy based-probe* »), les inconvénients sont relatifs à un manque de sensibilité ou de spécificité, en particulier lorsque sont considérés des échantillons biologiques complexes ou des échantillons de tissus. Encore une fois, les limitations sont dues à des contraintes méthodologiques de manipulation d'échantillon.

L'art antérieur décrit une méthode in vitro pour détecter spécifiquement, dans un échantillon biologique complexe (un extrait de tumeur), une métalloprotéase matricielle d'intérêt (la MMP-8 et/ou la MMP-12) uniquement dans sa forme active et comprenant une étape de mise en contact de l'échantillon biologique avec un ligand de la MMP d'intérêt capable de se fixer au site actif libre de la MMP, le ligand comprenant un inhibiteur pseudo-peptidique phosphinique, une étape de capture du complexe binaire entre la MMP d'intérêt et le ligand de la MMP d'intérêt par des billes magnétiques, et une étape de détection dudit complexe binaire, soit par électrophorèse, soit par spectrométrie de masse (Bregant et al. Journal of Proteome Research 2009, 8(5): 2482-2494). L'art antérieur décrit également des méthodes pour la détection de la forme active d'une MMP d'intérêt utilisant un substrat pseudo-peptidique phosphinique portant un groupe qui sera photo-activé et fixé de façon covalente à la MMP (Dabert-Gay et al. Journal of Biological Chemistry 2008, 283(45):31058-31067).

Le seul exemple dans la littérature décrit comme respectant les impératifs nécessaires à la détection de MMPs sous forme active concerne la détection de MMP12 humaine et utilise un système combinant l'utilisation d'un anticorps et d'un substrat fluorescent (LaPan et al. BMC Pulmonary Medecine 2010, 10 :40). Cependant, aucune donnée sur la capacité de tels systèmes à exclure la détection de MMP12 initialement inactivée par un inhibiteur n'est fournie. Pourtant, cette méthode comprend une étape de lavage préalable entre la fixation de la protéine sur l'anticorps et l'ajout du substrat, étape qui est susceptible d'éliminer les inhibiteurs naturels (TIMPs) fixés par la MMP12. Ainsi, cette méthode permet de distinguer entre proforme et forme activée, mais ne peut distinguer entre les formes activées dont le site actif est libre et celles dont le site actif est initialement occupé par un inhibiteur dans un échantillon biologique. Par ailleurs, une telle approche n'est utilisable qu'en format EIA (Test immuno-enzymatique).

Les inconvénients majeurs mentionnés ci-dessus sont de ne pas permettre la distinction entre les formes actives et les formes inactivées des MMPs, et/ou de laisser une ambiguïté quant à l'identification de la MMP détectée. Les limitations proviennent de contraintes méthodologiques de manipulation d'échantillon.

Ainsi, il existe toujours un fort besoin de méthodes permettant de détecter spécifiquement la forme active d'une MMP particulière, notamment à partir d'un échantillon biologique complexe qui peut comprendre une forte concentration protéique avec une faible représentation de la MMP considérée.

### RESUME DE L'INVENTION

Dans le contexte des MMPs qui présentent une forte homologie de séquence/structure et une faible spécificité de substrat, notamment pour les substrats synthétiques utilisés dans les tests, la présente invention fournit une méthode permettant de détecter spécifiquement uniquement la forme active d'une MMP particulière, et ce dans des milieux biologiques complexes avec une bonne sensibilité et rapidement.

La présente invention concerne une méthode *in vitro* pour détecter spécifiquement dans un échantillon biologique une métalloprotéase matricielle (MMP) d'intérêt uniquement dans sa forme active, comprenant
a- une étape de mise en contact de l'échantillon biologique avec un ligand de la MMP d'intérêt capable de se fixer au site actif libre de ladite MMP;
b- ultérieurement ou simultanément à l'étape a), une étape de mise en contact avec un anticorps spécifique de la MMP d'intérêt du résultat de l'étape a); et
c- une étape de détection du complexe ternaire entre la MMP d'intérêt, le ligand de la MMP d'intérêt, et l'anticorps spécifique de la MMP d'intérêt,
et dans laquelle le ligand comprend un inhibiteur pseudopeptidique phosphinique.

De préférence, soit le ligand de la MMP d'intérêt, soit l'anticorps spécifique de la MMP d'intérêt est immobilisé sur un support solide, et la détection est réalisée soit par la détection de l'anticorps lorsque le ligand est immobilisé sur le support, soit par la détection du ligand lorsque l'anticorps est immobilisé sur le support.

Dans un premier mode de réalisation préféré, la méthode comprend
a) la fourniture d'un support solide sur lequel est immobilisé un ligand de la MMP d'intérêt ;
b) la mise en contact du support solide avec l'échantillon de manière à permettre la fixation de la MMP d'intérêt présente dans l'échantillon au support solide par l'intermédiaire d'une liaison entre le ligand et la MMP d'intérêt ;
c) facultativement, l'élimination des MMPs non fixées et autres protéines de l'échantillon;
d) l'ajout d'un anticorps spécifique de la MMP d'intérêt de manière à permettre la formation du complexe ternaire entre la MMP d'intérêt, le ligand de la MMP d'intérêt, et l'anticorps spécifique de la MMP d'intérêt ;
e) l'élimination des anticorps libres ; et
f) la détection des anticorps dans le complexe ternaire, la détection de ceux-ci étant indicative de la présence dans l'échantillon de la MMP d'intérêt sous forme active.

Dans un deuxième mode de réalisation alternatif, la méthode comprend
a) la fourniture d'un support solide sur lequel est immobilisé un anticorps spécifique de la MMP d'intérêt ;
b) la mise en contact du support solide avec l'échantillon qui est préalablement ou simultanément mis en contact avec un ligand de la MMP d'intérêt, de manière à permettre la fixation de la MMP d'intérêt présente dans l'échantillon au ligand et l'immobilisation de la MMP d'intérêt au support solide par l'intermédiaire d'une liaison entre l'anticorps et la MMP d'intérêt;
c) l'élimination des ligands et MMP libres ; et
d) la détection du ligand dans le complexe ternaire entre la MMP d'intérêt, le ligand de la MMP d'intérêt, et l'anticorps spécifique de la MMP d'intérêt, la détection de celui-ci étant indicatif de la présence dans l'échantillon de la MMP d'intérêt sous forme active.

De préférence, la méthode utilise un test d'immunochromatographie (ICT) ou un test immunologique en phase solide, la phase solide pouvant être notamment une membrane (« Flow-through »), un puits de plaque de microtitration (par exemple, EIA) ou des bandelettes (« dipstick »). La détection du ligand ou de l'anticorps est réalisée par couplage covalent ou non-covalent de celui-ci à un marqueur détectable. Le marqueur détectable peut être sélectionné parmi le groupe consistant en un métal colloïdal, un colloïde non-métallique, du carbone, un traceur visible, fluorescent, luminescent ou chimio luminescent, une particule magnétique, un élément radioactif, des billes de latex portant un traceur visible ou fluorescent, et une enzyme, de préférence l'or colloïdal ou une enzyme.

Dans un mode de réalisation particulier, le ligand est couplé à une protéine porteuse, de préférence par l'intermédiaire d'un lien ou espaceur, en particulier un lien polyéthylèneglycol. La protéine porteuse peut être mono- ou poly-fonctionnalisée avec le ligand de la MMP d'intérêt. Elle peut être une sérum albumine, de préférence humaine ou bovine. Elle peut être également une enzyme comme une acétylcholinestérase .

L'échantillon biologique peut être un liquide ou fluide biologique ou un extrait tissulaire ou cellulaire.

La MMP d'intérêt peut être sélectionnée parmi MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-26, MMP-27 et MMP-28, de préférence parmi MMP-2, MMP-3, MMP-8, MMP-9, MMP-10, MMP-12, MMP-13 et MMP-14, de préférence est MMP-12.

De préférence, le ligand comprend un groupement de formule (I) : 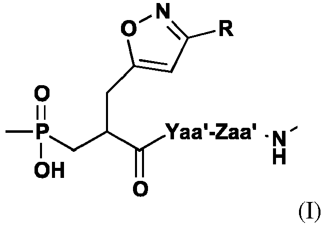 avec
Yaa' étant un acide aminé naturel autre que Asp, Pro, Gly, Cys et Gln, en particulier sélectionné parmi le groupe constitué de Ala, Arg, Asn, Glu, His, Ile, Leu, Lys, Met, Phe, Ser, Thr, Val, Trp et Tyr ;
Zaa' étant un acide aminé naturel autre que Pro et Cys, en particulier sélectionné parmi le groupe constitué de Ala, Arg, Asp, Asn, Gly, Gln, Glu, His, Ile, Leu, Lys, Met, Phe, Ser, Thr, Val, Trp et Tyr ;
R étant sélectionné parmi le groupe constitué de

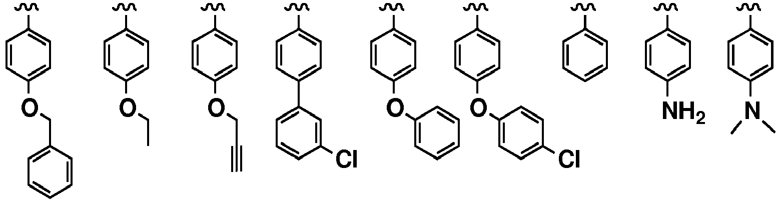

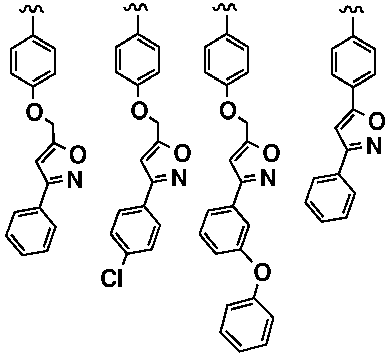

Dans un mode de réalisation préféré, le ligand comprend un groupement de formule (III)

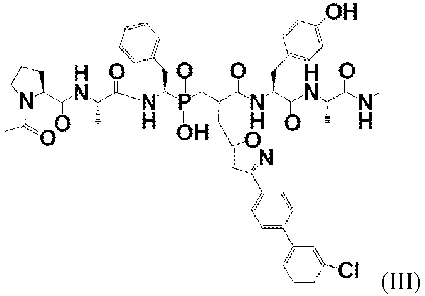

La présente invention concerne également un kit de détection spécifique d'une MMP d'intérêt uniquement dans sa forme active, comprenant
a. un anticorps spécifique de la MMP d'intérêt ;
b. un ligand de la MMP d'intérêt comprenant un inhibiteur pseudopeptidique phosphinique, de préférence fonctionnalisant une protéine porteuse ;
c. facultativement, un support solide sur lequel est immobilisé soit l'anticorps, soit le ligand ; et
d. facultativement, des réactifs permettant la détection de l'anticorps ou du ligand.

Enfin, la présente invention concerne l'utilisation d'une méthode selon l'invention ou d'un kit selon l'invention dans une méthode de diagnostic, en particulier de diagnostic du cancer, d'une infection virale, de l'arthrose, de l'arthrite rhumatoide, de la maladie de Dupuytren, de l'athérosclérose, des pathologies respiratoires à composantes inflammatoires, comme la broncho-pneumopathie chronique obstructive, l'emphysème, et l'asthme, ou de maladies neurodégénératives comme la sclérose en plaque, la myasthénie gravis, un accident vasculaire cérébral, la sclérose amyotrophique latérale ou la maladie d'Alzheimer.

### DESCRIPTION DETAILLEE DE L'INVENTION

La présente invention est relative à une méthode permettant de détecter uniquement la forme active d'une MMP d'intérêt avec une bonne sensibilité et une facilité de mise en oeuvre, y compris à partir des échantillons complexes. Plus spécifiquement, cette méthode combine d'une part l'utilisation d'un anticorps spécifique de la MMP d'intérêt, permettant ainsi la discrimination entre cette MMP d'intérêt et les autres MMP présentes dans l'échantillon, et d'autre part, l'utilisation d'un ligand de la MMP se fixant au site actif et comprenant un inhibiteur pseudopeptidique phosphinique, ce ligand permettant la discrimination entre la forme active de la MMP d'intérêt et les autres formes de la MMP d'intérêt qui peuvent être présentes dans l'échantillon (la proforme et la forme inactivée par l'inhibiteur naturel). Pour pouvoir permettre la détection spécifique de la forme active par rapport aux proformes ou formes inactivées, ce ligand est mis en présence avec l'échantillon, et ce sans étape préalable susceptible de modifier la liaison entre les MMP et les inhibiteurs naturels ou le propeptide comme des lavages, des activations ou des dénaturations. La détection du complexe ternaire entre le ligand, la MMP d'intérêt et l'anticorps spécifique permet de détecter spécifiquement la forme active de la MMP d'intérêt.

Cette méthode est novatrice car les méthodes précédemment décrites sont conçues de telle sorte qu'elles comprennent toutes des étapes de lavage entre la fixation de la MMP au support solide et la mise en présence avec le substrat ou des étapes de dénaturation. Or, ces étapes abolissent la liaison entre la MMP et les inhibiteurs naturels.

Ainsi, la présente invention concerne une méthode pour détecter dans un échantillon spécifiquement une métalloprotéase matricielle (MMP) d'intérêt uniquement dans sa forme active, comprenant
a) une étape de mise en contact de l'échantillon biologique avec un ligand de la MMP d'intérêt capable de se fixer au site actif libre de la MMP;
b) ultérieurement ou simultanément à l'étape a), une étape de mise en contact avec un anticorps spécifique de la MMP d'intérêt du résultat de l'étape a); et
c) une étape de détection du complexe ternaire entre la MMP d'intérêt, le ligand de la MMP d'intérêt, et l'anticorps spécifique de la MMP d'intérêt,
et dans laquelle le ligand comprend un inhibiteur pseudopeptidique phosphinique.

De préférence, soit le ligand de la MMP d'intérêt, soit l'anticorps spécifique de la MMP d'intérêt est immobilisé sur un support solide, et la détection est réalisée soit par la détection de l'anticorps lorsque le ligand est immobilisé sur le support, soit par la détection du ligand lorsque l'anticorps est immobilisé sur le support.

La MMP d'intérêt est de préférence sélectionnée parmi le groupe consistant en MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-26, MMP-27 et MMP-28. Dans un mode de réalisation particulier, elle est sélectionnée parmi le groupe consistant en MMP-2, MMP-3, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-26, MMP-27 et MMP-28. Dans un mode de réalisation préféré, elle est sélectionnée parmi le groupe consistant en MMP-2, MMP-3, MMP-8, MMP-9, MMP-10, MMP-12, MMP-13 et MMP-14. Dans un autre mode de réalisation préféré, elle est sélectionnée parmi le groupe consistant en MMP-2, MMP-3, MMP-8, MMP-9, MMP-12, MMP-13 et MMP-14. Dans un mode de réalisation particulièrement préféré, la MMP d'intérêt est MMP-12. La MMP d'intérêt est de préférence une MMP humaine. Cependant, elle peut provenir d'autres espèces dans lesquelles la détection de la forme active présente un intérêt diagnostique, comme pour les animaux domestiques (chien, chat ou cheval, par exemple), ou un intérêt expérimental, comme pour les animaux utilisés en laboratoire (souris ou rat, par exemple).

L'échantillon à tester est de préférence un échantillon biologique. Cet échantillon peut être par exemple, et de manière non-exhaustive, un prélèvement d'un liquide ou fluide biologique, et notamment un prélèvement sanguin, un prélèvement lymphatique, une expectoration, en particulier un lavage trachéo-bronchique, de la salive, de l'urine, de la bile, du suc pancréatique, du sperme, du fluide amniotique, du mucus, du suc gastrique, de la sueur, du liquide cérébrospinal, du liquide synovial, du liquide pleural, du liquide péritonéal, ou du liquide péricardique. Cet échantillon peut également être un extrait tissulaire, en particulier un extrait cellulaire. De préférence, l'échantillon testé est préparé à partir du prélèvement ou de l'extrait cellulaire ou tissulaire, notamment par dilution. La dilution peut être par un facteur 2, 5, 10, 20, 50 ou 100. Cependant, les MMPs étant généralement présentes en faible quantité, la dilution sera de préférence la plus faible possible pour rendre l'échantillon compatible avec la méthode mise en oeuvre dans le test. L'échantillon peut provenir d'une tumeur. L'échantillon peut être frais ou avoir été congelé.

Par définition, un échantillon est dit complexe lorsqu'il s'agit d'un prélèvement biologique ou qu'il est préparé à partir de celui-ci. La complexité de l'échantillon provient du grand nombre de protéines différentes dans des rapports de concentration variables présentes dans l'échantillon. Les concentrations de ces protéines peuvent être fortement élevées (x1000, x10000) par rapport à des cibles d'intérêt biologiques telles que les MMPs (dont les concentrations peuvent être estimées à des concentrations subnanomolaires : ≤1nM).

La première étape comprend la mise en contact de l'échantillon biologique avec un ligand de la MMP d'intérêt.

Un point important de la présente méthode est que l'échantillon biologique est mis en présence du ligand de la MMP d'intérêt sans étape préalable susceptible de modifier l'état de la MMP. Comme expliqué précédemment, les MMP peuvent être présentes dans trois états : 1) en proforme, c'est-à-dire avant le clivage du pro-peptide. Le pro-peptide est fixé dans le site actif ; 2) sous forme active, c'est-à-dire sans le pro-peptide ; et 3) sous forme inactivée, c'est-à-dire sans le pro-peptide mais avec un inhibiteur naturel des MMP lié un niveau du site actif. Les inhibiteurs naturels des MMP sont les TIMP (inhibiteurs tissulaires des métalloprotéases), notamment TIMP-1, TIMP-2, TIMP-3 et TIMP-4.

L'échantillon peut cependant subir des étapes de dilution ou d'extraction, tant que cela n'affecte pas l'état dans lequel se trouvent les MMPs dans l'échantillon. Les données expérimentales fournies ont prouvé que la méthode selon la présente invention est compatible avec les milieux utilisés pour diluer les échantillons biologiques ou pour préparer des extraits tissulaires ou cellulaires. Par contre, la méthode ne comprend pas d'étape d'immobilisation de la MMP suivie de lavage susceptible de modifier l'état de la MMP avant la mise en contact de l'échantillon avec le ligand.

Le ligand de MMP est un ligand capable de se lier au site actif libre de la MMP d'intérêt. On peut le qualifier également d'inhibiteur puisqu'il empêche durablement la fixation du substrat dans le site actif de la MMP et qu'il n'est pas clivé par la MMP. Il doit être capable de se fixer avec une très forte affinité et de maintenir cette interaction au cours des étapes de lavage. Cependant, il ne doit pas déplacer les inhibiteurs naturels liés aux MMPs. De préférence, le ligand doit présenter une constante d'inhibition Ki envers la MMP d'intérêt de 2 nanomolaires ou moins, en particulier un Ki subnanomolaire (inférieure ou égal à 1 nM, notamment un Ki inférieur à 1, 0,5 ou 0,1 nM). Par ailleurs, il est préférable de choisir des inhibiteurs ayant une spécificité pour les MMP par rapport à d'autres protéases présentes dans l'échantillon à tester. Enfin, un dernier paramètre important dans le choix des inhibiteurs est sa stabilité chimique. En effet, l'inhibiteur ne doit pas être métabolisé et sa structure ne doit pas être altérée dans l'échantillon, c'est-à-dire dans les fluides biologiques ou extraits cellulaires.

Il a été du mérite des inventeurs de définir les différents paramètres pour choisir l'inhibiteur et de choisir les inhibiteurs de type pseudopeptidique phosphinique.

En effet, de nombreux inhibiteurs de MMP ont été décrits. Certains présentent d'excellentes affinités pour les MMPs. Cependant, malgré cette affinité, ces inhibiteurs ne convenaient pas. Par exemple, une classe d'inhibiteurs bien connus est celle des inhibiteurs hydroxamates qui a été la plus développée pour mettre au point des inhibiteurs de MMP. Celle-ci n'a pourtant pas été retenue. En effet, ils sont peu spécifiques des MMPs et interagissent avec d'autres familles de métalloprotéases, comme les ADAM, ADAM-TS ou des métalloprotéases très abondantes comme la néprilysine ; cela les rend difficilement utilisables dans des échantillons complexes. Par ailleurs, ils sont très instables chimiquement puisque la fonction hydroxamate s'hydrolyse pour générer un carboxylate et des composés moins affins ; cela les rend difficilement utilisables dans des milieux complexes, notamment ceux utilisés pour préparer les extraits cellulaires.

La classe des pseudopeptides phosphiniques est connue de l'homme du métier et, par exemple, a été décrite dans Dive et al (2004, Cell Mol Life Sci, 61, 2010-2019) ; Fisher and Mobashery (2006, Cancer Metastasis Rev, 25, 115-136) ; WO00/43404, WO01/25264, WO07/062376, WO08/057254 et WO2011/0238 64.

Facultativement, le ligand de MMP peut être choisi pour présenter une spécificité pour la MMP d'intérêt par rapport aux autres MMP. Cependant, cet aspect n'est pas important dans la présente invention, le facteur important étant uniquement la bonne affinité pour la MMP d'intérêt. Dans un mode de réalisation préféré pour les ligands de MMP12, les inhibiteurs sont choisis parmi ceux décrits dans WO08/057254, WO2011/023864 ou Devel et al (2006, J Biol Chem, 281, 11152-11160).

Ainsi, le ligand comprend ou est un inhibiteur pseudopeptidique phosphinique.

Dans un mode de réalisation préféré, le ligand comprend un groupement de formule (I): 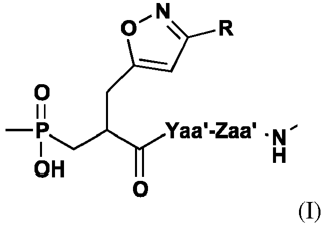 avec
Yaa' étant un acide aminé naturel autre que Asp, Pro, Gly, Cys et Gln, en particulier sélectionné parmi le groupe constitué de Ala, Arg, Asn, Glu, His, Ile, Leu, Lys, Met, Phe, Ser, Thr, Val, Trp et Tyr ;
Zaa' étant un acide aminé naturel autre que Pro et Cys, en particulier sélectionné parmi le groupe constitué de Ala, Arg, Asp, Asn, Gly, Gln, Glu, His, Ile, Leu, Lys, Met, Phe, Ser, Thr, Val, Trp et Tyr ;
R étant sélectionné parmi le groupe constitué de

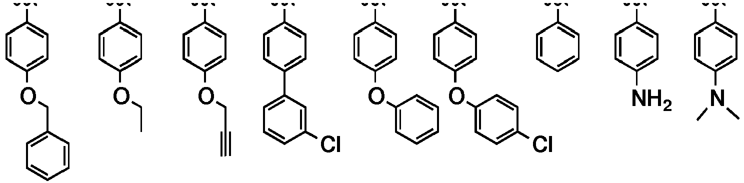

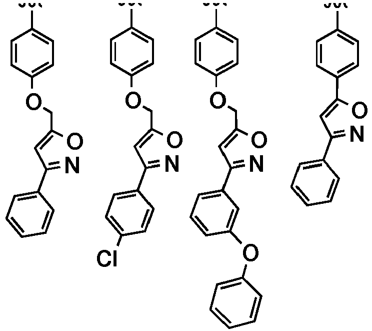

Les acides aminés définis dans le présent document sont représentés avec le symbole à trois lettres tel qu'indiqué ci-dessous:

| | | | | | |
|---|---|---|---|---|---|
| A | Ala | (alanine) | R | Arg | (arginine) |
| N | Asn | (asparagine) | D | Asp | (acide aspartique) |
| C | Cys | (cystéine) | Q | Gln | (glutamine) |
| E | Glu | (acide glutamique) | G | Gly | (glycine) |
| H | His | (histidine) | I | Ile | (isoleucine) |
| L | Leu | (leucine) | K | Lys | (lysine) |
| M | Met | (méthionine) | F | Phe | (phénylalanine) |
| P | Pro | (proline) | S | Ser | (sérine) |
| T | Thr | (thréonine) | W | Trp | (tryptophane) |
| Y | Tyr | (tyrosine) | V | Val | (valine) |

Plus particulièrement, le ligand comprend un groupement de formule (II) : 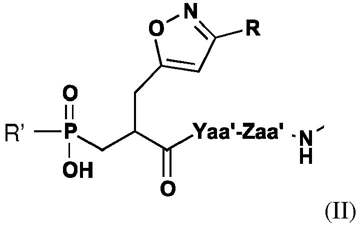 avec
R, Yaa' et Zaa' tels que définis dans la formule (I) ;
R' étant soit 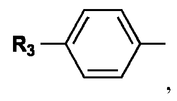 avec R3 étant H ou Br ; ou, soit 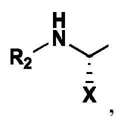 avec
X étant une chaîne latérale d'un acide aminé sélectionné parmi le groupe consistant en Gly, Phe, Ala, Val, Leu et Ileu ;
R2 étant sélectionné parmi le groupe consistant en 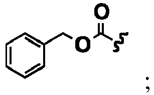 -C(=O)-Waa'-Xaa'- ; et -C(=O)-Vaa'-Waa'-Xaa'-

Vaa', Waa' et Xaa' étant un acide aminé naturel quelconque sauf Cys, en particulier sélectionné parmi le groupe constitué de Ala, Arg, Asp, Asn, Gly, Gln, Glu, His, Ile, Leu, Lys, Met, Phe, Pro, Ser, Thr, Val, Trp et Tyr.

Dans un mode de réalisation très particulier, le ligand est tel qu'il comprend un groupement de formule II dans laquelle
Yaa' étant Tyr, et Zaa' étant Ala ;
R étant 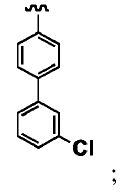 R' étant 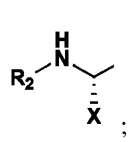 avec X étant la chaîne latéral de Phe et R2 étant -C(=O)-Waa'-Xaa'- avec Xaa' étant Ala et Waa' étant Pro.

Ainsi, le ligand comprend un groupement de formule (III) :

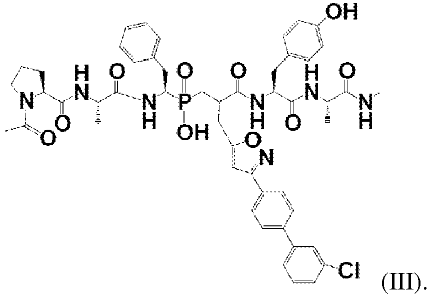

Dans un autre mode de réalisation très particulier, le ligand est tel qu'il comprend un groupement de formule II dans laquelle
Yaa' étant Tyr, et Zaa' étant Ala ;
R étant 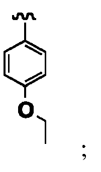 R' étant 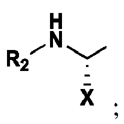 avec X étant la chaîne latéral de Phe et R2 étant -C(=O)-Waa'-Xaa'- avec Xaa' étant Ala et Waa' étant Pro.

Dans un autre mode de réalisation très particulier, le ligand est tel qu'il comprend un groupement de formule II dans laquelle

Yaa' étant Tyr, et Zaa' étant Ala ; R étant 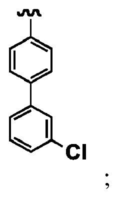 R' étant 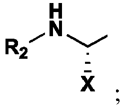 avec X étant la chaîne latéral de Phe et R2 étant -C(=O)- Vaa'-Waa'-Xaa'- avec Vaa' étant Phe, Xaa' étant Ala et Waa' étant Pro.

De préférence, le ligand inclut en outre un espaceur à l'une et/ou l'autre extrémité du groupement, en particulier à l'extrémité coté Zaa'. Facultativement, l'espaceur peut être-CH₂-C(=O)-NH-(CH₂)₃-O-(CH₂)₂-O-(CH₂)₂-O-(CH₂)₃-NH-.

Ainsi, le ligand peut comprendre un groupement de formule (III') :

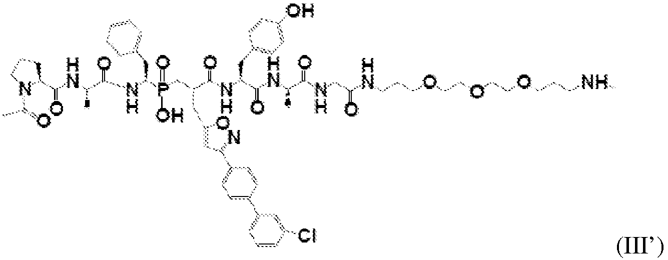

Dans un mode de réalisation préféré de la présente invention, le ligand est couplé à une protéine porteuse. Le lien entre la protéine porteuse et le ligand peut être covalent ou non-covalent.

Lorsque le lien est non-covalent, le ligand peut être immobilisé sur la protéine porteuse par un couple de molécules ayant une affinité l'une pour l'autre, par exemple un couple biotine-streptavidine ou biotine-avidine. Ainsi, le ligand est lié covalemment à une biotine, de préférence par l'intermédiaire d'un lien ou espaceur comme un polyéthylène glycol, et la protéine porteuse est liée à ou est une streptavidine. Le ligand P3 est une illustration du ligand lié à une biotine.

Lorsque le lien est covalent, la protéine porteuse fonctionnalisée avec un ligand de la MMP d'intérêt peut être toute protéine portant des acides aminés permettant la fonctionnalisation, par exemple lysine, histidine, tyrosine ou cystéine. Comme l'ont montré les exemples, la nature de cette protéine porteuse n'a pas d'impact sur la réalisation du test de détection puisque des résultats comparables ont été obtenus avec la BSA, la HSA, et l'acétylcholinestérase comme protéine porteuse.

La protéine porteuse peut être monofonctionnalisée (porte un seul ligand de MMP) ou polyfonctionnalisée (porte plusieurs ligands de MMP). Dans un mode de réalisation préféré, lorsque la technique d'immunochromatographie est mise en oeuvre avec immobilisation du ligand de MMP sur le support solide, une protéine porteuse polyfonctionnalisée sera préférée.

Le ligand de MMP est de préférence lié à la protéine porteuse à l'aide d'un lien ou espaceur. La fonction première de ce lien est d'assurer la disponibilité du ligand pour interagir avec le site actif de la MMP. De préférence, le lien présente une longueur d'au minimum environ 30 Angströms. Par exemple, le lien peut présenter une longueur de 100 à 200 Angströms. La fonction complémentaire, lorsque le ligand est particulièrement hydrophobe, est de faciliter la solubilité du ligand. On peut citer par exemple un lien/espaceur de type poly(éthylène glycol) (PEG), notamment des PEG d'au moins 10 répétitions, et en particulier de 20-40 répétitions, par exemple environ 30 répétitions. D'autres espaceurs sont bien connus de l'homme du métier et peuvent être utilisés.

De préférence, la masse moléculaire de la protéine porteuse, ou d'un monomère de la protéine porteuse lorsque celle-ci est multimérique, n'est pas trop élevé, de préférence inférieure à 100 kDa.

La protéine porteuse ne doit pas interférer avec l'activité des MMPs.

Dans un mode de réalisation, cette protéine porteuse ne présente pas d'activité enzymatique. Des exemples non-limitatifs de protéines porteuses sont une sérum albumine (notamment bovine ou humaine), une ovalbumine, une tyroglobuline, une toxoïde tétanique ou diphtérique, une hémocyanine de patelle (KLH), la protéine de liaison au maltose (MBP), et la flagelline. Dans un mode de réalisation préféré, la protéine porteuse est une sérum albumine, de préférence humaine ou bovine.

Dans un autre mode de réalisation, la protéine porteuse permet en outre la détection et peut présenter une activité enzymatique détectable. Par conséquent, elle est conjuguée directement ou indirectement à un marqueur détectable.

Par conjuguée indirectement est entendu que la protéine peut fixer un marqueur détectable, notamment via l'intermédiaire d'un couple de molécules ayant une affinité l'une pour l'autre, comme un couple biotine-streptavidine. Ainsi, la protéine porteuse peut avoir une ou plusieurs biotines attachées à sa surface et la streptavidine sera conjuguée au marqueur détectable.

Par conjuguée directement est entendu que la protéine présente à sa surface des marqueurs détectables. Ces marqueurs détectables peuvent être par exemple sélectionnés parmi le groupe consistant en un métal colloïdal, un colloïde non-métallique, du carbone, un traceur visible, fluorescent, luminescent ou chimio luminescent, une particule magnétique, un élément radioactif, des billes de latex portant un traceur visible ou fluorescent, et une enzyme dont l'activité peut être détectée. De préférence, le marqueur détectable est l'or colloïdal ou une enzyme. Les enzymes pouvant être utilisées dans ce type d'application sont bien connues de l'homme du métier, et à titre d'illustration, nous citons une acétylcholinestérase ou une peroxydase.

Dans un mode de réalisation particulier, la protéine porteuse est l'enzyme utilisée comme marqueur détectable. Les enzymes utilisées ou utilisables dans les tests immunoenzymatiques (EIA) ou ELISA sont bien connues de l'homme du métier et peuvent être mise en oeuvre dans la présente invention comme protéines porteuses. Ce mode de réalisation est d'intérêt, en particulier lorsque la protéine porteuse fonctionnalisée par le ligand de MMP est utilisée pour la détection de la MMP d'intérêt immobilisée sur le support dans le contexte d'un EIA.

Les méthodes de couplages (ou de fonctionnalisation) du ligand de MMP à la protéine porteuse sont bien connues de l'homme du métier. On peut citer à titre d'exemples le couplage par le carbodiimide, celui par le glutaraldéhyde, par la benzidine bis-diazotée, ou par un maléimide.

L'étape suivante, qui peut être ultérieure ou simultanée à l'étape a), est une étape de mise en contact avec un anticorps spécifique de la MMP d'intérêt du résultat de l'étape a).

L'anticorps doit être capable de discriminer la MMP d'intérêt des autres MMPs présentes éventuellement dans l'échantillon à tester. C'est grâce à la spécificité de l'anticorps que la méthode permet de détecter spécifiquement la MMP d'intérêt.

L'anticorps spécifique de la MMP d'intérêt peut être polyclonal ou monoclonal. Il peut être un IgG, IgM ou IgA, de préférence un IgG. L'anticorps doit se lier à la MMP de sorte à ne pas interférer avec la liaison de la MMP au ligand, c'est-à-dire ne pas se lier au site actif du domaine catalytique de la MMP. Il peut cependant se lier au domaine catalytique, mais en dehors du site actif de celui-ci.

De nombreux anticorps dirigés contre les MMPs sont disponibles commercialement pour chaque MMP, par exemple chez Sigma-Aldrich, R&D, Millipore, Aviva, GeneTex, MyBioSource.com, GenWay Biotech, Inc., Thermo Scientific Pierce Antibodies, Acris Antibodies GmbH, Cosmo Bio Co., Ltd, LifeSpan BioSciences.

Enfin, la méthode comprend une étape de détection du complexe ternaire entre la MMP d'intérêt, le ligand de la MMP d'intérêt, et l'anticorps spécifique de la MMP d'intérêt. La détection de ce complexe ternaire peut être réalisée via la détection de l'anticorps ou via la détection du ligand.

Dans un premier mode de réalisation préféré, le complexe ternaire est détecté par la détection de l'anticorps. Cet anticorps peut être marqué directement ou indirectement, de préférence directement. Par exemple, il peut être conjugué à des marqueurs détectables, par exemple des enzymes, des particules d'or ou des marqueurs colorés, fluorescents ou radioactifs. Alternativement, l'anticorps peut être détecté par des anticorps secondaires conjugués à des marqueurs détectables, notamment des anticorps dirigés contre l'anticorps spécifique de la MMP. Par exemple, si l'anticorps spécifique de la MMP est une immunoglobuline de souris, l'anticorps secondaire sera spécifique des immunoglobulines de souris. En outre, l'anticorps peut être conjugué à un membre d'un couple de molécules ayant une affinité l'une pour l'autre, par exemple conjugué à une biotine. L'autre membre, en particulier la streptavidine est conjuguée au marqueur détectable.

Dans un premier mode de réalisation alternatif, le complexe ternaire est détecté par la détection du ligand. Celle-ci peut se faire directement lorsque le ligand est conjugué à un marqueur détectable ou lorsque la protéine porteuse est une enzyme dont l'activité est détectable. Elle peut également se faire indirectement lorsque le ligand ou la protéine porteuse est conjugué à un membre d'un couple de molécules ayant une affinité l'une pour l'autre, par exemple conjugué à une biotine. L'autre membre, en particulier la streptavidine est conjuguée au marqueur détectable. Une dernière option est d'utiliser des anticorps dirigés contre la protéine porteuse pour le marquage.

La méthode comprend de préférence une immobilisation du complexe ternaire sur un support solide. Le support solide peut être une surface, une bille ou un tube. Le support solide peut être tout support compatible avec un test immunologique. De nombreux matériaux peuvent être utilisés comme support, notamment la nitrocellulose, le nylon, l'acétate de cellulose, les fibres de verre, ou d'autres polymères poreux.

L'immobilisation se fait soit par l'intermédiaire du ligand, soit par l'intermédiaire de l'anticorps. Lorsque l'immobilisation de la MMP a été faite par l'intermédiaire du ligand, alors la détection du complexe ternaire se fait par l'intermédiaire de la détection de l'anticorps. A contrario, lorsque l'immobilisation de la MMP a été faite par l'intermédiaire de l'anticorps, alors la détection du complexe ternaire se fait par l'intermédiaire de la détection du ligand.

Ainsi, dans un premier mode de réalisation particulier préféré où l'immobilisation du complexe ternaire se fait par l'intermédiaire du ligand, la méthode comprend
a) la fourniture d'un support solide sur lequel est immobilisé un ligand de la MMP d'intérêt ;
b) la mise en contact du support solide avec l'échantillon de manière à permettre la fixation de la MMP d'intérêt présente dans l'échantillon au support solide par l'intermédiaire d'une liaison entre le ligand et la MMP d'intérêt ;
c) facultativement, l'élimination des MMPs non fixées et autres protéines de l'échantillon ;
d) l'ajout d'un anticorps spécifique de la MMP d'intérêt de manière à permettre la formation du complexe ternaire entre la MMP d'intérêt, le ligand de la MMP d'intérêt, et l'anticorps spécifique de la MMP d'intérêt ;
e) l'élimination des anticorps libres ; et
f) la détection des anticorps dans le complexe ternaire, la détection de ceux-ci étant indicatif de la présence dans l'échantillon de la MMP d'intérêt sous forme active.

Les étapes c) et e) peuvent être réalisées par des étapes de lavage.

Facultativement, les étapes b) et d) peuvent être inversées. Ainsi, l'échantillon est mis en contact avec l'anticorps, puis ce mélange est mis en contact avec le support solide. De manière alternative, les étapes b) et d) peuvent être simultanées. Ainsi, l'échantillon et l'anticorps sont mis en contact avec le support solide.

Dans ce mode de réalisation, le ligand peut être immobilisé directement sur le support solide, éventuellement par l'intermédiaire d'un lien/espaceur. De préférence, le ligand est lié à une protéine porteuse qui est immobilisée sur le support solide. La protéine porteuse peut être mono- ou poly-fonctionnalisée avec le ligand. Dans un mode de réalisation particulier, la protéine porteuse est une sérum albumine, en particulier humaine ou bovine.

Ainsi, la présente invention concerne un support solide sur lequel un ligand tel que défini ci-dessus a été immobilisé. De préférence, le ligand est lié à une protéine porteuse qui est immobilisée sur le support solide. La protéine porteuse peut être mono- ou poly-fonctionnalisée avec le ligand. Dans un mode de réalisation particulier, la protéine porteuse est une sérum albumine, en particulier humaine ou bovine. Le support solide peut être une bandelette (adaptée à l'immunochromatographie), un puits d'une plaque de microtitration, un filtre ou une membrane, ou une bandelette (« dipstick »).

La présente invention concerne en particulier deux types de test.

Le premier est un test d'immunochromatographie. Cette méthode est bien connue de l'homme du métier. Le support est de préférence une bandelette adaptée à la chromatographie. Le ligand est immobilisé sur la zone de test. Dans un mode de réalisation préféré, une protéine porteuse polyfonctionnalisée avec le ligand (portant plusieurs ligands) est immobilisée. Alternativement, une protéine porteuse monofonctionnalisée avec le ligand (portant un ligand) peut aussi être immobilisée. De préférence, le support comprend en outre une zone de contrôle, cette zone portant un moyen de fixation des anticorps anti-MMP, par exemple des anticorps dirigés contre les anticorps anti-MMP. Par exemple, si l'anticorps anti-MMP est un anticorps de souris, on utilisera un anticorps spécifique des anticorps de souris. Il en est de même pour les anticorps de lapin, de rat ou de chèvre. L'échantillon est mis en contact avec le ligand par dépôt de l'échantillon du coté de la zone de dépôt d'échantillon, puis migration de celui-ci. Eventuellement, un ou plusieurs lavages sont ensuite faits. La MMP active, présentant initialement le site actif libre, est immobilisée sur la zone de test par interaction avec le ligand. Les autres formes sont éliminées de la zone test, car non-retenues sur cette zone. Les données expérimentales fournies ont montré que ce système permet l'immobilisation sélective des formes actives de la MMP, la proforme et la forme désactivée par les inhibiteurs n'étant pas retenues, et donc pas détectées ; et ce y compris dans divers milieux et dans des échantillons complexes. La MMP immobilisée sur la zone de test est ensuite détectée par ajout d'un anticorps spécifique de la MMP d'intérêt. Cet anticorps peut être marqué directement ou indirectement, de préférence directement. Ce mode de réalisation a été prouvé être spécifique de la forme active, sensible (environ 1,2 ng/ml dans un tampon) et compatible avec des milieux complexes (sensibilité de 10-20 ng/ml dans des échantillons complexes).

De manière alternative, l'échantillon peut être placé sur la bandelette au niveau de la zone de dépôt. Il migre le long de la bandelette et est mis en présence d'une zone présentant l'anticorps anti-MMP capable de migrer (pas immobilisé sur le support solide). L'échantillon, ainsi que l'anticorps, migrent ensuite le long de la bandelette jusqu'aux zones de test et de contrôle.

Il est également envisagé de détecter plusieurs MMPs d'intérêt différentes. Ainsi, le ligand est choisi de sorte à être compatible (c'est-à-dire capable de se fixer à) avec les MMPs d'intérêt et chaque bandelette est utilisée avec un anticorps spécifique d'une des MMPs d'intérêt différentes. Par exemple, si deux MMPs sont à détecter, il y aura une bandelette utilisée avec un anticorps spécifique de la première MMP et une autre utilisée avec un anticorps spécifique de la deuxième MMP.

L'invention est également relative à un kit permettant de réaliser le test. Ce kit comprend :
- une bandelette comprenant une zone de test sur laquelle est immobilisé le ligand de la MMP d'intérêt. De préférence, le ligand est couplé à une protéine porteuse, en particulier via un lien ou espaceur. La protéine porteuse est de préférence polyfonctionnalisée avec le ligand. De préférence, la bandelette comprend en outre un zone contrôle sur laquelle est immobilisé un moyen de fixation des anticorps anti-MMP. La bandelette comprend en outre une zone de dépôt de l'échantillon, et facultativement une zone portant les anticorps anti-MMP en aval de celle-ci et avant les zones de test.
- un anticorps spécifique de la MMP d'intérêt, de préférence marqué par une particule colorée (par exemple, particule d'or ou de latex), un fluorophore ou un radioélément ; et
- facultativement, des réactifs comme des tampons de lavage, de dilution ou d'extraction.

Lorsque plusieurs MMPs d'intérêt différentes sont détectées, le kit comprend au moins une bandelette par MMP d'intérêt et comprend en outre un anticorps spécifique par MMP d'intérêt. Par exemple, si deux MMPs sont à détecter, il y aura au moins un anticorps spécifique de la première MMP et un autre spécifique de la deuxième. Le kit pourra contenir un type unique de bandelettes et autant d'anticorps spécifiques que de MMP d'intérêts.

Le second est un test immunologique en phase solide, de préférence un test immunoenzymatique. Le support solide peut être notamment une membrane ou un filtre (« Flow-through »), un puits de plaque de microtitration (par exemple, EIA) ou des bandelettes (« dipstick »). Le ligand est immobilisé sur le support solide. Dans un mode de réalisation préféré, une protéine porteuse monofonctionnalisée avec le ligand (portant un ligand) est immobilisée. Alternativement, une protéine porteuse polyfonctionnalisée avec le ligand (portant plusieurs ligands) peut aussi être immobilisée. L'échantillon est ensuite mis en contact avec le ligand immobilisé. Après incubation, un ou plusieurs lavages sont éventuellement réalisés. Les données expérimentales fournies ont montré que ce système permet l'immobilisation sélective des formes actives de la MMP, la proforme et la forme désactivée par les inhibiteurs n'étant pas retenues, et donc non détectées ; et ce y compris dans divers milieux et dans des échantillons complexes. L'anticorps spécifique de la MMP d'intérêt est ensuite ajouté et un ou plusieurs lavages sont éventuellement réalisés avant détection de l'anticorps. L'anticorps est ensuite détecté.

Lorsque le support solide est une membrane ou un filtre (« Flow-through »), le ligand est immobilisé sur la membrane ou le filtre, ainsi que de préférence le moyen de contrôle (moyen de fixation des anticorps), sous forme de sites distincts. L'échantillon est ensuite appliqué sur la membrane ou le filtre et passe à travers la membrane ou le filtre, notamment par capillarité. L'anticorps est ensuite appliqué pour révéler la présence du complexe ligand-MMP. Des étapes de lavages peuvent également être ajoutées avant ou après l'ajout de l'anticorps.

Lorsque le support solide est un puits de plaque de microtitration, un test immunoenzymatique sera préféré pour la détection de l'anticorps. Cet anticorps peut être directement couplé à un marqueur détectable, de préférence une enzyme, ou être indirectement couplé à celui-ci via un couple biotine-streptavidine ou un anticorps secondaire couplé au marqueur. Ces techniques sont bien connues de l'homme du métier. L'enzyme peut être par exemple une péroxydase ou une acétylcholinestérase. Ce mode de réalisation a été prouvé être spécifique de la forme active, sensible (environ 20 pg/ml dans un tampon) et compatible avec des milieux complexes (sensibilité de 200 pg/ml dans des échantillons complexes).

Enfin, lorsque le support solide est une bandelette (« dipstick »), le support solide est de préférence un support non-poreux. Le ligand est immobilisé sur le support en un site distinct. De préférence, un moyen de contrôle (moyen de fixation des anticorps) est également immobilisé en un site distinct. La bandelette (« dipstick ») est alors plongée dans l'échantillon, puis dans une solution d'anticorps spécifique de la MMP à détecter. De préférence, entre les étapes, la bandelette (« dipstick ») est trempée dans des solutions de lavage. Ensuite, la présence de l'anticorps est détectée.

Dans ce mode de réalisation, il est également envisagé de détecter plusieurs MMPs d'intérêt différentes. Ainsi, le ligand est choisi de sorte à être compatible (c'est-à-dire capable de se fixer à) avec les MMPs d'intérêt et plusieurs anticorps spécifiques de chacune des MMPs d'intérêt différentes sont utilisés. Par exemple, si deux MMPs sont à détecter, il y aura un support solide utilisé avec un anticorps spécifique de la première MMP et un autre utilisé avec un anticorps spécifique de la deuxième. Si le support solide est un puits de plaque de microtitration, deux puits distincts seront utilisés. Si le support solide est une membrane, un filtre ou une bandelette, deux supports solides de même type seront mis en oeuvre.

L'invention est également relative à un kit permettant de réaliser le test. Ce kit comprend :
- un support solide sur lequel a été immobilisé le ligand de la MMP d'intérêt. De préférence, le ligand est couplé à une protéine porteuse, en particulier via un lien ou espaceur. Facultativement, il comprend en outre l'immobilisation d'un moyen de fixation de l'anticorps sur un site distinct de celui où est immobilisé le ligand, pour la réalisation d'un contrôle.
- un anticorps spécifique de la MMP d'intérêt. L'anticorps peut être directement couplé à un marqueur détectable, par exemple une enzyme. Alternativement, il peut être couplé à une molécule permettant la liaison non-covalente au marqueur détectable, par exemple une biotine ; et
- facultativement, des réactifs comme un marqueur détectable, par exemple l'enzyme, couplé à une molécule permettant la liaison à l'anticorps comme un anticorps secondaire ou une streptavidine, le substrat formant un produit coloré ou fluorescent après action de l'enzyme, et/ou des tampons de lavage, de dilution ou d'extraction.

Lorsque plusieurs MMPs d'intérêt différentes sont détectées, le kit comprend un anticorps spécifique par MMP d'intérêt. Par exemple, si deux MMPs sont à détecter, le kit comprend un anticorps spécifique de la première MMP et un anticorps spécifique de la deuxième.

Dans un deuxième mode de réalisation particulier alternatif où l'immobilisation du complexe ternaire se fait par l'intermédiaire de l'anticorps, la méthode comprend
a. la fourniture d'un support solide sur lequel est immobilisé un anticorps spécifique de la MMP d'intérêt ;
b. la mise en contact du support solide avec l'échantillon qui est préalablement ou simultanément mis en contact avec un ligand de la MMP d'intérêt, de manière à permettre la fixation de la MMP d'intérêt présente dans l'échantillon au ligand et l'immobilisation de la MMP d'intérêt au support solide par l'intermédiaire d'une liaison entre l'anticorps et la MMP d'intérêt;
c. facultativement, l'élimination des ligands et MMP libres et autres protéines de l'échantillon; et
d. la détection du ligand dans le complexe ternaire entre la MMP d'intérêt, le ligand de la MMP d'intérêt, et l'anticorps spécifique de la MMP d'intérêt, la détection de celui étant indicatif de la présence dans l'échantillon de la MMP d'intérêt sous forme active.

Dans ce mode de réalisation, comme expliqué précédemment, il est crucial que l'échantillon soit mis en présence du ligand avant ou simultanément à toute étape d'immobilisation de MMP sur le support solide et lavage.

La présente invention concerne donc également un ligand de MMPs tel que décrit ci-dessus couplé à un marqueur détectable. De préférence, elle concerne une protéine porteuse fonctionnalisée avec le ligand. Dans un mode de réalisation, la protéine porteuse est une enzyme dont l'activité est détectable. Alternativement, la protéine porteuse est couplée à un marqueur détectable. Dans un mode de réalisation particulier, la protéine porteuse est une sérum albumine, en particulier humaine ou bovine.

La présente invention concerne en particulier deux types de test.

Le premier est un test d'immunochromatographie. Dans ce mode de réalisation, l'anticorps spécifique de la MMP d'intérêt est immobilisé sur la zone de test. L'échantillon est incubé avec le ligand. De préférence, le ligand est lié à une protéine porteuse. La protéine porteuse peut être mono- ou poly-fonctionnalisée avec le ligand. Ensuite, l'échantillon mis en présence avec le ligand est déposé du coté de la zone de dépôt d'échantillon et la migration a lieu. Facultativement, un ou plusieurs lavages sont ensuite faits.

De manière alternative, l'échantillon peut être déposé sur la bandelette au niveau d'une zone de dépôt. Il est mis en présence du ligand après migration de l'échantillon jusqu'à une zone portant le ligand en aval mais avant la zone de test. L'ensemble migre ensuite vers la zone de test et entre en contact avec l'anticorps anti-MMP immobilisé sur le support solide.

Le complexe ternaire anticorps-MMP-ligand est détecté via la détection du ligand. En effet, dans cette configuration, toutes les formes de la MMP d'intérêt sont retenues dans la zone test. Cependant, seules les formes actives de celle-ci sont détectées puisque la détection se fait par l'intermédiaire du ligand qui se fixe uniquement à la forme active, la seule à présenter un site actif disponible. Pour permettre la détection du ligand, celui-ci est directement ou indirectement couplé à un marqueur détectable. Lorsque l'on utilise une protéine porteuse fonctionnalisée avec le ligand, il est également possible d'utiliser pour la détection un anticorps marqué spécifique de la protéine porteuse.

De préférence, le support solide comprend également une zone contrôle permettant de fixer le ligand. Par exemple, une MMP capable de lier le ligand peut être immobilisée sur le support solide.

Dans ce mode de réalisation, il est également envisagé de détecter plusieurs MMPs d'intérêt différentes. Ainsi, le ligand est choisi de sorte à être compatible (c'est-à-dire capable de se fixer à) avec les MMPs d'intérêt. Chaque bandelette peut être utilisée avec un anticorps spécifique à une des MMPs d'intérêt différentes immobilisé sur la zone de test. Par exemple, si deux MMPs sont à détecter, il y aura une bandelette présentant une zone de test sur laquelle est immobilisé un anticorps spécifique de la première MMP et une autre présentant une zone de test sur laquelle est immobilisé un anticorps spécifique de la deuxième. Alternativement, la bandelette comprend des zones distinctes de test, sur lesquelles un anticorps présentant une spécificité différente est immobilisé. Ainsi, si deux MMPs sont à détecter, il y aura une bandelette avec deux zones de test, une première sur laquelle est immobilisé un anticorps spécifique de la première MMP et une autre présentant une zone de test sur laquelle est immobilisé un anticorps spécifique de la deuxième.

L'invention est également relative à un kit permettant de réaliser le test. Ce kit comprend :
- une bandelette comprenant une zone de test sur laquelle est immobilisé un anticorps spécifique de la MMP d'intérêt et de préférence, une zone de contrôle sur laquelle est immobilisé un moyen de fixation du ligand, par exemple une MMP ;
- le ligand de la MMP d'intérêt, de préférence marqué par une particule colorée (par exemple, particule d'or ou de latex), un fluorophore ou un radioélément. De préférence, le ligand est couplé à une protéine porteuse, en particulier via un lien ou espaceur ; et
- facultativement, des réactifs comme des tampons de lavage, de dilution ou d'extraction.

Lorsque plusieurs MMPs d'intérêt différentes sont détectées, le kit peut comprendre une bandelette par MMP d'intérêt sur laquelle un anticorps spécifique d'une des MMPs d'intérêt a été immobilisé. Par exemple, si deux MMPs sont à détecter, le kit comprend une bandelette présentant une zone de test sur laquelle est immobilisé un anticorps spécifique de la première MMP et une autre présentant une zone de test sur laquelle est immobilisé un anticorps spécifique de la deuxième. Alternativement, le kit peut comprendre une bandelette comprenant plusieurs zones de test sur lesquelles un anticorps spécifique d'une des MMPs d'intérêt a été immobilisé. Par exemple, si deux MMPs sont à détecter, le kit comprend une bandelette présentant deux zones de test, une première sur laquelle est immobilisé un anticorps spécifique de la première MMP et une autre zone de test sur laquelle est immobilisé un anticorps spécifique de la deuxième.

Le second est un test immunologique en phase solide, de préférence un test immunoenzymatique. La phase solide peut être notamment une membrane ou un filtre (« Flow-through »), un puits de plaque de microtitration (par exemple, EIA) ou des bandelettes (« dipstick »). L'anticorps est immobilisé sur le support solide. Soit l'échantillon est pré-incubé en présence du ligand avant la mise en contact avec l'anticorps immobilisé sur le support solide, soit l'échantillon est mis en contact avec l'anticorps immobilisé sur le support solide simultanément au ligand. Dans un mode de réalisation préféré, l'échantillon est pré-incubé en présence du ligand avant la mise en contact avec l'anticorps immobilisé. De préférence, le ligand est lié à une protéine porteuse. La protéine porteuse peut être mono- ou poly-fonctionnalisée avec le ligand. Ensuite, un ou plusieurs lavages peuvent être réalisés. Finalement, la présence du ligand sur le support solide est détectée. De même que précédemment, toutes les formes de la MMP d'intérêt sont retenues sur le support solide, et seules les formes actives de celle-ci sont détectées car la détection se fait par l'intermédiaire du ligand qui se fixe uniquement à la forme active. Dans un mode de réalisation particulier, la protéine porteuse fonctionnalisée est un marqueur détectable, par exemple une enzyme capable de générer un signal coloré ou fluorescent suite à l'hydrolyse de son substrat. Dans un autre mode de réalisation, le ligand est couplé, directement ou indirectement via la protéine porteuse, à un membre d'un couple de molécules ayant une affinité l'une pour l'autre, notamment à un membre d'un couple biotine-streptavidine ou biotine-avidine, en particulier à une biotine. L'autre membre du couple est lié à un marqueur détectable.

Lorsque le support solide est une membrane ou un filtre (« Flow-through »), l'anticorps est immobilisé sur la membrane ou le filtre, ainsi que de préférence le moyen de contrôle (moyen de fixation du ligand comme une MMP), sous forme de sites distincts. L'échantillon, préalablement mis en présence du ligand, est ensuite appliqué sur la membrane ou le filtre et passe à travers la membrane ou le filtre, notamment par capillarité. Le ligand immobilisé sur la membrane ou le filtre est ensuite détecté. Des étapes de lavages peuvent également être ajoutées avant ou après l'ajout de l'échantillon.

Lorsque le support solide est un puits de plaque de microtitration, un test immunoenzymatique sera préféré pour la détection du ligand. Le ligand, ou la protéine porteuse, peut être directement couplé à un marqueur, de préférence une enzyme, ou être indirectement couplé à celui-ci via un couple biotine-streptavidine ou un anticorps secondaire couplé au marqueur détectable. Ces techniques sont bien connues de l'homme du métier. L'enzyme peut être par exemple une péroxydase ou une acétylcholinestérase.

Enfin, lorsque le support solide est une bandelette (« dipstick »), le support solide est de préférence un support non-poreux. L'anticorps est immobilisé sur le support en un site distinct. De préférence, un moyen de contrôle (moyen de fixation du ligand comme une MMP) est également immobilisé en un site distinct. La bandelette (« dipstick ») est alors plongée dans l'échantillon en présence du ligand ou pré-incubé avec celui-ci au préalable, puis le ligand immobilisé sur le support est détecté. De préférence, entre les étapes, la bandelette (« dipstick ») est trempée dans des solutions de lavage.

Dans ce mode de réalisation, il est également envisagé de détecter plusieurs MMPs d'intérêt différentes. Ainsi, le ligand est choisi de sorte à être compatible (c'est-à-dire capable de se fixer à) avec les MMPs d'intérêt et un ensemble de puits ou de zones de test distinctes présente un anticorps immobilisé spécifique à une des MMPs d'intérêt différentes à détecter. Par exemple, si deux MMPs sont à détecter, il y aura une zone de test avec un anticorps spécifique de la première MMP et une autre avec un anticorps spécifique de la deuxième. Alternativement, des supports solides distincts peuvent être mis en oeuvre pour chacune des MMPs à détecter.

L'invention est également relative à un kit permettant de réaliser le test. Ce kit comprend :
- un support solide comprenant une zone de test sur lesquelles a été immobilisé l'anticorps spécifique de la MMP d'intérêt. De préférence, le support solide comprend en outre une zone de contrôle sur laquelle a été immobilisé un moyen de fixation du ligand, par exemple une MMP.
- un ligand de la MMP d'intérêt. De préférence, le ligand est couplé à une protéine porteuse, en particulier via un lien ou espaceur. La protéine porteuse peut être un marqueur détectable, de préférence une enzyme. Alternativement, le ligand peut être couplé à une molécule permettant la liaison non-covalente au marquer, par exemple une biotine ; et
- facultativement, des réactifs comme l'enzyme couplée à une molécule permettant la liaison au ligand comme un anticorps spécifique de la protéine porteuse ou une streptavidine, le substrat formant un produit coloré ou fluorescent après action de l'enzyme, et/ou des tampons de lavage, de dilution ou d'extraction.

Lorsque plusieurs MMPs d'intérêt différentes sont détectées, le kit comprend un ensemble de zones de test présentant un anticorps immobilisé spécifique d'une des MMPs d'intérêt différentes à détecter. Par exemple, si deux MMPs sont à détecter, il y aura une zone de test avec un anticorps spécifique de la première MMP et une autre avec un anticorps spécifique de la deuxième.

Dans un mode de réalisation des méthodes et kits tout particulièrement préféré, la MMP d'intérêt est le MMP12, de préférence la MMP12 humaine.

Dans des modes de réalisation particulier, la méthode permet en outre de quantifier la forme active d'une MMP spécifique.

Etant donné qu'une dérégulation de l'activité des MMPs constitue une part significative des mécanismes pathogéniques associés à de nombreuses maladies, les méthodes et kits de la présente invention peuvent être utilisés pour le diagnostic. Ces maladies comprennent par exemple la destruction des cartilages et os dans l'arthrite rhumatoïde et dans l'ostéo-arthrite, le remodelage tissulaire lors de la croissance tumorale invasive ou l'angiogenèse tumorale, la dégradation de la protéine de base de la myéline dans les maladies neuroinflammatoires, l'ouverture de la barrière hémato-encéphalique après une lésion du cerveau, le renouvellement matricielle augmentée dans les lésions sténotiques, la perte de tonus de la paroi aortique dans les anévrismes, la dégradation tissulaire dans le ulcération gastrique, les fibroses hépatiques, l'affaissement des tissus conjonctifs dans les maladies parodontales, les lésions pulmonaires aigües et les syndromes de détresse respiratoire aigües.

Ainsi, la présente invention concerne l'utilisation d'une méthode selon l'invention ou d'un kit selon l'invention dans une méthode de diagnostic, en particulier de diagnostic du cancer, d'une infection virale, de l'arthrose, de l'arthrite rhumatoïde, de la maladie de Dupuytren, de l'athérosclérose, des pathologies respiratoires à composantes inflammatoires, comme la broncho-pneumopathie chronique obstructive, l'emphysème, et l'asthme, ou de maladies neurodégénératives comme la sclérose en plaque , la myasthénie gravis, un accident vasculaire cérébral, la sclérose amyotrophique latérale ou la maladie d'Alzheimer.

Par exemple, la MMP-1 sera utilisée pour la sclérose amyotrophique latérale ; la MMP-2 pour le cancer, la maladie de Dupuytren, la myasthénie gravis, ou la sclérose amyotrophique latérale ; la MMP-3 pour la sclérose en plaque , les accidents vasculaires cérébraux, ou la maladie d'Alzheimer ; la MMP-8 pour l'asthme ou la sclérose en plaque ; la MMP-9 pour le cancer, la sclérose en plaque , les accidents vasculaires cérébraux, ou la maladie d'Alzheimer; la MMP-10 pour la maladie d'Alzheimer; la MMP-12 pour l'athérosclérose ou des pathologies respiratoires à composantes inflammatoires, comme la BPCO ; la MMP-13 pour l'arthrose ou l'arthrite rhumatoïde ; et MMP-14 pour la maladie de Dupuytren.

D'autres aspects et avantages de la présente invention apparaîtront à la lecture des exemples qui suivent, qui doivent être considérés comme illustratifs et non limitatifs.

### DESCRIPTION DES FIGURES

**Figure 1** **:** Détection de MMP12h sur bandelettes couplée à l'utilisation du traceur OR-mAb12h. Bandelette BSA polyfonctionnalisée avec P1 en présence (1) ou en absence (2) de MMP12h, (3) Bandelette BSA non-fonctionnalisée avec P1 en présence de MMP12h, (4) Bandelette BSA polyfonctionnalisée avec P1 en présence d'une proforme de MMP12h, (5) et (6) Bandelette BSA polyfonctionnalisée avec P1 en présence de MMP12h préalablement incubée avec un inhibiteur compétitif de MMP12h à une concentration de 100 nM (°) ou 1 µM (°°).
**Figure 2** **:** Détection de MMP12h sur bandelettes BSA polyfonctionnalisée avec P1 couplée à l'utilisation du traceur OR-mAb12h en faisant varier la concentration de MMP12h dans l'échantillon, ainsi qu'en testant deux tampons : (7)-(11) : tampon Tris-HCl 50 mM pH6,8, CaCl₂ 10 mM, brij 0,01% ; (12)-(14) : mélange 50/50 de tampon Tris-HCl 50 mM pH6,8, CaCl₂ 10 mM, brij 0,01% et de tampon Tris-HCl 100mM pH 7,4, 0,5% tween-20, 1% Chaps, 0,1%NaN₃.
**Figure 3** **:** Détection de MMP12h sur bandelettes BSA polyfonctionnalisée avec P1 couplée à l'utilisation du traceur OR-mAb12h en faisant varier la nature de la MMP dans l'échantillon.
**Figure 4** **:** Détection de MMP12h dans des mélanges de MMPs sur bandelettes BSA polyfonctionnalisée avec P1 couplée à l'utilisation du traceur OR-mAb12h.
**Figure 5** **:** Détection de MMP12h dans des homogénats de protéines cytosoliques sur bandelettes BSA polyfonctionnalisée avec P1 couplée à l'utilisation du traceur OR-mAb12h.
**Figure 6** **:** Détection de MMP12h dans des lavages broncho-alvéolaires (BAL) sur bandelettes BSA polyfonctionnalisée avec P1 couplée à l'utilisation du traceur OR-mAb12h.
**Figure 7** **:** Détection de MMP13h sur bandelettes BSA polyfonctionnalisée avec P1 couplée à l'utilisation du traceur OR-mAbh.
**Figure 8** **:** Détection de MMP12m sur bandelettes BSA polyfonctionnalisée avec P1 couplée à l'utilisation du traceur OR-polyAb12m, en milieu tamponné ou au sein d'un mélange protéique.
**Figure 9****:** Détection de MMP12h sur bandelettes BSA-P1 (42), HSA-P2 (43) ou mAb12h (44)-(48) en présence (42)-(44), (46) et (48) ou en absence (45) et (47) de MMP12h.
**Figure 10** **:** Détection de MMP12h par EIA sur plaque BSA-P1 avec des concentrations croissantes de MMP12h, soit dans un tampon avec ou sans préincubation de la MMP12h avec 1 µM d'inhibiteur compétitif de MMP, soit présence d'un mélange des protéines cytosoliques.
**Figure 11** **:** Détection de MMP12h par EIA sur plaque BSA-P1 en testant trois milieux tamponnés, en présence ou absence d'1 µM d'inhibiteur compétitif de MMP.
**Figure 12** **:** Détection de MMP12h par EIA sur plaque BSA-P1, HSA-P2 ou mAb12h en présence de concentration croissante de MMP12h d'un mélange de protéines cytosoliques, avec ou sans préincubation de l'échantillon avec 1 µM d'inhibiteur compétitif de MMP.
**Figure 13** **:** Détection de MMP12h par EIA sur plaque mAb12h en présence de tampon, de MMP12h active ou de MMP12h inactivée, et à gauche : avec incubation préalable de l'échantillon dans le puits avant ajout de la biot-HSA-P2 ; ou à droite : avec incubation simultanée de l'échantillon et de la biot-HSA-P2 dans le puits.
**Figure 14** **:** Détection de MMP12h par EIA sur plaque HSA-P2 ou mAb12h en présence de MMP12h ou de ProMMP12h. Pour les plaques mAb12h, le traceur Biot-HSA-P2 a été ajouté soit après fixation de MMP12h sur la plaque, soit dans l'échantillon avant fixation de MMP12h sur la plaque.
**Figure 15** **:** Détection de MMP12h par EIA sur plaque HSA-P2 dans des lavages broncho-alvéolaires (BAL) comprenant des concentrations croissantes de MMP12h, avec ou sans préincubation de l'échantillon avec un inhibiteur compétitif de MMP.

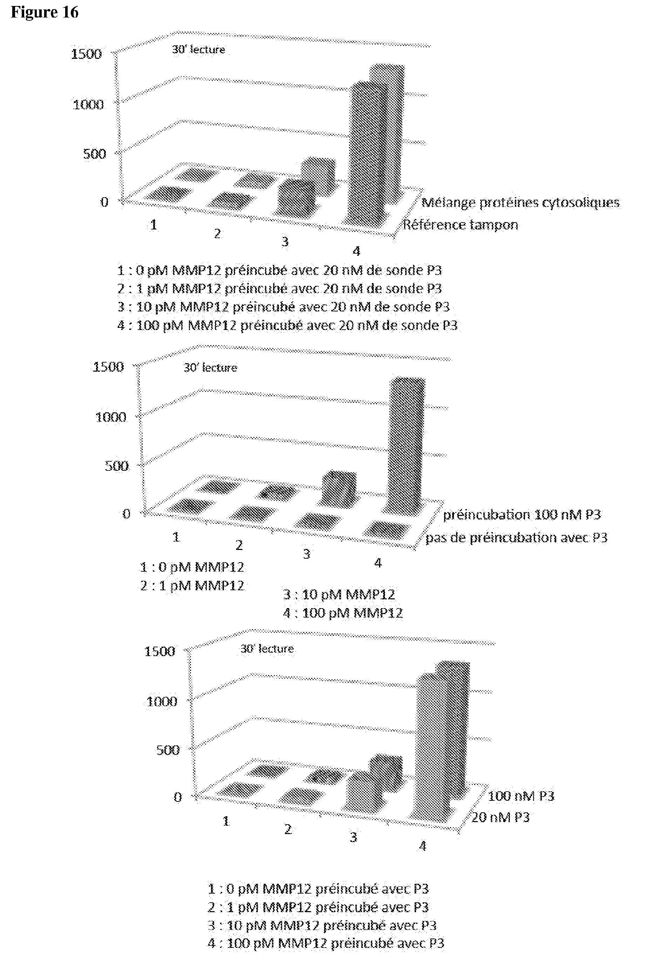

**Figure 16** **:** Détection de MMP12h par EIA sur plaque polyAb12h en présence de concentrations croissantes de MMP12h, dans un échantillon avec un mélange de protéines cytosoliques ou avec un tampon de référence, le traceur étant le ligand P3 qui comprend la biotine.

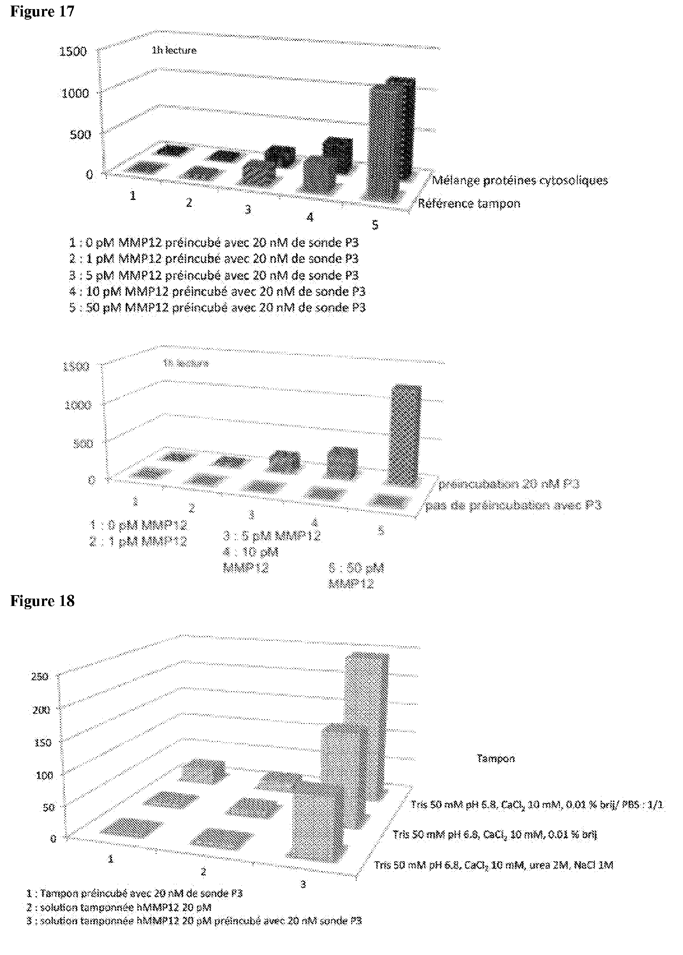

**Figure 17** **:** Détection de MMP12h par EIA sur plaque mAb12h en présence de concentrations croissantes de MMP12h, dans un échantillon avec un mélange de protéines cytosoliques ou avec un tampon de référence, le traceur étant le ligand P3 qui comprend la biotine.
**Figure 18** **:** Détection de MMP12h par EIA sur plaque mAb12h en présence de concentrations croissantes de MMP12h, dans un échantillon avec différents tampons, le traceur étant le ligand P3 qui comprend la biotine.
**Figure 19** **:** Dosage à l'aide d'un substrat fluorogénique de MMP-2, MMP-9, MMP-12, MMP13 et MMP-14 humaines présentes dans les surnageants à la suite de l'incubation des échantillons sur des plaques 1BSA-P1 et HSA-P2.
**Figure 20****:** Détection de MMP12h par EIA sur plaque CAS en présence de concentration croissante de MMP12h, de traceur AchE(G4)-P1 et d'anticorps monoclonal mAb12h: Absorbances mesurées à 1 heure en fonction de la concentration de MMP12h active
**Figure 21** **:** Détection de MMP12h par EIA sur plaque mAb12h en présence de tampon, de MMP12h active ou de MMP12h inactivée, et à gauche : en présence de AchE(G4)-P1 ; ou à droite : en présence de biot-HSA-P2 dans le puits.
**Figure 22** **:** Détection de MMP2h par EIA sur plaque BSA-P1 avec des concentrations croissantes de MMP2h dans un tampon.
**Figure 23****:** Détection de MMP9h par EIA sur plaque BSA-P1 avec des concentrations croissantes de MMP9h dans un tampon.

### EXEMPLES

### MATERIELS ET METHODES

Le format des différentes bandelettes mises en oeuvre dans les expériences étaient :
- Bandelette BSA-P1: bandelette sur laquelle est absorbée de la BSA polyfonctionnalisée avec P1.
- Bandelette HSA-P2 : bandelette sur laquelle est absorbée de la HSA monofonctionnalisée avec P2.
- Bandelette mAb12h : bandelette sur laquelle est absorbé un anticorps anti-MMP12 humaine.

Le format des différentes plaques mises en oeuvre dans les expériences étaient :
- Plaque BSA-P1 : plaque sur laquelle est absorbée de la BSA polyfonctionnalisée avec P1.
- Plaque HSA-P2 : plaque sur laquelle est absorbée de la HSA monofonctionnalisée avec P2.
- Plaque mAb12h : plaque sur laquelle est absorbé un anticorps anti-MMP12 humaine.
- Plaque polyAb12 : plaque sur laquelle est absorbé un anticorps anti-MMP12 humaine ou murine.

Les traceurs utilisés sont les suivants :
- OR- mAb12h : un anticorps anti-MMP12 humaine couplé à une bille d'or, utilisé avec les bandelettes BSA-P1 et BSA-P2.
- OR-BSA-P1 : la BSA polyfonctionnalisée avec P1 couplée à une bille d'or, utilisé avec la bandelette mAb12h.
- OR-BSA-P2 : la BSA monofonctionnalisée avec P2 couplée à une bille d'or, utilisé avec la bandelette mAb12h.
- OR-HSA-P2 : la HSA monofonctionnalisée avec P2 couplée à une bille d'or, utilisé avec la bandelette mAb12h.
- biotine-HSA-P2 ou biot-HSA-P2 : la HSA monofonctionnalisée avec P2 couplée à une biotine.
- biotine-BSA-P2 ou biot-BSA-P2 : la BSA monofonctionnalisée avec P2 couplée à une biotine.
- AchE(G4)-P1 : AchE(G4) polyfonctionnalisée avec P1

### LIGANDS P1, P2 et P3

### Structures des ligands P1, P2 et P3

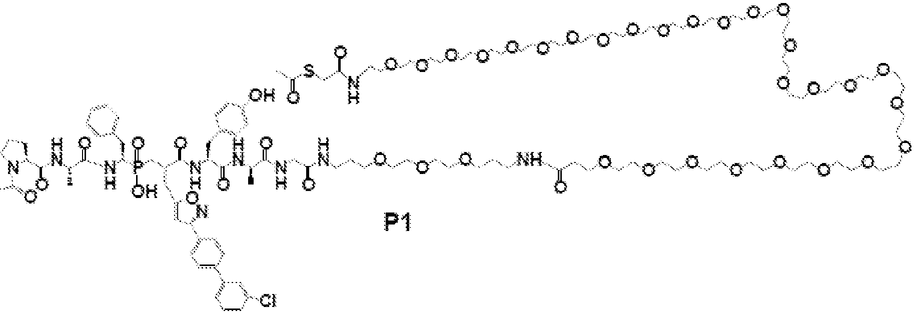

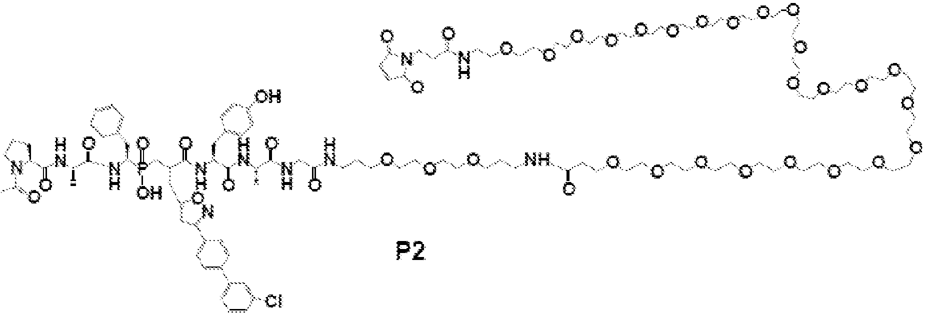

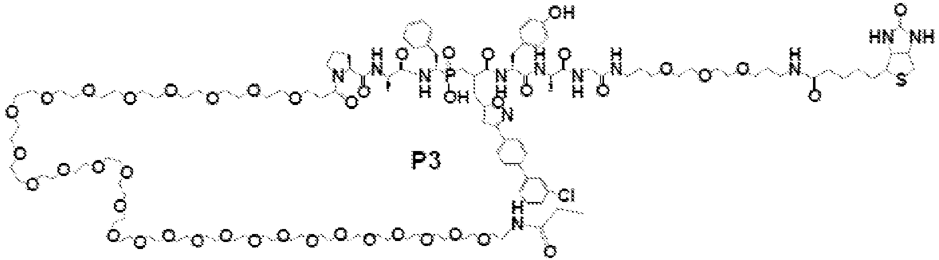

### Stratégie de synthèse du ligand P1

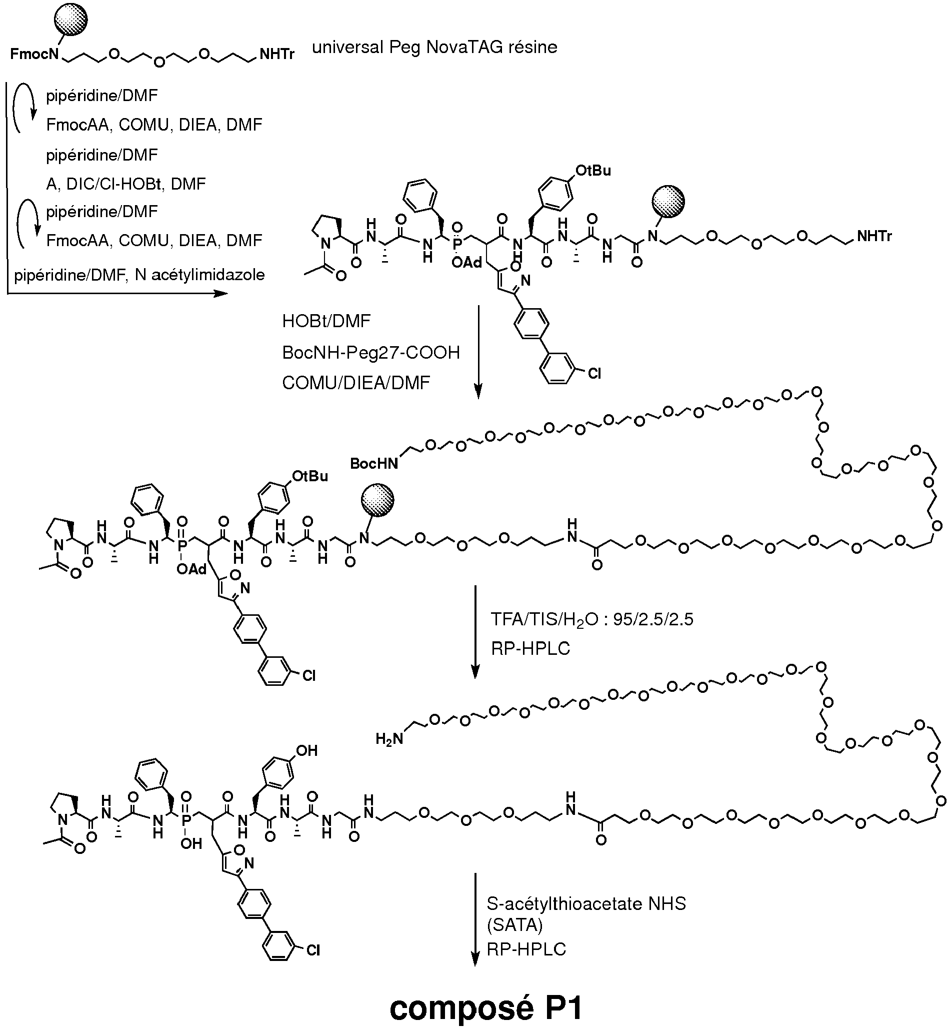

### Stratégie de synthèse du ligand P2

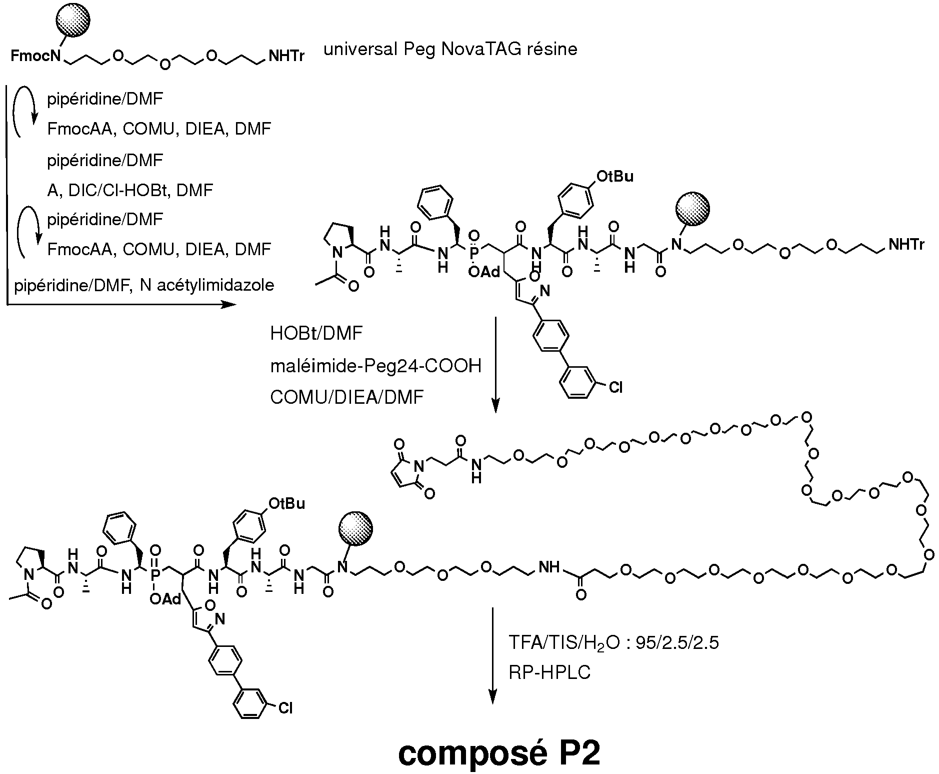

### Stratégie de synthèse du ligand P3

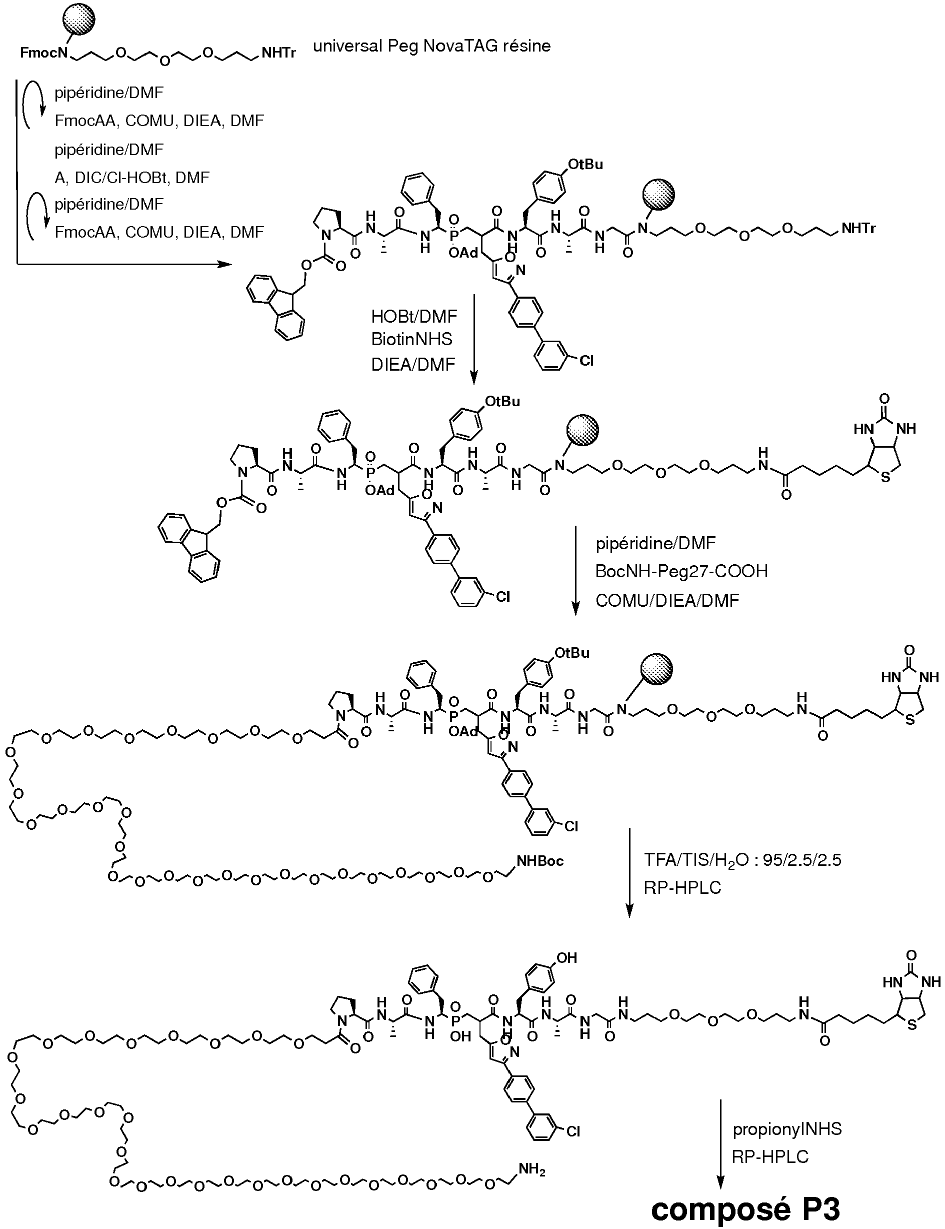

Tous les réactifs et solvants commercialement disponibles ont été utilisés tels que reçus, sans purification additionnelle. Le support solide (pegNovaTag resin), les acides aminés naturels protégés par le groupement Fmoc, la biotine activée sous forme de succinimide, l'agent de couplage COMU utilisés pour les synthèses des sondes proviennent de chez Novabiochem. Les liens additionnels de type polyéthylène glycol proviennent de chez Iris bitotech. La *N,N*-diméthylformamide anhydre (DMF) est commercialisé par la société Fluka. Le 6-chloro-1-hydroxybenzotriazole (ClHOBt) et le diisopropylcarbodiimide (DIC) utilisés pour l'incorporation du bloc phosphinique sont respectivement commercialisés par la société Molekula et par la société Aldrich. L'acide trifluoroacétique (TFA) et le triisopropylsilane (TIS) sont commercialisés par la société Aldrich. La BSA (sérum albumine bovine) est commercialisée par la société Merck et la HSA (sérum albumine humaine) provient de la société Aldrich.

Les séparations analytiques et préparatives RP-HPLC ont été effectuées, respectivement, sur un appareil de séparation de marque Shimadzu et un appareil de marque Gilson en utilisant soit une colonne analytique de type Ascentis express (100 x 4,6mm, 100Å) soit une colonne semi-préparative de type Kromasil AIT C 18 (250 x 20 mm, 10 µm, 100Å) avec respectivement des débits de 1, 2 et 3 mL. min⁻¹. La détection a été effectuée à 230 nm et 275 nm. Un système de solvant consistant de (A) 0,1% TFA dans 90% eau-10% acétonitrile et (B) 0,09% TFA dans 90% acétonitiile-10% eau a été utilisé. Les temps de rétention (tR) obtenus en mode analytique (colonne Ascentis Express) sont reportés en minutes.

Les mesures de densité optique (DO) des composés P1, P2 et P3 purifiés ont été effectuées avec un spectrophotomètre Beckman DU640B. L'enregistrement des spectres de masse implique une co-précipitation de l'échantillon avec un volume de matrice 4-HCCA (acide Cyano-4- hydroxycinnamique) à 10 mg/mL dans un mélange eau/acétonitrile/TFA : 1/1/0,01. Les spectres de masse des composés P1, P2 et P3 ont été enregistrés en utilisant un spectromètre de masse MALDI-TOF 4800 (Applied Biosystems, Foster City, USA) en mode réflectron négatif dans l'intervalle m/z de 800-3000. Chaque spectre est le résultat de 1000 à 2000 coups (20 positions différentes à l'intérieur de chaque tâche et 50 coups par sous-spectre) et une calibration interne utilisant des kits CALmix standards vendus par Applied biosystems (MDS Sciex).

La synthèse des composés P1, P2 et P3 s'a été effectuée indépendamment et manuellement sur support solide à partir de :
- résine Universal PegNova Tag comportant un espaceur PEG (polyéthylène glycol) protégé par un groupement trityl,
- d'acides aminés naturels protégés par la fonction Fmoc sur le N-terminal ainsi que par un groupement tertiobutyl dans le cas de la tyrosine,
- de liens PEG dont la fonction amine terminale est protégée par un groupement Boc ou fonctionnalisée par un maléimide.

Ces synthèses utilisent en outre un bloc phosphinique synthétique commun : le bloc A comportant une fonctionnalité carboxylique (COOH), une fonction amine protégée par un groupement Fmoc (Fmoc), une fonction phosphoryle protégée par un groupement adamantyl (Ad) et une chaîne latérale incorporant un hétérocyclique de type isoxazole et deux groupements phényls dont un comporte un atome de chlore.

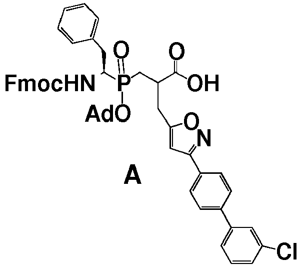

L'agent de couplage utilisé pour assembler ces unités était le COMU à l'exception du couplage du bloc A qui utilise un mélange DIC/Cl-HOBt. Lors du couplage, les acides aminés naturels ont été utilisés en excès (5 eq. par rapport au nombre de fonctions amines de la résine utilisée) en présence de DIEA (10 eq.) dans le DMF. Le couplage du bloc A a impliqué l'utilisation de ce bloc avec un excès 1.5 eq dans le DMF. Le couplage des liens PEG a impliqué l'utilisation de 3 eq. de ces liens en présence de 6 équivalents de DIEA dans le DMF. Des étapes de capping (piégeage des amines libres n'ayant pas réagi) ont été réalisées à la suite de chaque couplage par une solution d'acétylimidazole. L'élimination du groupement Fmoc de l'extrémité N-terminale des peptides ou pseudo-peptides supportés a été réalisé par un mélange DMF/pipéridine (1/1) suivi de lavages avec du DMF et du dichlorométhane. La déprotection du groupement trityl porté par le lien PEG fonctionnalisant la résine a été réalisée sur support solide à l'aide d'une solution d'HOBt 0,6 M dans un mélange TFE/CH₂Cl₂ : 1/1. L'élimination des groupements Boc porté par les liens PEG et des groupements protecteurs de la tyrosine et du bloc A ont été faits pendant la séparation du pseudopeptide de la résine impliquant le traitement de la résine par un mélange TFA/TIS/H₂O: 95/2,5/2,5. Les mélanges obtenus ont ensuite été purifiés par HPLC et ont conduit à l'obtention du composé P2 et à des précurseurs des composés P1 et P3 isolés chacun sous forme pures. Les précurseurs des composés P1 et P3 obtenus ont été couplés dans le DMF en présence de DIEA respectivement avec des dérivés succinimides de l'acétylthioactétate et de l'acide propionique pour conduire respectivement après élimination de l'excès des réactifs succinimide par HPLC au composés pures P1 et P3.

Les puretés des composés P1, P2 et P3 ont été attestées par les spectres obtenus en RP-HPLC analytique. Les spectres présentaient un pic unique et les temps de rétentions exprimés en minutes pour un gradient de 0 à 100% de B en 10 min. étaient respectivement 6,65 pour P1, 6,4 pour P2 et 6,4 pour P3.

Les composés P1, P2 et P3 ont été caractérisés par spectrométrie de masse en utilisant le mode reflector négatif.

Les constantes d'inhibition Ki des composés P1, P2 et P3 ont été déterminées vis-à-vis des MMP humaines 2, 3, 8, 9, 12, 13, 14 et vis-à-vis de la MMP12 murine. Les protéases recombinantes ont été utilisées dans des volumes de 100 µL à des concentrations de 0,05, 0,3, 0,03, 0,05, 0,03 ; 0,015 et 0,5 nM respectivement pour les MMP humaines 2, 3, 8, 9, 12, 13, 14 et à une concentration de 0,1 nM pour la MMP12 murine. La détermination des Ki dans ce test utilisait un substrat fluorogénique commercial MCA-Mat à 15 µM suivant une procédure analogue à celle décrite dans Devel et al, (2006, J. Biol. Chem., 281(16):11152-60). On rappellera que, plus le Ki d'un composé est faible, plus son potentiel d'inhibition vis-à-vis de la cible choisie est élevé. Les Ki ainsi que les quantités d'enzyme utilisées dans chaque test d'inhibition sont mentionnées dans le tableau ci-dessous.

| | MMP | 2h | 3h | 8h | 9h | 12h | 13h | 14h | 12m |
|---|---|---|---|---|---|---|---|---|---|
| | nM | 0,05 | 0,3 | 0,03 | 0,05 | 0,03 | 0,015 | 0,5 | 0,1 |
| Ki | P1 | 0,12 | 0,75 | 0,09 | 0,28 | 0,03 | 0,017 | 0,53 | 0,08 |
| nM | P2 | 0,15 | 1 | 0,12 | 0,48 | 0,05 | 0,03 | 0,7 | 0,09 |
| | P3 | 0,3 | 1,55 | 0,47 | 0,73 | 0,26 | 0,07 | 1,82 | 0,66 |

### PROTEINES FONCTIONNALISEES

### Sérum albumines fonctionnalisées

Le ligand P1 a été utilisé pour polyfonctionnaliser la BSA conduisant à BSA-P1, alors que le ligand P2 a été utilisé pour monofonctionnaliser de la HSA ou la BSA conduisant respectivement à HSA-P2 ou BSA-P2.

### Préparation de BSA polyfonctionnalisée par le ligand P1 : BSA-P1

BSA-P1 était issu du couplage du ligand P1 comportant un groupement SATA avec de la BSA fonctionnalisée par des groupements maléimides. La présence de ces groupements sur la BSA était issue du couplage d'un lien hétérofonctionnel (N-succinimidyl-4-(N-maléimidométhyl)-cyclohexane-1-carboxylate : SMCC) à la BSA commerciale.

Le couplage du ligand P1 contenant un SATA à la BSA-SMCC a été réalisé (ratio molaire inhibiteur/BSA-SMCC égal à 10) après déprotection de la fonction thiol du ligand P1 par ajout d'une solution d'hydroxylamine 1M pH 7 pendant 30 minutes à température ambiante.

La solution obtenue a été utilisée sans purification supplémentaire pour :
- être absorbée soit sur bandelettes destinées à la technique ICT, soit au fond des puits des plaques destinées à la technique EIA
- les préparations fournissant le traceur OR-BSA-P1.

### Préparation de HSA monofonctionnalisée par le ligand P2 : HSA-P2

HSA-P2 a été issue du couplage du ligand P2 comportant un groupement maléimide avec de la HSA.

La HSA commerciale isolée à partir de plasma humain a été préalablement placée en conditions réductrices contrôlées afin d'accéder à la réduction totale de la cystéine en position 64 de la HSA, initialement sous forme d'une mélange de forme réduite (SH = thiol libre) et thiol oxydé (adduit avec cystéine endogène du plasma). Ce traitement, réalisé à partir d'une solution de HSA à 2mg/mL en présence de 2 équivalents molaires de DTT dans un tampon phosphate de sodium 100 mM pH 6, a été suivi d'une élimination de l'agent réducteur à l'aide d'un filtron 10KDa. Le mélange résultant a été mis en présence d'un équivalent molaire du ligand P2 pour fournir HSA-P2. La solution obtenue a été utilisée sans purification supplémentaire pour :
- être absorbée soit sur bandelettes destinées à la technique ICT, soit au fond des puits des plaques destinées à la technique EIA
- les préparations fournissant les traceurs biotine-HSA-P2 ou OR-HSA-P2.
Le même protocole permet la préparation de BSA-P2.

### AchE(G4) fonctionnalisée

0,1 nmol de AchE(G4-SMCC), forme tétramérique de l'acétylcholinestérase (commercialisé par SPI-BIO) ont été couplées via un lien thioéther à 2,7 nmoles de P1 pour conduire à AchE(G4)-P1, AchE polyfonctionnalisée par le ligand P1. 10 µL d'une solution d'hydroxylamine 1M ont été ajoutés à 2,7 nmoles de P1 dans 40 µL de tampon phosphate de sodium 100 mM pH 6 EDTA 5.10⁻³ M. Après 30 minutes d'incubation à température ambiante, la solution contenant P1 a été additionnée à 62,5 µL de tampon phosphate de sodium 100 mM pH 6 EDTA 5.10⁻³ M contenant 0,1 nmol de AchE(G4-SMCC). L'ensemble a été agité pendant une nuit à 4°C puis une étape de purification sur un Biogel A015M (gel d'exclusion stérique) utilisant un tampon phosphate 100 mM pH 7,4, 0,4 M NaCl, 0,5% BSA en présence de NaN₃ a permis d'isoler une solution stock de traceur AchE(G4)-P1 ayant une activité de 368 UE/min./mL. Le suivi de cette étape de purification a été effectué par dosage des fractions collectées à l'aide d'acétylthiocholine et de DTNB.

### TEST IMMUNOCHROMATOGRAPHIQUE SUR BANDELTTES (ICT)

### Préparation des bandelettes

La réalisation de l'ICT a impliqué la préparation de bandelettes fonctionnalisées par BSA-P1, HSA-P2 ou mAb12.

La préparation des bandelettes fonctionnalisées (dimensions finales : 0,5 cm x4,5 cm) a été obtenues à partir de deux composants : membrane de nitrocellulose (zone de réaction) et buvard de nitrocellulose (zone d'adsorption facilitant la migration par capillarité).

La zone de réaction a été utilisée pour l'immobilisation de 2 types d'entités immobilisés selon deux lignes :
- ligne test ou zone de test,
- ligne contrôle de migration d'anticorps résultant de l'immobilisation d'anti immunoglobulines de souris à 800-100µG/mL dans du PBS (CAS) ou d'anti immunoglobulines de lapin (SAL).

La ligne test ou zone de test correspond à l'immobilisation :
- de la sérum albumine fonctionnalisée par le ligand P1 ou P2 (BSA-P1 ou HSA-P2) ou
- d'un anticorps d'intérêt monoclonal ou polyclonal anti-MMP12h.

Les lignes tests ont été déposées à la main à l'aide d'un peigne 12x0,3 µL pour le système d'électrophorèse-PHASTSYSTEM (Pharmacia) à raison de 2µL/ cm sur la zone de dépôt de l'échantillon à partir de solution à 200 ng/cm pour BSA-P1 et 2 mg/cm pour HSA-P2 ou les anticorps monoclonal ou polyclonal.

La ligne contrôle résultait de l'immobilisation de CSA ou SAL déposés à raison de 1 µL/cm d'une solution dans le PBS respectivement à 0,1 mg/mL et à 0,5 mg/mL en utilisant un distributeur automatisé (Bio Dot -Air jet XYZ 3050).

A la suite de ces dépôts, les membranes ont été séchées dans une étuve à 40°C pendant 1 heure. Elles ont été ensuite incubées pendant 30 minutes à deux reprises à température ambiante (20°C) sous oscillation lente dans une solution de saturation constituée de tampon phosphate de sodium 10 mM pH 7,4 contenant 0,15 M de NaCl et 0,5% BSA en présence de NaN₃. Ces incubations ont vocation à bloquer tous les sites de liaison résiduels. Les membranes ont été ensuite soumises à des lavages avec de l'eau utltrapure (H2O milliQ) puis incubées pendant 30 minutes à température ambiante dans une solution constituée de tampon phosphate de sodium 10 mM pH 7,4 contenant 0,15 M de NaCl et 0,1% de tween 20. Les membranes ont été ensuite égouttées sur un papier absorbant de type sopalin puis séchées pendant 15 min. à 40°C dans l'étuve puis les membranes de dépôt et d'absorption de l'échantillon ont été collées sur le bas et le haut de la membrane de nitrocellulose avant d'être découpées en bandelettes de 5 mm de largeur à l'aide d'une guillotine automatisée programmable (Guillotine Cutting-CM 400-Bio Dot). Les bandelettes BSA-P1, HSA-P2, mAb obtenues ont été conservées dans des étuis plastiques à température ambiante et à l'abri de la lumière.

### Préparation des traceurs OR-BSA-P1, OR-HSA-P2, OR-BSA-P2 et OR-mAb

L'obtention des traceurs OR-BSA-P1, OR-HSA-P2, OR-BSA-P2 et OR-mAb12 a nécessité la préparation d'une solution stock d'or colloïdal. Cette solution a été obtenue par addition sous agitation constante de 4 mL d'une solution de chlorure d'or (AuCl₂) à 0,2% à une solution bouillante d'eau ultrapure (40 mL d'H₂O MilliQ) à laquelle 1 mL de solution à 1% de citrate de sodium ont été préalablement ajouté. A la suite de l'apparition d'une couleur « lie de vin » de la solution (faisant suite à une coloration noire), le retour de la solution obtenue à température ambiante a été effectué. La solution stock a été conservée à 4°C, à l'abri de la lumière.

A 25µg d'anticorps ou sérum albumine fonctionnalisée (dans 50µL maxi) en solution dans du tampon phosphate de sodium 10mM ph=7,4, NaCl 0,15 M, 0,01% NaN₃ a été ajoutée 1mL de solution stock d'or colloïdal et 100µL tampon borax (trisodium citrate) 20mM ph=9. Après une heure d'incubation sous rotation mécanique (4°C), 100 µL de tampon borax 20mM ph=9 BSA 1% ont été ajoutées au mélange suivi d'une centrifugation à 15000g durant 50 minutes 4°C. Suite à la centrifugation, le surnageant a été éliminé et le culot repris par 1mL de tampon Borate 2mM, BSA 1%, azide 0,01%. Après resuspension du culot, la solution obtenue a été centrifugée à 15000g pendant 50 minutes à température ambiante. Après élimination du surnageant le culot a été repris par 250µL de tampon Borate 2mM, BSA 1%, NaN₃ 0,01%. Les traceurs or OR-BSA-P1, OR-HSA-P2, OR-BSA-P2 et OR-mAb12 conservés à une concentration finale de 100µg/mL ont été stockés à 4°C à l'abri de la lumière.

### Réalisation de l'ICT

L'utilisation de bandelettes fonctionnalisées par BSA-P1 ou HSA-P2 a été couplée à une révélation par les traceurs OR-mAb12, OR-polyAb12. L'utilisation de bandelettes fonctionnalisées par les anticorps a été couplée à une révélation par les traceurs OR-BSA-P1, OR-HSA-P2 ou OR-BSA-P2.

Le test a été réalisé à température ambiante dans une plaque de microtitration 96 puits. 100 µL d'un échantillon natif, dilué ou constitué ou 50 µL d'un échantillon ont été placés dans un puits respectivement vide ou contenant 50 µL du tampon Tris-HCl 100mM pH 6,8, 0,5% tween-20, 1% Chaps, 0,1%NaN₃ déjà présent dans le puits.

### Bandelettes fonctionnalisées par BSA-P1 ou HSA-P2 couplées à une révélation par les traceurs OR-mAb12, OR-polyAb12

Après absorption de la solution contenue dans le puits, des lavages de la bandelettes ont été réalisés à l'aide de tampon Tris-HCl 100mM pH 7,4, 0,5% tween-20, 1% Chaps, 0,1%NaN₃ ou Tris-HCl 50 mM pH6,8, CaCl₂ 10 mM, brij 0,01%. Les volumes et le nombre d'itération de ces lavages différaient selon que l'échantillon traité, un échantillon contenant ou pas de milieux biologiques. Un lavage de 50 µL, puis 30 µL, puis 20 µL, a été réalisé dans le cas de l'analyse d'un échantillon contenant du milieu biologique, alors qu'un lavage de 30 µL suivi d'un lavage de 20 µL a été effectué pour les échantillons contenant uniquement des protéines recombinantes (avec adjonction ou pas de compétiteur). A la suite de l'addition des 20 µL, un volume de 5 à 10 µL du traceur a été additionné dans le puits lorsque le traceur est l'anticorps monoclonal. La migration du traceur permettait la visualisation de la protéine capturée soit par la BSA-P1, soit la HSA-P2, par focalisation d'un signal visible à l'oeil nu le long de la bande test déposée. L'intensité du signal observé a été qualitativement estimée à l'oeil nu.

### Bandelettes fonctionnalisées par les anticorps couplées à utile révélation par les traceurs OR-BSA-P1, OR-HSA-P2 ou OR-BSA-P2.

Dans ce cas, le traceur peut, ou pas, être incubé avec l'échantillon dès le départ. Après agitation du mélange, l'insertion verticale de la bandelette dans le puits a été faite du coté de la zone de dépôt d'échantillon. Le liquide montait le long de la bandelette par capillarité. La migration du traceur permettait la visualisation à la protéine capturée par l'anticorps monoclonal par focalisation d'un signal visible à l'oeil nu le long de la bande test déposée. L'intensité du signal observé a été qualitativement estimée à l'oeil nu.

### TEST IMMUNOLOGIQUE (EIA)

### Préparation des plaques fonctionnalisées par BSA-P1. HSA-P2, mAb12 ou polyAb12.

Les plaques portant BSA-P1, HSA-P2 ou l'anticorps monoclonal ont été préparées à partir de plaques couramment utilisées pour les dosages immunologiques. 100 µL de solutions à 2µg/mL (BSA-P1), 5µg/mL (HSA-P2) et 5µg/mL (l'anticorps monoclonal -mAb ; ou polyclonal - polyAb) dans du tampon Phosphate de sodium 50 mM pH 6 ont été utilisés pour fonctionnaliser les puits des plaques. Après une incubation d'une nuit à 4°C et retrait du surnageant, 300 µL d'une solution de tampon phosphate de sodium 10 mM pH 7,4 contenant 0,15 M de NaCl et 0,1% BSA, 0,1% NaN₃ ont été ajoutés dans les puits pour bloquer tous les sites de liaison résiduels. Les plaques ont été conservées dans ce tampon à 4°C avant utilisation.

### Préparation des traceurs :

### 1 - Traceurs Biot-mAb12 et biot-HSA-P2

Biot-mAb12 et Biot-HSA-P2 ont été préparés à partir de solution de mAb 12 ou de HSA-P2 dans du tampon borate 100 mM pH 9 et d'une solution de biotine active sous forme d'un ester N-hydroxysuccinimide (biotine-NHS) à 5mg/mL dans le DMF préparée extratemporanément.

Biot-mAb12 : 250 µg de mAb12 (125µL d'une solution à 2 mg/mL dans du PBS) ont été placés dans 200 µL de tampon Borate 100mM ph=9,0 puis l'addition de 20µL de biotine-NHS à 5mg/mL a été réalisée. A la suite d'une incubation de 45 minutes à température ambiante, 115 µL de tampon Tris 1M ph=8,0 ont été ajoutés au mélange. Une incubation de 10 minutes suivie de l'addition d'un volume 650 µL de tampon phosphate 100mM pH 7,4, 0,1% BSA, 0,15M NaCl, 0,01% NaN₃ EIA (0,1% BSA) pour atteindre une concentration de traceur Biot-mAb12 de 200 µg/mL. La solution stock de Biot-mAb12 a été conservée à 4°C.

Biot-HSA-P2 : 175 µg de HSA-P2 (87 µL d'une solution à 2 mg/mL dans du PBS) ont été placés dans 200 µL de tampon Borate 100mM ph=9,0 puis l'addition de 9µL de biotine-NHS à 5mg/mL a été réalisée. A la suite d'une incubation de 45 minutes à température ambiante, 100 µL de tampon Tris 1M ph=8,0 ont été ajoutés au mélange. Une incubation de 10 minutes a été suivie de l'addition d'un volume 475 µL de tampon phosphate 100mM pH 7.4, 0,1% BSA, 0,15M NaCl, 0,01% NaN₃ EIA pour atteindre une concentration stock de traceur Biot-HSA-P2 de 200 µg/mL. La solution stock de Biot-HSA-P2 a été conservée à 4°C.

### 2 - Traceur P3

Le ligand P3 a été utilisé tel qu'il a été isolé après purification après avoir été mis en solution dans le DMSO.

### 3 - Traceur AchE(G4)-P1

Le traceur AchE(G4)-P1 a été utilisé tel qu'il a été isolé après purification.

### Réalisation de l'EIA

L'utilisation de plaques fonctionnalisées par BSA-P1 ou HSA-P2 a été couplée à une révélation *via* les traceurs biot-mAb12h.

L'utilisation de plaques fonctionnalisées par les anticorps (mAb12 ou polyAb12) a été couplée à une révélation *via* les traceurs biot-HSA-P2, P3 ou AchE(G4)-P1.

Après mise à température ambiante des plaques fonctionnalisées, les puits ont été lavés avec un laveur automatique de plaques par un cycle de 5 lavages d'un volume de 300 µL utilisant du tampon phosphate 10 mM, Tween 0,1%, pH 7,4.

Dans le cas des tests EIA utilisant le traceur biot-mAb12h ou le traceur biot-HSA-P2 (dans une version séquentielle), les échantillons d'un volume de 100 µL dans des tampons variables ont alors été placés dans les puits pour une incubation soit de 4 heures, soit d'une nuit. Cette étape correspondant à l'étape de capture des espèces d'intérêt dans l'échantillon a été suivie d'un retrait du surnageant et d'une série de lavages avec trempage de 2 à 5 minutes sous agitation à l'aide de tampon Tris HCl 100 mM, 10 mM CaCl₂, NaCl 1M, 0,5 % tween. S'est ensuivi l'addition de traceur biotinylé à une concentration 1*g/mL utilisée dans du tampon PBS pH 7,4, NaCl 0,15 M, 0,1% BSA, NaN3 0,01% pour biot-mAb12 et dans du tampon Tris HCl 50 mM, pH 6,8, CaCl₂ 10 mM, brij 0,01% pour biot-HSA-P2. L'incubation avec le traceur a été réalisée pendant une nuit à 4°C avant le retrait du surnageant, une nouvelle série de lavages à l'aide de tampon phosphate 50 mM, Tween 20 0,1%. 1 UE/min/mL de strepatvidine AchE(G4) (acétylcholinestérase) a été additionnée suivi d'une incubation de 2h avant retrait des surnageants, lavages et addition d'acetylthiocholine et de DTNB (DTNB=5,10⁻⁴ M, Acétyl thiocholine 1,4.10⁻⁵ M dans du tampon phosphate 10 mM). La détection de la protéine d'intérêt a été mesurée *via* une mesure de l'activité de AchE(G4) dont l'action sur son substrat en présence de DTNB fournit une absorbance à 414 nm.

Dans le cas des tests EIA utilisant le traceur biot-HSA-P2 (dans une version simultanée), l'échantillon a été préincubé avec biot-HSA-P2 puis placé en plaque. A la suite de la capture de la protéine d'intérêt par l'anticorps greffé et des lavages, 1 UE/min/mL de streptavidine AchE(G4) a été incubée, puis, après retrait du surnageant, la détection de la protéine d'intérêt a été réalisée par addition d'acetylthiocholine et DTNB et mesure de l'absorbance produite à 414 nm.

Dans le cas des tests EIA utilisant le traceur P3, l'échantillon a été préincubé avec une concentration de 20 ou 100 nM de P3 puis placé en plaque. A la suite de la capture de la protéine d'intérêt par l'anticorps greffé (plaque mAb12h, plaque polyAb12m, plaque polyAb12h), 1 UE/min/mL de streptavidine AchE(G4) a été incubée, puis, après retrait du surnageant, la détection de la protéine d'intérêt a été réalisée par addition d'acetylthiocholine et DTNB et mesure de l'absorbance produite à 414 nm.

Dans le cas des tests EIA utilisant le traceur AchE (G4)-P1, l'échantillon a été préincubé en plaque avec 2 UE/min/mL AchE(G4)-P1. A la suite de la capture de la protéine d'intérêt par l'anticorps greffé (ex : plaque mAb12h) ou par l'anticorps en solution utilisé avec des plaques CAS ou SAL, après retrait du surnageant, la détection de la protéine d'intérêt a été réalisée par addition d'acetylthiocholine et DTNB et mesure de l'absorbance produite à 414 nm.

### RESULTATS

### TEST IMMUNOCHROMATOGRAPHIQUE SUR BANDELTTES (ICT)

### Bandelette BSA -P1 : résultats

Les bandelettes BSA-P1 correspondent à des bandelettes sur lesquelles ont été immobilisés 100 ng de BSA polyfonctionnalisée par le ligand P1. L'utilisation des bandelettes BSA-P1 a été couplée à l'utilisation du traceur OR-mAb12h (anticorps monoclonal anti-MMP12 humaine).

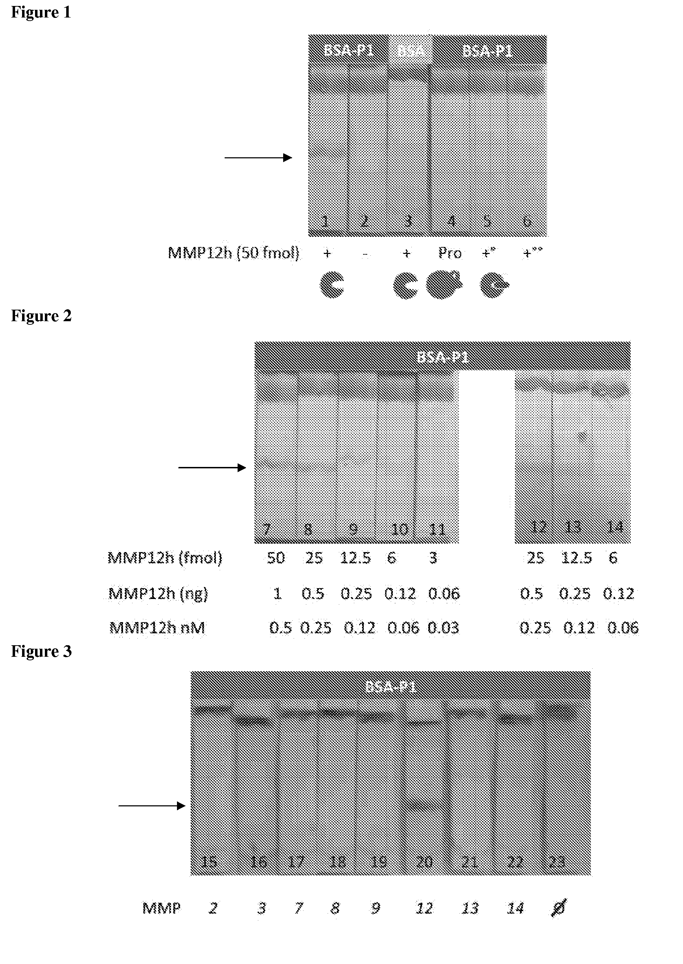

### Détection de la MMP12 humaine (MMP12h) - [Figure 1]

Les bandelettes BSA-P1 couplée à l'utilisation du traceur OR-mAb12h permettent la détection positive de MMP12h (1) via la visualisation d'un signal visible à l'oeil nu au niveau de la zone centrale d'immobilisation de BSA-P1 sur la bandelette. Cette détection est spécifique de l'utilisation de MMP12h dans l'expérience puisque, en l'absence de MMP12h (2), la migration le long de la bandelette du traceur OR-mAb12h a conduit à l'absence de signal observable au niveau de la zone d'immobilisation de BSA-P1 sur la bandelette. La détection positive de MMP12h (1) résulte d'une interaction entre la MMP12h et le ligand P1 porté par la BSA-P1. En effet, la migration de MMP12h et de traceur OR-mAb12h le long d'une bandelette portant de la BSA non modifiée immobilisée ne conduit pas à l'observation d'un signal positif (3), alors que le contrôle de migration en haut de la bandelette garantit une migration effective du traceur OR. La détection positive de MMP12h (1) implique une interaction de la MMP12h au niveau de son site actif étant donné que l'utilisation d'une solution de MMP12h préalablement incubée avec un inhibiteur spécifique de type phosphinique (compétiteur) de la MMP12h ayant un Ki de 0.2 nM au regard de la MMP12h (appelé « Compound 1 » ou RXP470.1 et décrit dans Devel et al, 2006, J Biol Chem, 281, 11152-11160) à une concentration de 100 nM (°) ou 1 µM (°°) ne conduit pas à l'observation d'un signal (respectivement 5, 6) au niveau de la zone d'immobilisation de BSA-P1. De même, la proMMP12, correspondant à la MMP12h inhibée par son propre propeptide, n'est pas détectée par le dispositif (4).

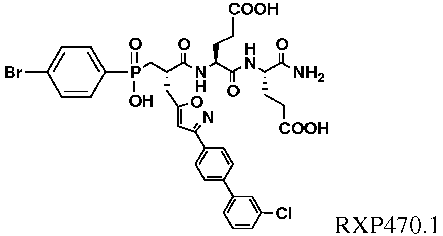

L'ensemble de ces expériences a permis de conclure que l'utilisation de bandelettes BSA-P1 couplée à l'utilisation du traceur OR-mAb12h permet la détection de MMP12h au lieu d'immobilisation de BSA-P1 *via* la formation d'un complexe ternaire impliquant d'une part des interactions moléculaires entre le site actif de la MMP12h et le ligand fonctionnalisant la BSA et d'autre part des interactions moléculaires entre un domaine de la MMP12h et l'anticorps monoclonal anti-MMP12h lié à l'or traceur (OR-mAb12h). Seule la MMP12h présentant un site actif libre, et donc active, peut être détectée, la proforme de la MMP12h et une forme préalablement inactivée par un inhibiteur de la MMP12h ne sont pas détectées. Ainsi, ce dispositif est bien adapté à la détection spécifique de la forme active de la MMP12h.

### Sensibilité de détection de MMP12h en milieu tamponné - [Figure 2]

L'utilisation d'une gamme de concentration de MMP12h dans du tampon Tris-HCl 50 mM pH6,8, CaCl₂ 10 mM, brij 0,01% et de bandelettes BSA-P1 couplées à l'utilisation de OR-mAb12h a indiqué que le système développée permet la détection de MMP12h à partir d'échantillons tamponnés contenant jusqu'à 6 fmol de MMP12h, ce qui est équivalent à une quantité en masse de 0,12 ng ou une concentration de 0,06 nM (10). Cependant, l'utilisation de ce tampon produit un effet de migration non uniforme comme le traduit la forme en vague de la bande révélée. L'utilisation d'une gamme de concentrations de MMP12h dans un mélange 50/50 de tampon Tris-HCl 50 mM pH6,8, CaCl₂ 10 mM, brij 0,01% et de tampon Tris-HCl 100mM pH 7,4, 0,5% tween-20, 1% Chaps, 0,1%NaN₃ a permis de rectifier cet effet, et dans ce cas le seuil de détection a atteint une valeur entre 6 et 12,5 fmol (soit entre 0,12 et 0,25 ng ou une concentration molaire entre 0,06 nM et 0,12 nM) comme le montrent les bandelettes (13) et (14).

### Spécificité du dispositif pour la détection de MMP12h versus d'autres MMPs - [Figure 3]

La sélectivité du système pour la détection de la MMP12h par rapport à d'autres MMPs a été vérifiée par des expériences utilisant des solutions indépendantes de MMP-2, -3, -7, -8, -9, -12, -13 et -14. Ces expériences ont confirmé la sélectivité de l'anticorps monoclonal bien que les MMP utilisées, qui partagent une grande homologie, aient pu être capturées par le ligand P1 fonctionnalisant la BSA immobilisée sur la bandelette. En effet, après migration des MMPs et du traceur OR-mAb, le seul signal positif est observé à partir de la solution de MMP12h (20), alors que les autres solutions de MMP conduisent à l'absence de signaux visibles sur les bandelettes (15, 16, 17, 18, 19, 21, 22), également non détecté en absence de MMP12h (23).

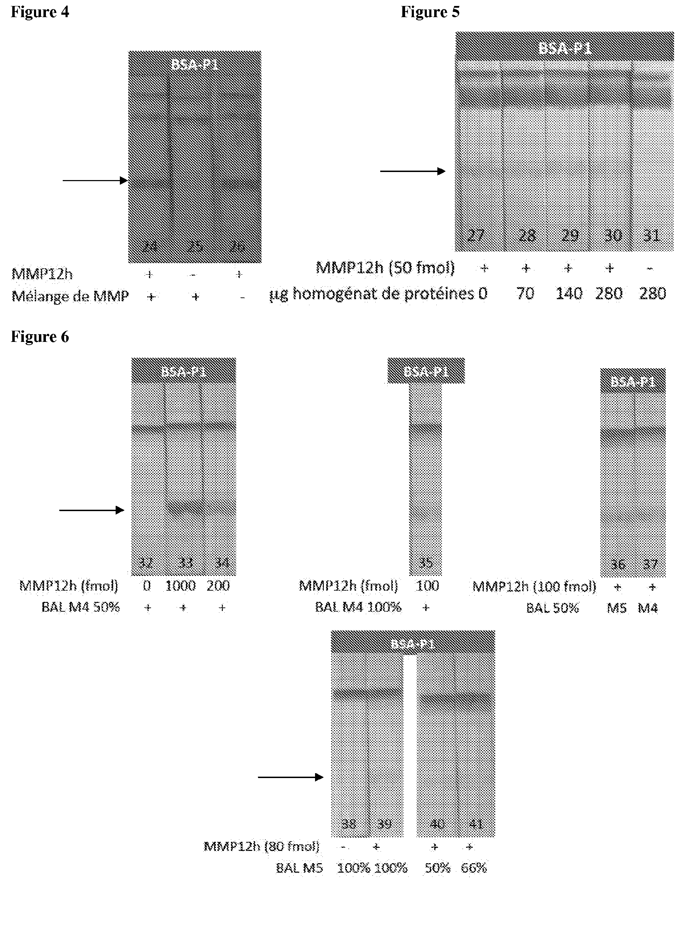

### Spécificité du dispositif pour la détection de MMP12h en présence d'autres MMPs - [Figure 4]

La capacité du dispositif à permettre la détection, à partir d'un mélange de MMPs, de la seule MMP12 a également été testé en comparant des expériences utilisant des solutions contenant différentes MMPs (2, 9, 13, 14) incluant la MMP12 (24) ou pas (25) et une solution ne contenant que de la MMP12h (26). Aucun signal au site d'immobilisation de la BSA-P1 sur la bandelette n'est visible à partir du mélange de MMP (25), alors qu'un signal est détecté à partir du mélange incluant la MMP12h (24). Ce signal a une intensité comparable avec celle obtenue à partir d'une solution de MMP12h en milieu tamponné (26).

### Détection en milieu complexe

Homogénat de protéines cytosoliques - [Figure 5] : Lorsque 50 fmoles de MMP12h sont additionnées à 70, 140 ou 280 µg d'un mélange complexe de protéines cytosoliques, la MMP12h a été détectée par le système bandelettes BSA-P1 couplée à OR-mAb12h par un signal (28, 29, 30) d'intensité comparable à celui observé lorsque la MMP12h utilisée est en présence de tampon (27). Le signal observé à partir des mélanges protéiques comportant de la MMP12h ajoutée est bien issu de la MMP12h étant donné que 280 µg d'homogénat des protéines variées utilisées n'ont pas produit de signal observable au site de l'immobilisation de BSA-P1 sur la bandelette (31). Ainsi, l'utilisation de bandelettes BSA-P1 couplée à une révélation par OR-mAb12h est possible avec des mélanges complexes de protéines et fournit dans le cas de protéines cytosoliques un signal de détection positif lorsque de la MMP12h est présente à seulement 0,0003%.

Lavages broncho-alvéolaires (BAL) [Figure 6]: La possibilité de détection de MMP12h par le dispositif de bandelettes BSA-P1 couplé à l'utilisation de traceur OR-mAb12h a également été testée par des ajouts de MMP12h à des lavages broncho-alvéolaires de souris. Ces expériences ont été prévues afin de caractériser la compatibilité d'un tel système pour détecter de la MMP12h à partir de BAL humains. Dans cette perspective deux types de milieux ont été évalués, nommés BAL M4 et BAL M5. BAL M4 100% correspond à un BAL de souris obtenu dans du PBS et dilué au demi par du tampon Tris-HCl 50 mM pH6,8, CaCl₂ 10 mM, brij 0,01%. BAL M5 100% correspond à un BAL de souris obtenu dans le PBS dilué au demi dans Tris-HCl 50 mM pH6,8, CaCl₂ 10 mM, brij 0,01%, puis à nouveau dilué au demi dans Tris-HCl 50 mM pH6,8, CaCl₂ 10 mM, brij 0,01%, NaCl 1M, urée 2M. Lorsque les pourcentages indiqués sont inférieurs à 100%, les dilutions de BAL M4 100% et BAL M5 100% ont été faites avant migration dans les puits de la plaque de microtitration avec le tampon suivant Tris-HCl 100mM pH 7,4, 0,5% tween-20, 1% Chaps, 0,1%NaN₃. Les résultats des expériences réalisées à partir des lavages broncho-alvéolaires ont montré que la détection de MMP12h est possible dans ces milieux. En effet, alors que les milieux seuls BAL M4 et BAL M5 ont conduit à une absence de signal au site d'immobilisation de BSA-P1 (32, 38), un signal a permis la détection d'une picomole (33), de 200 fmoles (34) et de 100 fmoles de MMP12h (35), que le milieu BAL M4 soit utilisé tel quel (35) ou dilué à 50% par du tampon Tris-HCl 100mM pH 7,4, 0,5% tween-20, 1% Chaps, 0,1%NaN₃ (33, 34). La détection de 100 fmoles de MMP12h est compatible avec la présence d'urée et de NaCl dans le milieu (respectivement à des concentrations de 1M et de 500 mM). En effet, le signal de détection de 100 fmoles de MMP12h ajoutée à 100 µL de BAL M5 100% (36) est comparable à celui de 100 fmoles de MMP12h ajoutée à 100 µL de BAL M4 100% (37). Le signal de détection de 80 fmoles de MMP12h ajoutée à 100 µL de BAL M5 100% (39) est comparable à celui de 80 fmoles de MMP12h ajoutée à 100 µL de BAL M5 50% (40) et BAL M5 66% (41).

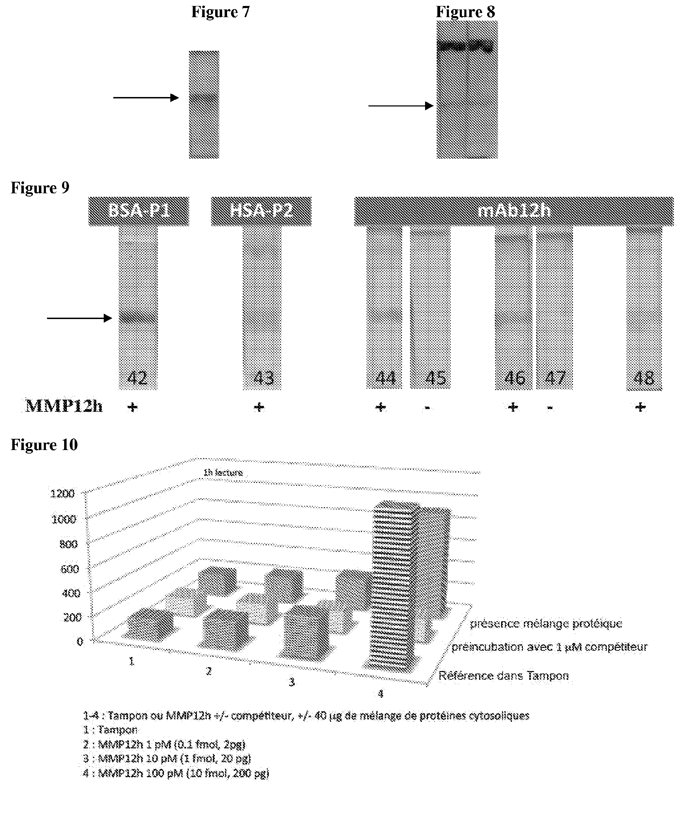

### Applicabilité du dispositif à la détection d'autres MMPs -[Figure 7, Figure 8]

Le dispositif a été testé pour la détection de MMP13 humaine active. Le traceur utilisé est un anticorps monoclonal anti-MMP13 humaine avec une bandelette chargée en BSA-P1.

Un signal positif est observé lorsque que de la MMP13h active est utilisée dans l'expérience (Figure 7). Le dispositif a été testé pour la détection de MMP12 murine active (Figure 8). Le traceur utilisé était un anticorps polyclonal anti-MMP12 murine. Un signal positif est observé lorsque que de la MMP12m active est utilisée dans l'expérience, en milieu tamponné ou en présence de 40 µg d'extrait de protéines cytosoliques. Ces résultats montrent l'opérationnalité du système à capturer des MMPs de nature différente, qui sont ensuite détectables à l'aide d'un anticorps spécifique de la MMP considérée.

### Autres Bandelette: résultats

En plus des bandelettes BSA-P1, les autres types de bandelettes produites étaient des bandelettes nommées bandelettes HSA-P2 et bandelettes mAb12h. Ces bandelettes correspondent à des bandelettes sur lesquelles ont été immobilisés respectivement de la HSA monofonctionnalisée par le ligand P2, et de l'anticorps monoclonal mAb12h. L'utilisation des bandelettes HSA-P2 est couplée, comme pour les bandelettes BSA-P1, à l'utilisation du traceur OR-mAb12h. L'utilisation des bandelettes mAb12h est couplée à l'utilisation des traceurs OR-BSA-P1 ou OR-HSA-P2 ou encore OR-BSA-P2 (traceur or de la BSA fonctionnalisé par un ligand P2).

### Comparaison efficacité de détection de la MMP12h par les bandelettes BSA-P1, HSA-P2 et mAb12h -[Figure 9]

Une même solution tamponnée de MMP12h a été utilisée avec les différentes bandelettes révélées respectivement par le traceur correspondant. Un signal positif est observé quel que soit le type de bandelettes engagée dans l'expérience lorsque de la MMP12h est utilisée dans l'expérience (42, 43, 44, 46 et 48) indiquant que :
- la HSA-P2 immobilisée sur la bandelette est capable de capturer la MMP12h utilisée dans l'expérience et révélée au site de l'immobilisation de HSA-P2 sur la bandelette par le traceur OR-mAb12h (43) ;
- OR-BSA-P1 (44), OR-HSA-P2 (46) et OR-BSA-P2 (48) constituent des traceurs capables de détecter la MMP12h capturée par le mAb12h immobilisé sur les bandelettes.

Ces résultats apportent une preuve que la partie inhibitrice constitué par le ligand P1 ou P2 de la BSA ou de la HSA peut constituer un partenaire du complexe ternaire ligand/MMP/mAb, que la sérum albumine soit immobilisée sur bandelette ou constitue le traceur. A partir des bandelettes mAb12h, le signal observé détectant la MMP12h est d'intensité comparable que la sérum albumine composant le traceur soit polyfonctionnalisée (44) ou monofonctionnalisée (46) ou encore que la sérum albumine soit humaine (46) ou bovine (48). Ainsi, lorsque la sérum albumine est utilisée comme traceur, le degré de fonctionnalisation de la sérum albumine par le ligand de la MMP d'intérêt a un effet négligeable sur la capacité du traceur à détecter la MMP12h capturée par le mAb12h immobilisé sur la bandelette. Par contre, ce degré de fonctionnalisation apparaît avoir un effet sur la capacité de la sérum albumine immobilisée sur bandelettes à capturer la MMP12h utilisée dans l'expérience. En effet, l'intensité du signal observé à partir d'une bandelette HSA-P2 couplée à l'utilisation de OR-mAb12h (43) est nettement plus faible que celui observé à partir d'une même solution de MMP12h et d'une bandelette BSA-P1 couplée à l'utilisation de OR-mAb12h (42). Etant donné la nature commune du traceur utilisé dans ces deux cas (42, 43) et les capacités inhibitrices comparables des ligands P1 et P2, ces résultats indiquent une capacité de capture exacerbée pour les bandelettes préparées à partir de solution de sérum albumine polyfonctionnalisée (BSA-P1) par rapport à des bandelettes préparées à partir de solution de sérum albumine monofonctionnalisée (HSA-P2). De plus, le signal observé sur la bandelette BSA-P1 par la révélation avec le OR-mAb12h (42) est également plus fort que ceux observés à partir d'une même solution de MMP12h en utilisant des bandelettes avec du mAb12h que le traceur utilisé corresponde à OR-BSA-P1 (44), OR-HSA-P2 (46) et OR-BSA-P2 (48).

### Synthèse des résultats formant bandelette

Le système de bandelettes comportant de la BSA-P1 immobilisée couplé à l'utilisation d'un traceur mAb12h permet la détection spécifique et sélective en milieu tamponné de 6 fmol (0,12 ng) de MMP12h présentant un site actif libre (forme active de la MMP12h) par 100 µL d'échantillon, soit une sensibilité de détection de 1,2 ng/mL. La détection de MMP12h n'est opérante par ce système si et seulement si la MMP12h présente son site actif libre, a contrario de sa proforme avec son propeptide ou de la forme inactivée par un inhibiteur. En effet, le dispositif ne donne pas de signal positif lorsque la ProMMP12h et un complexe MMP12h/inhibiteur sont utilisés dans l'analyse. Parmi les MMPs testées, seule la MMP12h génère un signal positif et ce signal peut être observée même lorsque la MMP12h est initialement en mélange avec d'autres MMPs ayant une forte homologie. Le dispositif est compatible pour la détection de MMP12h (1 ng) initialement dans un milieu riche en protéines (240 µg), ce qui représente une possibilité de détection de la MMP12h lorsque cette dernière représente 0,003% des protéines totales. De plus, le système développé est compatible avec la détection de MMP12h placée dans des lavages broncho-alvéolaires (BAL) de souris. A ce jour, la sensibilité de détection démontrée dans ces milieux est de 80 fmol par 100 µL de BAL, soit 16 ng/mL. Ainsi, les bandelettes développées sont en outre compatibles avec des milieux complexes comme des extraits cellulaires ou des lavages broncho-alvéolaires. Il est à noter que les bandelettes, format ICT, présentent un avantage comme méthode de détection d'une MMP d'intérêt active dans un échantillon biologique car les bandelettes sont stables dans le temps et permettent une analyse rapide.

La sérum albumine fonctionnalisée par le ligand de la MMP d'intérêt peut être utilisée, dans la perspective de détecter des MMPs, à la fois en tant qu'entité immobilisée sur bandelettes pour capturer la cible MMP ultérieurement révélée par un anticorps monoclonal (42, 43, Figure 9), ou en tant que traceur capable de révéler la présence de la MMP d'intérêt capturée par un anticorps monoclonal immobilisée sur bandelettes (44, 46, 48, Figure 9). Le degré de fonctionnalisation de la sérum albumine par le ligand a peu d'incidence sur la capacité de la sérum albumine fonctionnalisée à détecter de la MMP12h capturée, alors que ce degré de fonctionnalisation a une incidence notable sur la capacité de la sérum albumine à capturer de la MMP12h pour une révélation ultérieure. Cet effet est corrélé positivement avec l'augmentation du degré de fonctionnalisation. Enfin, le format permettant la meilleure sensibilité de détection correspond au format basé sur le couplage de l'utilisation de sérum albumine polyfonctionnalisée par le ligand de la MMP cible immobilisée sur le support solide et d'un traceur constitué d'un anticorps monoclonal marqué à l'or.

### Conclusion format bandelette

Le format bandelettes offre la plus grande sensibilité de détection exclusive de MMP active lorsque de la sérum albumine polyfonctionnalisée par un ligand de la MMP d'intérêt est utilisée comme agent de capture, de concentration et de focalisation sur la bandelette de la MMP d'intérêt, la présence de celle-ci étant révélée *a posteriori* grâce à un anticorps monoclonal ou polyclonal marqué, par exemple à l'or colloidal. Cependant, la sérum albumine mono fonctionnalisée par un ligand de la MMP d'intérêt est également efficace pour la capture de MMP d'intérêt, mais avec une moindre sensibilité. La sérum albumine mono- ou poly-fonctionnalisée par un ligand de la MMP d'intérêt peut en outre être valorisée en tant que traceur lorsqu'elle est marquée, notamment à l'or colloidal, et utilisée avec des bandelettes fonctionnalisées par l'anticorps spécifique de la MMP cible.

### TEST EIA

La possibilité de transférer les approches explorées dans le format ICT (Bandelettes) a été évaluée dans la conception de tests de type EIA qui résulteraient en un signal positif dans l'unique cas de la présence de la MMP d'intérêt sous forme active, c'est-à-dire présentant son site actif inoccupé. Dans cette perspective, plusieurs types de plaques ont été préparés :
- plaque BSA-P1, présentant une immobilisation de la BSA polyfonctionnalisée avec le ligand P1 ;
- plaque HSA-P2, présentant une immobilisation de la HSA monofonctionnalisée avec le ligand P2 ;
- plaque mAb12h, plaque polyAb12h et plaque polyAb12m, présentant respectivement une immobilisation d'un anticorps monoclonal anti-MMP12 humaine, d'un anticorps polyclonal anti-MMP12 humaine, et d'un anticorps polyclonal anti-MMP12 murine.

L'utilisation des plaques BSA-P1 et les plaques HSA-P2 a été couplée à une détection en utilisant un anticorps monoclonal marquée à la biotine (Biot-mAb12h), secondairement détecté par de la streptavidine- AchE(G4) capable de cliver l'acétylthiocholine en présence de DTNB, ce qui génère un signal d'absorbance à 414 nm.

L'utilisation de plaques mAb12h, plaques polyAb12h et plaques polyAb12m est respectivement couplée à une détection en utilisant :
- Biot-HSA-P2, P3 ou l'AchE(G4)-P1 dans le cas des plaques mAb12h;
- P3 dans le cas des plaques polyAb12h et des plaques polyAb12m.

Dans tous les cas, le signal mesuré est celui de l'absorbance à 414 nm généré par AchE(G4) clivant l'acétylthiocholine en présence de DTNB. Les données présentées ci-après résultent pour chaque point de la mesure sur au minimum en triplicate de l'expérience.

### Plaque BSA-P1 : résultats

### Sensibilité de détection en milieu simple et en milieu complexe - [Figure 10]

L'utilisation de plaque BSA-P1 couplée à une détection via Biot-mAb12 a conduit à la détection de MMP12h, que la MMP12h soit en milieu tamponnée de type Tris-HCl 50 mM pH 6,8, CaCl₂ 10 mM, brij 0,01% ou en présence de 40 µg d'un mélange de protéines cytosoliques dans le même tampon. La détection de la MMP12h est positive jusqu'à des concentrations initiales de 1 pM en milieu tamponné et entre 1 et 10 pM dans un milieu complexe constitué de 40 µg d'un mélange de protéines cytosoliques, ce qui représentent respectivement des quantités initiales de MMP12h dans le volume d'analyse de 100 µL de 0,1 fmol (soit 2pg) et entre 0,1 et 1 fmol (soit entre 2 et 20 pg).

La spécificité de la capture de la MMP12h *via* son site actif inoccupée (donc uniquement de la forme active de MMP12h) par le ligand P1 porté par la BSA a été vérifiée par l'abolition de la génération d'absorbance lorsque l'échantillon analysé contient de la MMP12h préalablement inhibée en solution, en milieu simple ou complexe, par un inhibiteur de MMP (compétiteur).

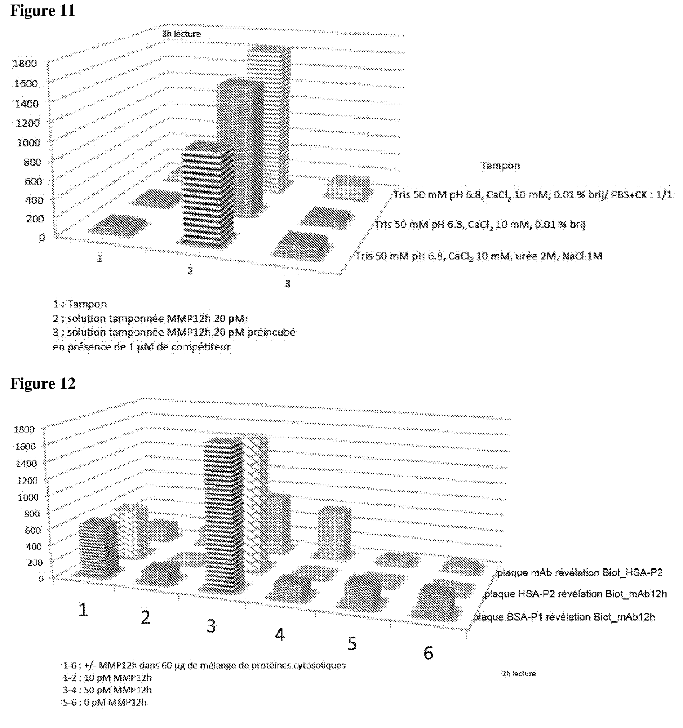

### Compatibilité avec différents tampons - [Figure 11]

La compatibilité du système de plaque BSA-P1 couplée à une détection *via* Biot-mAb12h a été établie pour différents tampons, usuellement utilisés comme diluant de milieux biologiques ou solution d'extraction de tissus. On a comparé les absorbances obtenues par l'analyse de solutions d'un volume de 100 µL de MMP12h à 20 pM dans :
- du tampon de type Tris-HCl50 mM pH6,8, CaCl₂ 10 mM, brij 0,01% ; ou
- dans un mélange de tampons Tris-HCl50 mM pH6,8, CaCl₂ 10 mM, brij 0,01%/PBS : 1/1 contenant un cocktail d'inhibiteurs de protéases à l'exception d'inhibiteurs de métalloprotéases ; ou
- dans un tampon de type Tris-HCl50 mM pH6,8, CaCl₂ 10 mM, urée 2M, NaCl 1M.

Une compatibilité du système de détection de la MM12h avec ces différents types de milieux tamponnés a pu être observée, ainsi qu'avec la présence d'un mélange de molécules organiques constituant le cocktail d'inhibiteurs de protéases. L'absence d'absorbance générée lorsque la MMP12h est préalablement inhibée en solution par un inhibiteur de MMP (compétiteur), indique la spécificité des signaux observés, puisque seule la MMP12h avec un site actif inoccupée (forme active) est détectée dans l'expérience.

Les absorbances générées sont d'intensités proches mais non identiques. Le signal mesuré pour une concentration initiale de MMP12h de 20 pM est supérieur dans un mélange de tampons Tris-HCl 50 mM pH6,8, CaCl₂ 10 mM, brij 0,01%/PBS (1/1) contenant un cocktail d'inhibiteurs de protéases (à l'exception d'inhibiteurs de métalloprotéases) par rapport celui observé dans un tampon Tris-HCl 50 mM pH6,8, CaCl₂ 10 mM, brij 0,01%, ce dernier étant supérieur à celui observé dans du tampon de type Tris-HCl 50 mM pH6,8, CaCl₂ 10 mM, urée 2M, NaCl 1M. Ces différences d'intensité peuvent refléter la capacité variable selon le tampon de la MMP12h à conserver sa structure tridimensionnelle tout autant que la capacité du ligand P1 porté par la BSA à pouvoir interagir efficacement avec sa cible.

### Comparaison des plaques BSA-P1, des plaques HSA-P2 et des plaques mAbl2h pour la détection de MMP12h en milieu complexe : résultats - [Figure 12]

Dans ces expériences, les échantillons ont été incubés en plaque puis, le retrait du surnageant a été suivi de lavages et de l'addition du traceur. L'utilisation de solutions identiques de MMP12h à 10 ou 50 pM en présence de 40 µg d'un mélange de protéines cytosoliques pour la détection de MMP12h par les plaques BSA-P1, HSA-P2 et mAB12h indiquent que :
- la MMP12h est aussi bien détectée par le système plaque BSA-P1//traceur Biot-mAb12h que par le système plaque HSA-P2//traceur Biot-mAb12h, ce qui indique que dans le cas d'un format EIA, le degré de substitution de la sérum albumine n'a pas d'effet notable sur la qualité de la détection de la MMP12h. Il est néanmoins à noter que, dans cette expérience, l'incubation des échantillons a été d'une nuit ce qui peut favoriser une capture optimale en termes de quantité de l'espèce d'intérêt.
- le système HSA-P2//traceur Biot-mAb12h détecte en milieu complexe une quantité de MMP12h de 1 fmol par 100 µL d'échantillon, ce qui correspond à une concentration initiale de MMP12h de 10 pM.
- comme le système BSA-P1//traceur Biot-mAb12h, le système HSA-P2//traceur Biot-mAb12h ne détecte pas la MMP12h lorsque l'accès au site actif de cette dernière est préalablement empêché par un compétiteur, rendant le système spécifique de la détection de MMP12h ayant son site actif inoccupé (MMP12h sous forme active).
- le bruit de fond généré à partir d'un échantillon de protéines complexes par les plaques BSA-P1 est plus élevé que celui observé à partir de plaque HSA-P2 (comparaison des champs 5 et 6). Ce bruit de fond de valeur supérieure peut également être aussi visualisé au niveau des expériences réalisées avec une préincubation des échantillons avec un compétiteur (champs 2 et 4).

Par contre, le système plaque mAb12h//traceur Biot-HSA-P1 dans son utilisation séquentielle, se distingue des deux autres systèmes par le fait que :
- la sensibilité de détection est plus faible. En effet, lorsqu'une solution de MMP12h à une concentration de 10 pM dans 40 µg d'un mélange de protéines cytosoliques est utilisée, l'absorbance observée dans le cas du mAb12h//traceur Biot-HSA-P1 ne peut permettre de conclure à la détection positive de MMP12h du fait de l'intensité du signal comparable à celui observé en absence de MMP12h.
- lorsque l'accès au site actif de la MMP12h a été préalablement empêché par un compétiteur, la MMP12h est néanmoins détectée (champs 4) par un signal d'intensité comparable à celui obtenu à partir d'une solution de MMP12h en absence de compétiteur (champs 3). Cette détection de MMP12h, préalablement inactivée, trouve probablement une explication dans l'élimination du compétiteur par les lavages faisant suite à la capture de MMP12h.

Le système plaque mAb12h//traceur Biot-HSA-P1, lorsque que l'incubation avec la plaque et le traceur sont séquentielles, permettrait ainsi la détection de MMP12h, mais sans être sélectif de la forme active de MMP12h (c'est-à-dire ayant son site actif inoccupé).

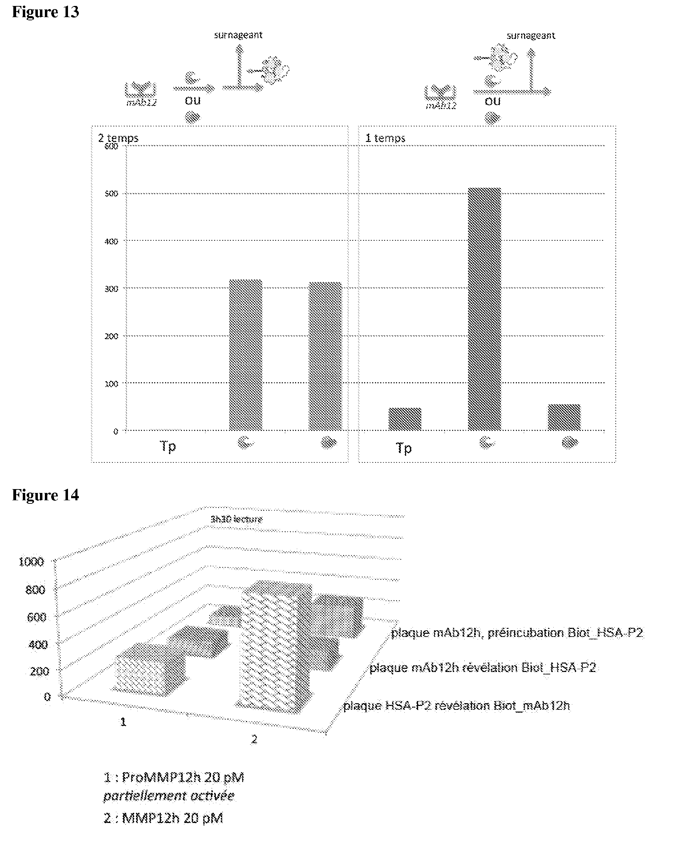

### Comparaison du moment d'addition du traceur Biot-HSA-P2 dans les tests utilisant des plaques mAb12h pour la détection de MMP12h : résultats - [Figure 13]

Dans ces experiences, les échantillons ont été incubés en plaque en présence ou en absence du traceur Biot-HSA-P2 puis, le retrait du surnageant a été suivi de lavages et de l'addition du traceur Biot-HSA-P2 lorsque celui-ci n'était pas présent pendant l'incubation de l'échantillon.

Les résultats montrent que lorsque des plaques sont fonctionnalisées par un anticorps, seul un protocole en 1 temps (l'échantillon contenant la MMP à détecter et le traceur ont été incubé ensemble dès le départ dans un puits fonctionnalisé par l'anticorps capable de reconnaître la MMP à détecter) permet une discrimination claire entre la présence de formes actives et de formes initialement inactivées (en complexe avec un inhibiteur) de MMP12h. Dans un protocole en deux temps (traceur biot-HSA-P2 est ajouté post-incubation de l'échantillon contenant la MMP et retrait du surnageant), un signal positif de détection est obtenu que la MMP soit initialement active ou en complexe avec un inhibiteur. Ainsi, la détection de la seule forme active de MMP requiert que tous les réactants soient incubés ensemble dès le départ pour permettre le dosage de MMP active via l'absorbance mesurée proportionnelle à la quantité de complexe complexe biot-HSA-P2//MMP active.

Ce dispositif a été validé pour la détection de MMP12h (50 pM) en utilisant des plaques chargées avec du mAb12h et pour la détection de MMP12m (50 pM) en utilisant des plaques chargées avec polyAb12m.

### Comparaison des plaques HSA-P2 et des plaques mAb12h pour la détection spécifique de MMP12h active par rapport à la ProMMP12h: résultats [Figure 14]

Des solutions de MMP12h et de ProMMP12h (proforme de MMP12h autoinhibée par son propre propeptide) à 20 pM dans de tampon Tris-HCl 50 mM pH6,8, CaCl₂ 10 mM, brij 0,01% ont été utilisées pour les systèmes de plaques HSA-P2//traceur Biot-mAb12h et de plaques mA12h/traceur Biot-HSA-P2 afin d'évaluer la capacité de ces systèmes à exclure la détection de ProMMP12h par rapport à celle de la MMP12h.

Dans le cas de l'utilisation de plaques mAb12h, à nouveau, deux types de moments d'addition du traceur ont été évalués. Un cas implique, comme précédemment, une addition du traceur à la suite de l'étape de capture après retrait du surnageant et lavages (plaque mAb12h, révélation Biot-HSA-P2), alors que le second résulte de l'incubation, dès le départ, de l'échantillon avec le traceur Biot-HSA-P2 (plaque mAb12h, préincubation Biot-HSA-P2). Les résultats indiquent que, comme précédemment observé, la MMP12h est mieux détectée par le système impliquant une plaque HSA-P2 que par le système impliquant une plaque fonctionnalisée par mAb12h, quel que soit le moment d'addition du traceur.

Par contre, la détection de MMP12h apparaît plus efficace pour des plaques mAb12h lorsque le traceur est incubé dès le départ avec l'échantillon (champs 2, plaque mAb12h, préincubation Biot-HSA-P2). De plus, ce protocole d'incubation en 1 temps est également plus apte à exclure la détection de ProMMP12, dont le signal associé est plus faible lorsque le traceur Biot-HSA-P2 est présent pendant l'incubation de l'échantillon sur la plaque mAb12h.

Pour ce qui concerne l'analyse de la solution de ProMMP12h par la plaque HSA-P2, l'intensité du signal tend à montrer la détection de MMP12h à partir de cet échantillon. Ainsi, il peut en être déduit que la solution de ProMMP12h comprend en outre une portion de MMP12h activée. Des mesures d'activité des solutions utilisées à l'aide d'un substrat fluorogénique ont permis d'expliquer ces résultats. En effet, ces mesures ont indiqué que la solution utilisée de ProMMP12h contenait une proportion estimée à 20% de MMP12h capable de dégrader le substrat fluorogénique. Ainsi, l'échantillon de ProMMP12h correspond en réalité à un mélange de ProMMP12h occupée au niveau de son site actif, et de MMP12h dont l'inoccupation du site actif donne à cette espèce la capacité de dégrader un substrat ou encore d'interagir avec l'inhibiteur P2 porté par la HSA immobilisée sur les plaques HSA-P2. Le système HSA-P2//traceur Biot-mAb12h est capable de détecter cette proportion de MMP12h sous forme active dans l'échantillon de ProMMP12h. Ainsi le système plaque HSA-P2//traceur Biot-mAb12h est bien capable de distinguer la présence de MMP12h et de ProMMP12h en fournissant un signal de détection positif dans l'unique cas de solutions contenant de la MMP12h active.

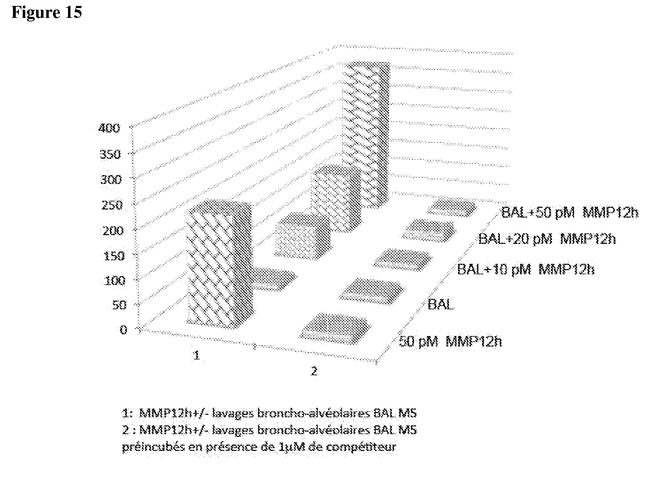

### Plaques HSA-P2 pour la détection de MMP12h dans des lavages broncho-alvéolaires : résultats - [Figure 15]

La possibilité de détection de MMP12h par le dispositif plaque HSA-P2 couplé à l'utilisation de traceur Biot-Ab12h a également été testée par ajout de différentes quantités de MMP12h à des lavages broncho-alvéolaires de souris. Ces expériences ont été prévues afin de caractériser la compatibilité d'un tel système pour détecter de la MMP12h à partir de BAL humains. Dans cette perspective, le milieu BAL M5 100% a été utilisé. Ce milieu correspond à un BAL de souris obtenu dans le PBS dilué au demi dans Tris-HCl 50 mM pH6,8, CaCl2 10 mM, brij 0,01%, puis à nouveau dilué au demi dans Tris-HCl 50 mM pH6,8, CaCl2 10 mM, brij 0,01%, NaCl 1M, urée 2M.

Les résultats nous indiquent une compatibilité du système HSA-P2//traceur Biot-Ab12h avec la présence de protéines présentes dans les BAL pour la détection de MMP12h. Les signaux observés (champs 1) sont abolis si les échantillons sont préincubés avec un inhibiteur compétiteur ciblant le site actif de MMP12h d'intérêt.

La sensibilité de détection dans le milieu BAL utilisé est de 1 fmol de MMP12h par 100 µL d'échantillon, ce qui représente une concentration molaire de 10 pM et une concentration massique de 200 pg/mL.

Les mêmes profils de résultats sont obtenus lorsque les plaques utilisées sont fonctionnalisées par des anticorps polyclonaux anti-MMP12h (plaque polyAb12h) en présence de MMP12h. Le traceur était le ligand P3 qui comprend une partie biotine. L'utilisation de mAb12h immobilisé sur plaque dans cette stratégie permet d'atteindre les mêmes niveaux de sensibilité que l'utilisation de polyAb12h.

Ce système est compatible avec des incubations réalisées dans différents types de tampon comme Tris-HCl 50 mM pH6,8, CaCl₂ 10 mM, brij 0,01% ou un mélange Tris-HCl 50 mM pH6,8, CaCl₂ 10 mM, brij 0,01%/PBS : 1/1 contenant un cocktail d'inhibiteurs de protéases à l'exception d'inhibiteurs de métalloprotéases, ou encore de type Tris-HCl 50 mM pH6,8, CaCl₂ 10 mM, urée 2M, NaCl 1M. Ainsi, le système est compatible pour la détection de la MM12h diluée par différents types de milieux tamponnés.

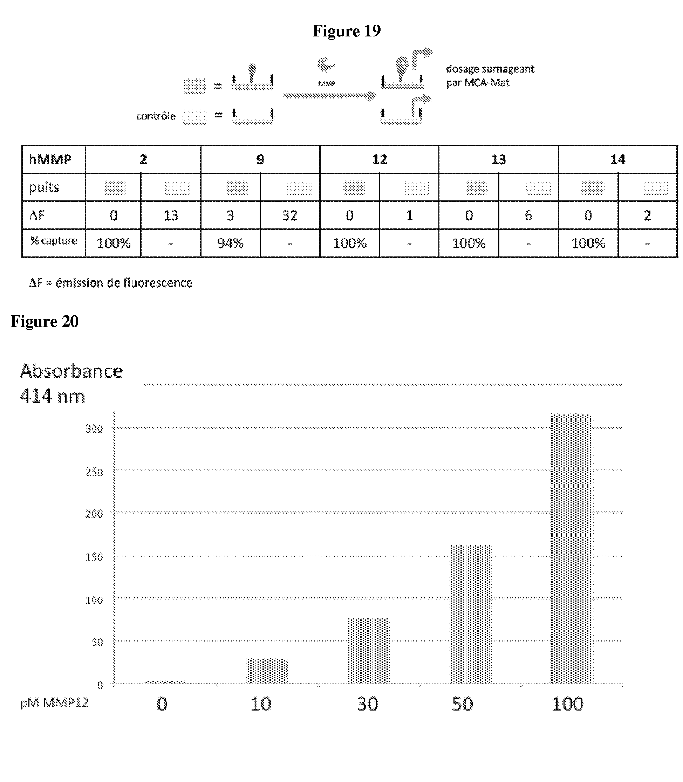

### Capture d'autres MMPs : résultats [Figure 19]

La capacité du système en version EIA pour capturer plusieurs MMPs a été démontrée par un dosage enzymatique des surnageants post-capture. Ce dosage n'est possible que dans ce format (il ne l'est pas dans le format bandelette) et opère par l'ajout au surnageant isolé après incubation du substrat fluorogénique commercial MCA-Mat dont le clivage en milieu tamponné par les MMPs génère un signal de fluorescence. La mesure de fluorescence est attendue nulle si la MMP a été capturée par l'entité chargée dans le puits (en l'occurrence la sérum albumine fonctionnalisée par un ligand de MMP). Ces mesures ont été réalisées par rapport à des surnageants provenant de puits contrôle chargés à l'aide de sérum albumine exempte de ligand de MMP (contrôle), afin de s'assurer que la capture de la MMP opère bien *via* une interaction spécifique avec le ligand de MMP. Ces expériences ont été réalisées avec des concentrations initiales de MMP de 100 pM, concentration compatible avec une émission de fluorescence (ΔF) mesurable par le fluorimètre utilisé et pour l'ensemble des MMP testées.

Les résultats présentés dans la Figure 19 indiquent clairement que l'étape de capture du dispositif est opérante pour l'ensemble des MMPs testées, ce qui est un prérequis obligatoire pour la détection des MMPs par suite à l'aide d'anticorps monoclonaux. Ainsi, les captures démontrées efficaces pourront être valorisées pour chaque MMP par l'utilisation d'anticorps monoclonaux spécifique de la MMP d'intérêt à détecter.

### Utilisation de AchE(G4) fonctionnalisé avec le Ligand P1 : [Figure 20]

AchE(G4-SMCC), la forme tétramérique de l'acétylcholinesterase (commercialisé par SPI-BIO), a été fonctionnalisé avec le ligand P1 pour conduire à AchE(G4)-P1. Ainsi, la liaison du ligand P1 peut être directement détectée via AchE.

Dans un premier test, l'ensemble des trois partenaires, à savoir AchE(G4)-P1, MMP12h, et mAb12h sont incubés ensemble dans un puits fonctionnalisés par du CAS, entité capable de reconnaître l'ensemble du répertoire des anticorps murins anti-humain. Après élimination du surnageant, lavages et addition d'un analogue de substrat de l'acétylcholine, l'absorbance mesurée est proportionnelle à la quantité de complexe AchE(G4)-P1//MMP12h active//mAb12h et permet ainsi le dosage de la MMP12h active. Ce système a été montré efficace pour la détection de MMP12h active jusqu'à des concentrations de 10 pM (50 µL, 100 pg/mL), avec quantité fixe de mAb12h (50 µL, 10 ng/mL). De façon analogue, ce dispositif a été validé pour la détection de MMP12m, à l'aide d'une quantité fixe d'anticorps polyclonal anti-MMP12m (immunopurifié), jusqu'à des concentrations de MMP12m active de 10 pM (50 µL, 100 pg/mL). Dans ce cas, la plaque est fonctionnalisée par du SAL, entité capable de reconnaître l'ensemble du répertoire des anticorps de lapin anti-souris.

Une variante de ce dispositif utilise des puits fonctionnalisés directement par les anticorps spécifiques de la MMP à détecter (plutôt que du CAS ou SAL). Dans ce dispositif, la AchE(G4)-P1 et l'échantillon contenant la MMP, soit MMP12h soit MMP12m, sont incubés ensembles dès le départ dans un puits fonctionnalisé soit par mAb ou polyAb. Après élimination du surnageant, lavages et addition d'un analogue de substrat de l'acétylcholine (AchE(G4)), l'absorbance mesurée est proportionnelle à la quantité de complexe AchE(G4)-P1//MMP active, et ne correspond ainsi qu'au dosage de la MMP active. Ce dispositif a été validé dans son principe pour la détection de MMP12h (50 pM) en utilisant des plaques chargées avec du mAb12h et pour la détection de MMP12m (50 pM) en utilisant des plaques chargées avec polyAb12m ainsi que dans différents tampons exempts de cocktail d'inhibiteur de protéases.

Il a pu être montré que ce dispositif fonctionne également lorsque le traceur AchE(G4)-P1 est ajouté post-incubation de l'échantillon contenant la MMP dans le puits. Cependant dans ce cas, la détection n'est pas spécifique de la forme active de la MMP.

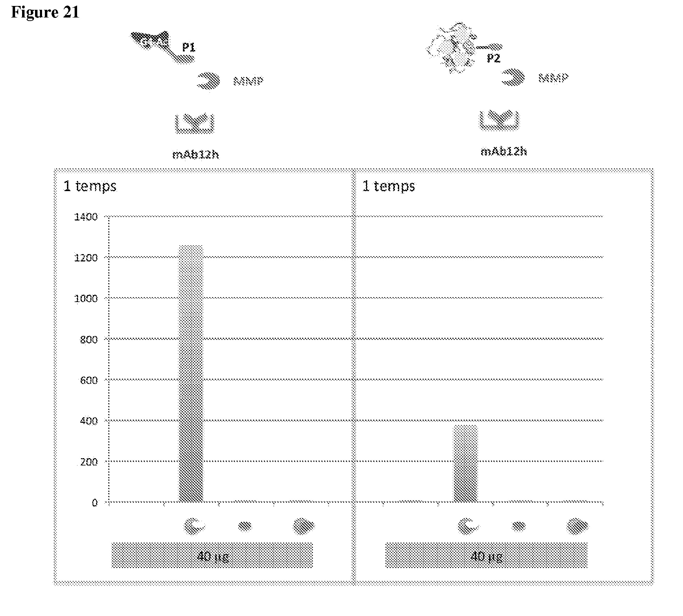

### Comparaison de AchE(G4)-P1 et biot-HSA-P2 pour la détection de MMP12h dans des milieux complexes [Figure 21]

L'utilisation des plaques fonctionnalisées par mAb12h avec la sérum albumine biotinylée ou la AchE(G4) respectivement porteuses des ligands P2 et P1 comme traceurs, avec une incubation simultanée de l'échantillon avec le traceur porteur des ligands P1 ou P2 ont été évaluées pour la MMP12h en présence de milieux complexes exemplifiés par un extrait de protéines cytosoliques. Les deux systèmes conduisent qu'à la détection positive de MMP12h active. La MMP12h initialement en interaction avec un inhibiteur compétitif ne peut interagir ni avec P1, ni avec P2 et n'est donc pas détectée. La présence de milieux complexes (40 µg d'extrait de protéines cytosoliques) et le temps d'incubation n'affectent pas la capacité de détection du système. L'activité catalytique de la AchE(G4) et la possibilité de reconnaissance de la sérum albumine portant le ligand (en interaction avec la cible d'intérêt) par la streptavidine- AchE(G4) ne se trouvent pas perturbées par le temps d'incubation et la présence d'extraits cellulaires doués d'une activité protéolytique. Cependant, le signal du système utilisant directement la AchE(G4) est plus élevé que celui utilisant biot-HSA-P2, secondairement détectée par la streptavidine- AchE(G4).

### Conclusion format EIA

Dans ce format, la sérum albumine mono- et poly-fonctionnalisée par un ligand de MMP constitue des agents de capture, concentration, efficace de la MMP d'intérêt, dont la présence est révélée *a posteriori* grâce à un anticorps monoclonal ou polyclonal spécifique de la MMP d'intérêt. Dans ce cas, l'utilisation de sérum albumine monofonctionnalisée par le ligand de MMP permet de limiter le coût du dispositif, ainsi que le bruit de fond.

Dans le format EIA utilisant un anticorps chargée, la sérum albumine et la AchE(G4)-monofonctionnalisée par le ligand de MMP peuvent être utilisées en tant qu'entités traceuses pour la détection de formes actives de MMP.

La détection exclusive de formes actives de MMP dans le format EIA nécessite que l'échantillon comprenant la MMP à détecter soit en contact dès le départ avec le ligand, ce qui est possible :
- avec des plaques fonctionnalisée par la sérum albumine porteuse de P1 ou P2, ou
- avec des plaques fonctionnalisées par des anticorps spécifiques de la MMP d'intérêt à condition que l'échantillon soit incubé en présence le ligand de MMP (par exemple, sérum albumine ou AchE(G4) portant le ligand de MMP), préalablement ou simultanément à l'incubation dans les puits.

Ces trois systèmes ont été montré compatibles avec la présence de mélanges complexes de protéines, qui n'affecte pas la capacité d'interaction de la MMP ciblée avec le ligand de MMP portée par la protéine porteuse et garantit une détection exclusive des seules formes actives de MMP.

Il est important de noter néanmoins que les signaux obtenus pour la détection de MMP12h sont de façon systématique d'intensité plus élevée lorsque des plaques fonctionnalisées par la sérum albumine porteuse de P1 ou P2 sont utilisées par rapport à l'utilisation de plaques fonctionnalisées par des anticorps.

### Détection MMP2h et MMP9h

La compatibilité de la stratégie développée pour la MMP12 a pu être établie pour la détection d'autres MMPs sous forme active, notamment la MMP2 et la MMP9 humaine.

Des plaques BSA-P1 fonctionnalisées par un ligand du site actif des MMP ont été utilisées pour la phase de capture et l'anticorps approprié a été utilisé pour l'étape de révélation. Elles ont été préparées comme décrit précédemment.

Les anticorps Biot-mAb2 et Biot-mab9 ont été utilisés. Ce sont des anticorps monoclonaux fonctionnalisé par des molécules de biotine. Biot-mab9 est un anticorps monoclonal anti-MMP9h reconnaissant la MMP9 humaine. Biot-mab2 est un anticorps monoclonal anti-MMP2h reconnaissant la MMP2 humaine. Biot-mAb2 a été obtenu après une étape de biotinylation de l'anticorps commercial monoclonal murin contre la MMP2 humaine (Anti-MMP-2 (Ab-8) Mouse (VB3) ; Calbiochem (EMD Millipore). Biot-mAb9 correspond à un anticorps monoclonal murin contre la MMP9 humaine qui est déjà biotinylé (MMP-9 (7-11C) : sc-13520 ; Santa Cruz Biotechnology, INC).

La préparation Biot-mAb2 a été réalisée à partir de solution de mAb2 dans du tampon borate 100 mM pH 9 et d'une solution de biotine active sous forme d'un ester N-hydroxysuccinimide (biotine-NHS) à 5mg/mL dans le DMF préparée extemporanément. Plus précisément, 25 µg de mAb2 (25µL d'une solution à 1 mg/mL dans du PBS) ont été placés dans 125 µL de tampon Borate 100mM ph=9,0 puis l'addition de 10µL de biotine-NHS à 5mg/mL a été réalisée. A la suite d'une incubation de 45 minutes à température ambiante, 50 µL de tampon Tris 1M ph=8,0 ont été ajoutés au mélange. Une incubation de 10 minutes suivie de l'addition d'un volume 790 µL de tampon phosphate 100mM pH 7,4, 0,1% BSA, 0,15M NaCl, 0,01% NaN₃ pour atteindre une concentration de traceur Biot-mAb2 de 25 µg/mL. La solution stock de Biot-mAb2 a été conservée à 4°C.

L'utilisation de plaques fonctionnalisées par BSA-P1 a été couplée à une révélation *via* le traceur biot-mAb2h ainsi qu'à celle via le traceur biot-mAb9h pour détecter respectivement de la MMP2h et de la MMP9h.

Après mise à température ambiante des plaques fonctionnalisées, les puits ont été lavés avec un laveur automatique de plaques par un cycle de 5 lavages d'un volume de 300 µL utilisant du tampon phosphate 100 mM, Tween 0,1%, pH 7,4.

Dans les tests EIA utilisant les plaques BSA-P1 et le traceur biot-mAb2h ou le traceur biot-mAb9h, les échantillons d'un volume de 100 µL contenant des concentrations croissantes de formes actives de MMP2h ou MMP9h en solution dans du tampon phosphate 50mM ou Tris.HCl 50 mM pH 7,4, 0,1% BSA, 0,15M NaCl, 0,01% NaN₃ ont alors été placés dans les puits pour une incubation de 3h à 25°C et d'une nuit à 4°C. Cette étape correspondant à l'étape de capture des espèces d'intérêt dans l'échantillon a été suivie d'un retrait du surnageant et d'une série de lavages avec trempage de 2 à 5 minutes sous agitation à l'aide de tampon Tris HCl 100 mM, 10 mM CaCl₂, NaCl 1M, 0,5 % Tween. S'est ensuivie l'addition de traceur biotinylé biot-mAb2 ou biot-mAb9 à une concentration 1 µg/mL utilisée dans du tampon PBS ou Tris.HCl 50 mM pH 7,4, NaCl 0,15 M, 0,1% BSA, NaN3 0,01%. L'incubation avec le traceur a été réalisée pendant une nuit à 4°C avant le retrait du surnageant, une nouvelle série de lavages à l'aide de tampon phosphate 50 mM, Tween 20 0,1%. 1 UE/min/mL de strepatvidine AchE(G4) (acétylcholinestérase) a été additionnée suivie d'une incubation de 2h avant retrait des surnageants, lavages et addition d'acetylthiocholine et de DTNB (DTNB= 5,10 ⁻⁴ M, Acétyl thiocholine 1,4.10⁻⁵ M dans du tampon phosphate 10 mM). La détection de la protéine d'intérêt a été mesurée *via* une mesure de l'activité de AchE(G4) dont l'action sur son substrat en présence de DTNB fournit une absorbance à 414 nm.

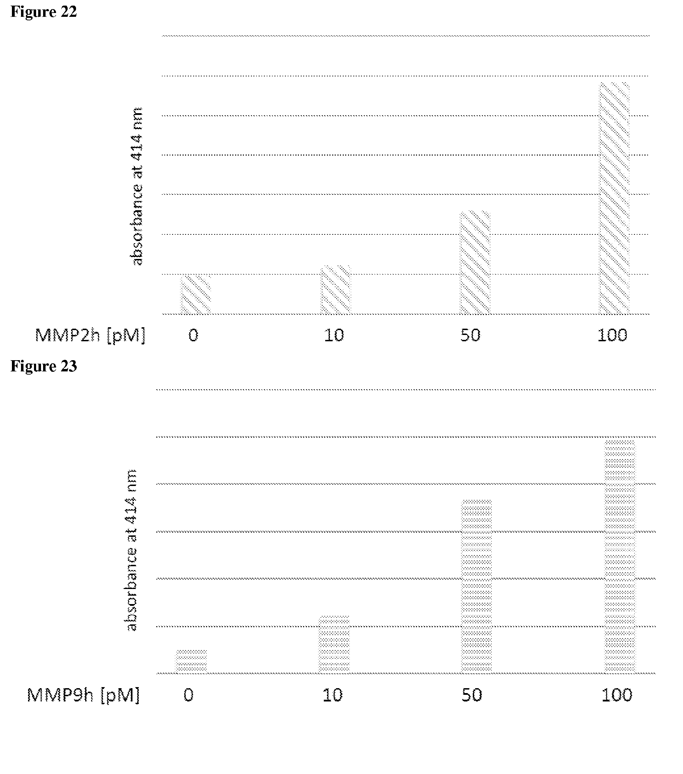

L'utilisation de plaque BSA-P1 couplée à une détection via Biot_mAb2h ou Biot_mAb9h conduit à la détection respective de MMP2h (Figure 22) et de MMP9h (Figure 23) en milieu tamponné Tris.HCl 50 mM pH 7,4, 0,1% BSA, 0,15M NaCl, 0,01% NaN₃. La détection de la MMP2h et MMP9h a pu être démontrée positive pour des concentrations initiales de 10 pM en milieu tamponné, ce qui représente respectivement des quantités initiales de MMP2h et MMP9h de 1 fmol (soit respectivement 66 pg et 65 pg) dans le volume d'analyse de 100 µL.

Ces résultats indiquent que les étapes de capture et de détection positive de formes actives de MMP ne sont pas dépendantes de la taille et de la masse moléculaire de la MMP. La détection reste uniquement dépendante du type d'anticorps utilisé et de la présence de MMP sous forme active.

Au delà de la détection de MMP 12, 2 ou 9 présentes dans l'échantillon avec des sensibilités comparables de l'ordre du pM (10⁻¹² M), l'utilisation d'autres sources d'anticorps monoclonaux anti-MMP existants notamment dans le commerce permet de disposer de systèmes de détection pour l'ensemble des MMP.

## Revendications

1. Méthode *in vitro* pour détecter spécifiquement dans un échantillon biologique une métalloprotéase matricielle (MMP) d'intérêt uniquement dans sa forme active, comprenant
a) une étape de mise en contact de l'échantillon biologique avec un ligand de la MMP d'intérêt capable de se fixer au site actif libre de la MMP;
b) ultérieurement ou simultanément à l'étape a), une étape de mise en contact avec un anticorps spécifique de la MMP d'intérêt du résultat de l'étape a); et
c) une étape de détection du complexe ternaire entre la MMP d'intérêt, le ligand de la MMP d'intérêt, et l'anticorps spécifique de la MMP d'intérêt,
et dans laquelle le ligand comprend un inhibiteur pseudopeptidique phosphinique.

2. Méthode selon la revendication 1, dans laquelle soit le ligand soit l'anticorps est immobilisé sur un support solide, et la détection est réalisée soit par la détection de l'anticorps lorsque le ligand est immobilisé sur le support, soit par la détection du ligand lorsque l'anticorps est immobilisé sur le support.

3. Méthode selon la revendication 1 ou 2, comprenant
a) la fourniture d'un support solide sur lequel est immobilisé un ligand de la MMP d'intérêt ;
b) la mise en contact du support solide avec l'échantillon de manière à permettre la fixation de la MMP d'intérêt présente dans l'échantillon au support solide par l'intermédiaire d'une liaison entre le ligand et la MMP d'intérêt ;
c) facultativement, l'élimination des MMPs non fixées ;
d) l'ajout d'un anticorps spécifique de la MMP d'intérêt de manière à permettre la formation du complexe ternaire entre la MMP d'intérêt, le ligand de la MMP d'intérêt, et l'anticorps spécifique de la MMP d'intérêt ;
e) facultativement, l'élimination des anticorps libres ; et
f) la détection des anticorps dans le complexe ternaire, la détection de ceux-ci étant indicatif de la présence dans l'échantillon de la MMP d'intérêt sous forme active.

4. Méthode selon la revendication 1 ou 2, comprenant
a) la fourniture d'un support solide sur lequel est immobilisé un anticorps spécifique de la MMP d'intérêt ;
b) la mise en contact du support solide avec l'échantillon qui est préalablement ou simultanément mis en contact avec un ligand de la MMP d'intérêt, de manière à permettre la fixation de la MMP d'intérêt présente dans l'échantillon au ligand et l'immobilisation de la MMP d'intérêt au support solide par l'intermédiaire d'une liaison entre l'anticorps et la MMP d'intérêt;
c) facultativement, l'élimination des ligands et MMP libres ; et
d) la détection du ligand dans le complexe ternaire entre la MMP d'intérêt, le ligand de la MMP d'intérêt, et l'anticorps spécifique de la MMP d'intérêt, la détection de celui-ci étant indicatif de la présence dans l'échantillon de la MMP d'intérêt sous forme active.

5. Méthode selon l'une quelconque des revendications 1-4, dans laquelle la méthode utilise un test d'immunochromatographie (ICT) ou un test immunologique en phase solide, la phase solide pouvant être notamment une membrane (« Flow-through »), un puits de plaque de microtitration (par exemple, EIA) ou des bandelettes (SPT).

6. Méthode selon l'une quelconque des revendications 1-5, dans laquelle la détection du ligand ou de l'anticorps est réalisée par couplage covalent ou non-covalent de celui-ci à un marqueur détectable.

7. Méthode selon la revendication 6, dans laquelle le marqueur détectable est sélectionné parmi le groupe consistant en un métal colloïdal, un colloïde non-métallique, du carbone, un traceur visible, fluorescent, luminescent ou chimio luminescent, une particule magnétique, un élément radioactif et une enzyme, de préférence l'or colloïdal ou une enzyme.

8. Méthode selon l'une quelconque des revendications 1-7, dans laquelle le ligand est couplé à une protéine porteuse, de préférence par l'intermédiaire d'un lien ou espaceur, en particulier un lien polyéthylèneglycol.

9. Méthode selon la revendication 8, dans laquelle la protéine porteuse est mono- ou poly-fonctionnalisée avec le ligand de la MMP d'intérêt.

10. Méthode selon l'une quelconque des revendications 8-9, dans laquelle la protéine porteuse est une sérum albumine, de préférence humaine ou bovine.

11. Méthode selon l'une quelconque des revendications 1-10, dans laquelle l'échantillon biologique est un liquide ou fluide biologique ou un extrait tissulaire ou cellulaire.

12. Méthode selon l'une quelconque des revendications 1-11, dans laquelle la MMP d'intérêt est sélectionnée parmi MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-26, MMP-27 et MMP-28, de préférence parmi MMP-2, MMP-3, MMP-8, MMP-9, MMP-10, MMP-12, MMP-13 et MMP-14, de préférence est MMP-12.

13. Méthode selon l'une quelconque des revendications 1-12, dans laquelle le ligand comprend un groupement de formule (I) : 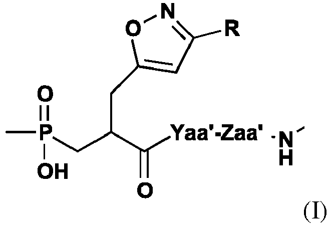 avec
Yaa' étant un acide aminé naturel autre que Asp, Pro, Gly, Cys et Gln, en particulier sélectionné parmi le groupe constitué de Ala, Arg, Asn, Glu, His, Ile, Leu, Lys, Met, Phe, Ser, Thr, Val, Trp et Tyr ;
Zaa' étant un acide aminé naturel autre que Pro et Cys, en particulier sélectionné parmi le groupe constitué de Ala, Arg, Asp, Asn, Gly, Gln, Glu, His, Ile, Leu, Lys, Met, Phe, Ser, Thr, Val, Trp et Tyr ;
R étant sélectionné parmi le groupe constitué de

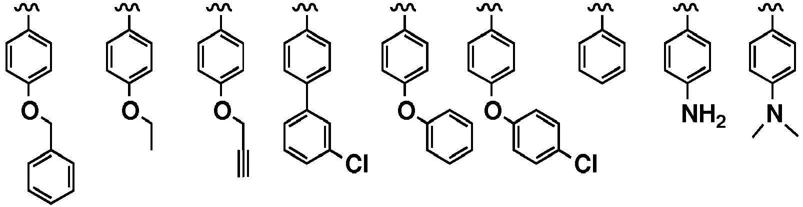

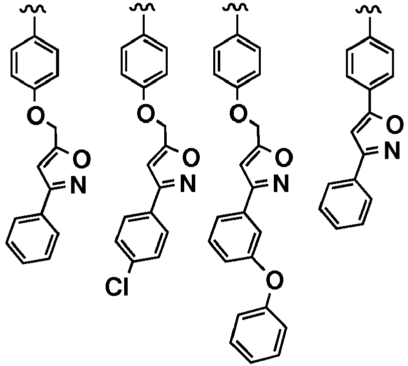

14. Méthode selon la revendication 13, dans laquelle le ligand comprend un groupement de formule (III)

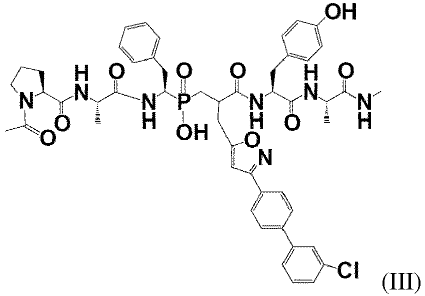

15. Méthode selon l'une quelconque des revendications 1-14 pour la détection de MMP12, MMP2 ou MMP9, de préférence MMP12.

16. Kit de détection spécifique d'une MMP d'intérêt uniquement dans sa forme active, comprenant
- un anticorps spécifique de la MMP d'intérêt ;
- un ligand de la MMP d'intérêt comprenant un inhibiteur pseudopeptidique phosphinique, de préférence fonctionnalisant une protéine porteuse ;
- facultativement, un support solide sur lequel est immobilisé soit l'anticorps, soit le ligand ; et
- facultativement, des réactifs permettant la détection de l'anticorps ou du ligand.

17. Utilisation de la méthode selon l'une quelconque des revendications 1-15 ou d'un kit selon la revendication 16 dans une méthode de diagnostic, en particulier de diagnostic du cancer, d'une infection virale, de l'arthrose, de l'arthrite rhumatoïde, de la maladie de Dupuytren, de l'athérosclérose, des pathologies respiratoires à composantes inflammatoires, comme la broncho-pneumopathie chronique obstructive, l'emphysème, et l'asthme, ou de maladies neurodégénératives comme la sclérose en plaque, la myasthénie gravis, un accident vasculaire cérébral, la sclérose amyotrophique latérale ou la maladie d'Alzheimer.

## Patentansprüche

1. *In vitro* Verfahren zum spezifischen Nachweis einer Matrix-Metalloprotease (MMP) von Interesse ausschließlich in ihrer aktiven Form in einer biologischen Probe, umfassend
a) einen Schritt des Inkontaktbringens der biologischen Probe mit einem Liganden der MMP von Interesse, der in der Lage ist, an die freie aktive Stelle der MMP zu binden;
b) anschließend oder gleichzeitig bei Schritt a) einen Schritt des Inkontaktbringens des Ergebnisses von Schritt a) mit einem Antikörper, der für die MMP von Interesse spezifisch ist; und
c) einen Schritt des Nachweises des ternären Komplexes zwischen der MMP von Interesse, dem Liganden der MMP von Interesse und dem Antikörper, der für die MMP von Interesse spezifisch ist,
und wobei der Ligand einen Phosphinsäure-Pseudopeptid-Hemmer umfasst.

2. Verfahren gemäß Anspruch 1, wobei entweder der Ligand oder der Antikörper auf einem festen Träger immobilisiert ist und der Nachweis entweder durch den Nachweis des Antikörpers erfolgt, wenn der Ligand auf dem Träger immobilisiert ist, oder durch den Nachweis des Liganden erfolgt, wenn der Antikörper auf dem Träger immobilisiert ist.

3. Verfahren gemäß Anspruch 1 oder 2, umfassend
a) Bereitstellen eines festen Trägers, auf dem ein Ligand der MMP von Interesse immobilisiert ist;
b) Inkontaktbringen des festen Trägers mit der Probe, um die Bindung der in der Probe vorhandenen MMP von Interesse an den festen Träger über eine Bindung zwischen dem Liganden und der MMP von Interesse zu ermöglichen;
c) gegebenenfalls Entfernen der nichtgebundenen MMPs;
d) Zugabe eines Antikörpers, der für die MMP von Interesse spezifisch ist, um die Bildung des ternären Komplexes zwischen der MMP von Interesse, dem Liganden der MMP von Interesse und dem Antikörper, der für die MMP von Interesse spezifisch ist, zu ermöglichen;
e) gegebenenfalls Entfernen der freien Antikörper; und
f) Nachweis der Antikörper in dem ternären Komplex, wobei der Nachweis der Antikörper auf das Vorhandensein der MMP von Interesse in aktiver Form in der Probe hinweist.

4. Verfahren gemäß Anspruch 1 oder 2, umfassend
a) Bereitstellen eines festen Trägers, auf dem ein Antikörper immobilisiert ist, der für die MMP von Interesse spezifisch ist;
b) Inkontaktbringen des festen Trägers mit der Probe, die zuvor oder gleichzeitig mit einem Liganden der MMP von Interesse in Kontakt gebracht worden ist, um die Bindung der in der Probe vorhandenen MMP von Interesse an den Liganden und die Immobilisierung der MMP von Interesse an den festen Träger über eine Bindung zwischen dem Antikörper und der MMP von Interesse zu ermöglichen;
c) gegebenenfalls Entfernen der freien Liganden und MMP; und
d) Nachweis des Liganden in dem ternären Komplex zwischen der MMP von Interesse, dem Liganden der MMP von Interesse und dem Antikörper, der für die MMP von Interesse spezifisch ist, wobei der Nachweis des Liganden auf das Vorhandensein der MMP von Interesse in aktiver Form in der Probe hinweist.

5. Verfahren gemäß einem der Ansprüche 1 bis 4, wobei das Verfahren einen immunchromatographischen Test (ICT) oder einen Festphasenimmunassay verwendet, wobei die feste Phase insbesondere eine Membran ("Flow-through"), eine Vertiefung einer Mikrotiterplatte (zum Beispiel EIA) oder Teststreifen (SPT) sein kann.

6. Verfahren gemäß einem der Ansprüche 1 bis 5, wobei der Nachweis des Liganden oder des Antikörpers mittels kovalenter oder nichtkovalenter Kopplung dieser an einen nachweisbaren Marker erfolgt.

7. Verfahren gemäß Anspruch 6, wobei der nachweisbare Marker aus der Gruppe ausgewählt ist, bestehend aus einem kolloidalen Metall, einem Nichtmetall-Kolloid, Kohlenstoff, einer sichtbaren, fluoreszierenden, lumineszierenden oder chemolumineszierenden Markierungssubstanz, einem magnetischen Partikel, einem radioaktiven Element und einem Enzym, vorzugsweise kolloidalem Gold oder einem Enzym.

8. Verfahren gemäß einem der Ansprüche 1 bis 7, wobei der Ligand an ein Trägerprotein, vorzugsweise über einen Linker oder Spacer, insbesondere einen Polyethylenglycol-Linker, gekoppelt ist.

9. Verfahren gemäß Anspruch 8, wobei das Trägerprotein mit dem Liganden der MMP von Interesse mono- oder polyfunktionalisiert ist.

10. Verfahren gemäß einem der Ansprüche 8 bis 9, wobei das Trägerprotein Serumalbumin, vorzugsweise humanes oder bovines Serumalbumin, ist.

11. Verfahren gemäß einem der Ansprüche 1 bis 10, wobei die biologische Probe eine biologische Flüssigkeit oder Fluid oder ein Zell- oder Gewebeextrakt ist.

12. Verfahren gemäß einem der Ansprüche 1 bis 11, wobei die MMP von Interesse aus MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-26, MMP-27 und MMP-28, vorzugsweise aus MMP-2, MMP-3, MMP-8, MMP-9, MMP-10, MMP-12, MMP-13 und MMP-14, ausgewählt ist, vorzugsweise MMP-12 ist.

13. Verfahren gemäß einem der Ansprüche 1 bis 12, wobei der Ligand eine Gruppe mit der Formel (I) umfasst: 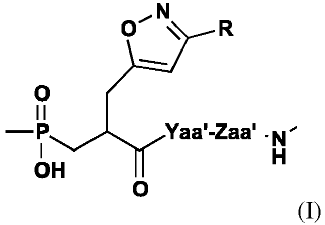 wobei
Yaa' eine natürliche Aminosäure außer Asp, Pro, Gly, Cys und Gln ist, insbesondere aus der Gruppe ausgewählt ist, bestehend aus Ala, Arg, Asn, Glu, His, Ile, Leu, Lys, Met, Phe, Ser, Thr, Val, Trp und Tyr;
Zaa' eine natürliche Aminosäure außer Pro und Cys ist, insbesondere aus der Gruppe ausgewählt ist, bestehend aus Ala, Arg, Asp, Asn, Gly, Gln, Glu, His, Ile, Leu, Lys, Met, Phe, Ser, Thr, Val, Trp und Tyr;
R aus der Gruppe ausgewählt ist, bestehend aus

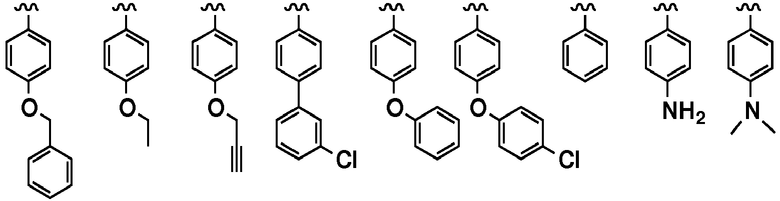

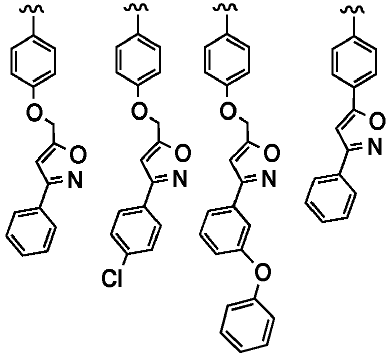

14. Verfahren gemäß Anspruch 13, wobei der Ligand eine Gruppe mit der Formel (III) umfasst

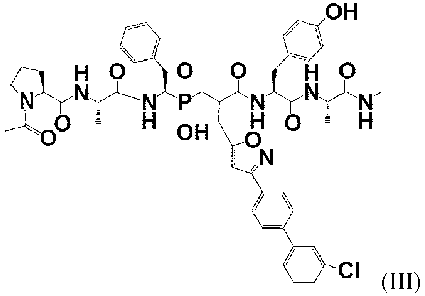

15. Verfahren gemäß einem der Ansprüche 1 bis 14 zum Nachweis von MMP12, MMP2 oder MMP9, vorzugsweise MMP12.

16. Kit zum spezifischen Nachweis einer MMP von Interesse ausschließlich in ihrer aktiven Form, umfassend
- einen Antikörper, der für die MMP von Interesse spezifisch ist;
- einen Liganden der MMP von Interesse, umfassend einen Phosphinsäure-Pseudopeptid-Hemmer, der vorzugsweise ein Trägerprotein funktionalisiert;
- gegebenenfalls einen festen Träger, auf dem entweder der Antikörper oder der Ligand immobilisiert ist; und
- gegebenenfalls Reagenzien, die den Nachweis des Antikörpers oder des Liganden erlauben.

17. Anwendung des Verfahrens gemäß einem der Ansprüche 1 bis 15 oder Verwendung eines Kits gemäß Anspruch 16 in einem Diagnoseverfahren insbesondere zur Diagnose von Krebs, einer viralen Infektion, Arthrose, rheumatoider Arthritis, Morbus Dupuytren, Arteriosklerose, von respiratorischen Erkrankungen mit entzündlichen Komponenten, wie chronisch-obstruktiver Bronchopneumopathie, Emphysem und Asthma, oder von neurodegenerativen Krankheiten wie Multipler Sklerose, Myasthenie gravis, Schlaganfall, amyotropher Lateralsklerose oder Morbus Alzheimer.

## Claims

1. In vitro method for specifically detecting in a biological sample a matrix metalloproteinase (MMP) of interest only in its active form, comprising
a) a step of contacting the biological sample with a ligand of the MMP of interest capable of binding to the free active site of the MMP;
b) subsequently to or simultaneously with step a), a step of contacting the result of step a) with an antibody specific for the MMP of interest; and
c) a step of detecting the ternary complex between the MMP of interest, the ligand of the MMP of interest, and the antibody specific for the MMP of interest,
and wherein the ligand comprises a phosphinic pseudopeptide inhibitor.

2. Method according to claim 1, wherein either the ligand or the antibody is immobilized on a solid support, and detection is achieved either by detecting the antibody when the ligand is immobilized on the support, or by detecting the ligand when the antibody is immobilized on the support.

3. Method according to claim 1 or 2, comprising
a) providing a solid support on which a ligand of the MMP of interest is immobilized;
b) contacting the solid support with the sample so as to allow attachment of the MMP of interest present in the sample to the solid support via a binding between the ligand and the MMP of interest;
c) optionally, removing the unattached MMPs;
d) adding an antibody specific for the MMP of interest to allow formation of the ternary complex between the MMP of interest, the ligand of the MMP of interest, and the antibody specific for the MMP of interest;
e) optionally, removing the free antibodies; and
f) detecting antibodies in the ternary complex, this detection being indicative of the active form of the MMP of interest being present in the sample.

4. Method according to claim 1 or 2, comprising
a) providing a solid support on which an antibody specific for the MMP of interest is immobilized;
b) contacting the solid support with the sample which has been previously or is simultaneously contacted with a ligand of the MMP of interest, so as to allow attachment of the MMP of interest present in the sample to the ligand and immobilization of the MMP of interest on the solid support via a linkage between the antibody and the MMP of interest;
c) optionally, removing the free ligands and MMPs; and
d) detecting the ligand in the ternary complex between the MMP of interest, the ligand of the MMP of interest, and the antibody specific for the MMP of interest, this detection being indicative of the presence in the sample of the active form of the MMP of interest.

5. Method according to any one of claims 1-4, wherein the method uses an immunochromatographic test (ICT) or a solid phase immunoassay, the solid phase possibly being a membrane (flow-through), a well of a microtiter plate (EIA for example), or strips (SPT).

6. Method according to any one of claims 1-5, wherein detection of the ligand or antibody is obtained by its covalent or non-covalent coupling to a detectable marker.

7. Method according to claim 6, wherein the detectable marker is selected from the group consisting of a colloidal metal, a non-metal colloid, carbon, a visible, fluorescent, luminescent, or chemiluminescent tracer, a magnetic particle, a radioactive element, and an enzyme, preferably colloidal gold or an enzyme.

8. Method according to any one of claims 1-7, wherein the ligand is coupled to a carrier protein, preferably via a linker or spacer, in particular a polyethylene glycol linker.

9. Method according to claim 8, wherein the carrier protein is mono- or polyfunctionalized with the ligand of the MMP of interest.

10. Method according to any one of claims 8-9, wherein the carrier protein is serum albumin, preferably human or bovine.

11. Method according to any one of claims 1-10, wherein the biological sample is a biological liquid or fluid, or a tissue or cell extract.

12. Method according to any one of claims 1-11, wherein the MMP of interest is selected from among MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-26, MMP-27, and MMP-28, preferably from among MMP-2, MMP-3, MMP-8, MMP-9, MMP-10, MMP-12, MMP-13, and MMP-14, is preferably MMP-12.

13. Method according to any one of claims 1-12, wherein the ligand comprises a moiety of formula (I) : 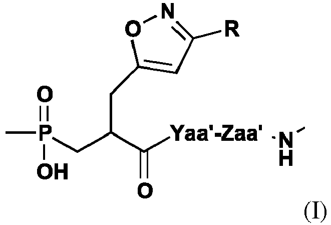 where
Yaa' is an natural amino acid other than Asp, Pro, Gly, Cys, and Gln, in particular selected from the group consisting of Ala, Arg, Asn, Glu, His, Ile, Leu, Lys, Met, Phe, Ser, Thr, Val, Trp, and Tyr;
Zaa' is a natural amino acid other than Pro and Cys, in particular selected from the group consisting of Ala, Arg, Asp, Asn, Gly, Gln, Glu, His, Ile, Leu, Lys, Met, Phe, Ser, Thr, Val, Trp, and Tyr;
R is selected from the group consisting of

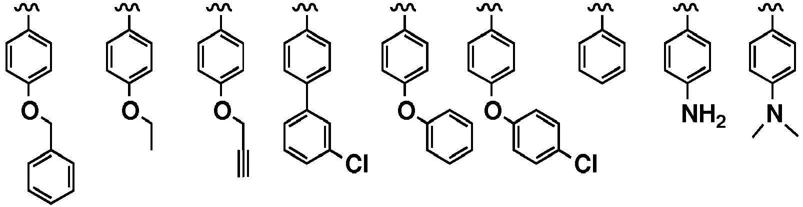

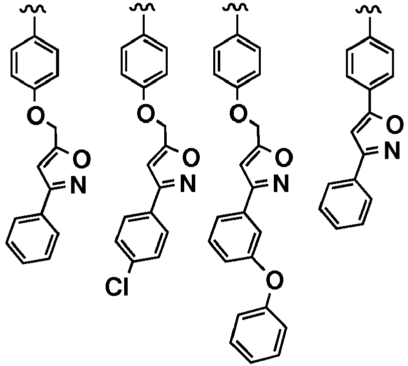

14. Method according to claim 13, wherein the ligand comprises a moiety of formula (III)

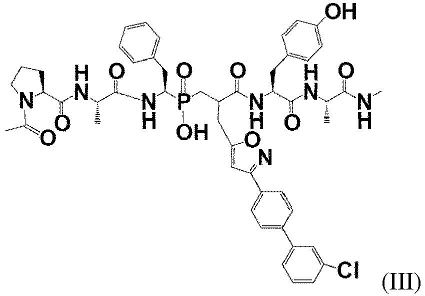

15. Method according to any one of claims 1-14 for the detection of MMP12, MMP2, or MMP9, preferably MMP12.

16. Kit for specifically detecting an MMP of interest solely in its active form, comprising
- an antibody specific for the MMP of interest;
- a ligand of the MMP of interest, comprising a phosphonic pseudopeptide inhibitor, preferably functionalizing a carrier protein;
- optionally, a solid support on which is immobilized either the antibody or the ligand; and
- optionally, reagents enabling detection of the antibody or ligand.

17. Use of the method according to any one of claims 1 to 15 or use of a kit according to claim 16 in a diagnostic method, in particular for the diagnosis of cancer, viral infection, osteoarthritis, rheumatoid arthritis, Dupuytren's contracture, atherosclerosis, respiratory diseases with inflammatory components such as chronic obstructive pulmonary disease, emphysema, and asthma, or neurodegenerative diseases such as multiple sclerosis, myasthenia gravis, stroke, amyotrophic lateral sclerosis, or Alzheimer's disease.
